(19) Europäisches Patentamt
European Patent Office
Office européen des brevets

(11) Veröffentlichungsnummer: **0 002 836**
**B1**

(12) # EUROPÄISCHE PATENTSCHRIFT

(45) Veröffentlichungstag der Patentschrift 27.10.82

(51) Int. Cl.³: **C 07 C 103/84,** C 07 C 103/48, C 07 C 103/66, A 61 K 31/195

(21) Anmeldenummer 78101869.2

(22) Anmeldetag 29.12.78

(54) **Acylhydrocarbylaminoalkansäuren, ihre Herstellung und Verwendung sowie sie enthaltende Arzneimittel.**

(30) Priorität 30.12.77 LU 78805

(43) Veröffentlichungstag der Anmeldung 11.07.79 Patentblatt 79/14

(45) Bekanntmachung des Hinweises auf die Patenterteilung: 27.10.82 Patentblatt 82/43

(84) Benannte Vertragsstaaten: **BE CH DE FR GB IT LU NL SE**

(56) Entgegenhaltungen:
**FR-A-1 554 477**
**LU-A- 69 970**

**GB-A-1 130 490;**
**Shigehiko Sugasawa and Tozo Fujii (Univ. Tokyo) — Chem. Pharm. Bull. (Tokyo) 6, 587-90 (1958).**

(73) Patentinhaber: **Byk Gulden Lomberg Chemische Fabrik GmbH, Byk-Gulden-Strasse 2, D-7750 Konstanz (DE)**

(72) Erfinder: **Krastinat, Walter, Dr., Erfurter Strasse 9, D-7750 Konstanz (DE)**
Erfinder: **Riedel, Richard, Dr., Bruelstrasse 22, D-7750 Konstanz (DE)**

**EP 0 002 836 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen)

BUNDESDRUCKEREI BERLIN

Acylhydrocarbylaminoalkansäuren, ihre Herstellung und Verwendung sowie sie enthaltende Arzneimittel

N-Niederalkyl-γ-aminobuttersäure-benzylester bzw. N-Phenyl-γ-aminobuttersäureniederalkylester sollen zur Behandlung von Magengeschwüren Anwendung finden [JA-PS 38(63)-15368 bzw. 38(63)-7324]. N-(2-Alkoxybenzoylaminosäuren wird eine antipyretische und analgetische Wirkung zugeschrieben (DE-OS 2 407 016). N-Trialkoxybenzoyl-aminoalkansäuren besitzen bei der Behandlung von Herzkrankheiten, z. B. Herzinfarkt, Herzischämie oder Herzarrhythmie, eine große Bedeutung (DE-OS 2 034 192, 2 050 949, 2 131 626, 2 131 674, 2 131 675, 2 131 679 und 2 131 680). N-Propionyl-ε-aminocapronsäure ist als Wirkstoff für kosmetische Präparate zur Behandlung von Anomalien bei der Vernarbung der Haut, die mit Störungen der Bindegewebsbildung zusammenhängen, vorgesehen (DE-AS 1 922 193). Gegebenenfalls durch eine Alkylgruppe mit 1 bis 3 Kohlenstoffatomen substituierte N-Acyl-aminoalkansäuren und vorzugsweise ihre langkettigen Alkylester werden in Mitteln zum Schutze, zur Pflege und zur Behandlung der Haut verwendet (DE-OS 2 234 399). 4-[Methyl-benzoyl-amino]-buttersäure und 6-[Methyl-benzoyl-amino]-hexansäure-(1) werden in Beilstein's Handbuch der organischen Chemie (EIII9, 1150 bzw. 1158) beschrieben. Eine Beschreibung der Herstellung von 5-(N-Methyl-acetamido)-valeriansäure findet sich in J. Chem. Soc. [London] C 1969, 1863. N-Benzoyl-N-phenyl-α-aminosäuren sollen entzündungshemmende und analgetische Wirkungen besitzen (DE-OS 1 768 173), während bei der Untersuchung von N-Benzoyl-anilinoalkancarbonsäuren [D. Evans et al. J. Med. Chem. 12 (1969) 1006-10] die entsprechenden Buttersäuren keine entzündungshemmende Wirkung zeigten. In der DE-OS 1 917 036 werden cholerisch wirkende acylierte Anilinocarbonsäuren beansprucht, denen noch weitere Wirkungen zugesprochen werden (DE-OS 2 450 680). α-Phenylbenzyliden-ω-aminoalkancarbonsäuren und ihre Derivate sollen in antiepileptisch wirkenden Mitteln verwendet werden (DE-OS 2 634 288).

In der FR-A-1 554 477 werden Amidocarbonsäuren beschrieben, die als Bestandteil von Korrosionsschutzölen Verwendung finden sollen. Im Chem. Pharm. Bull. (Tokyo) 6, 587-90 (1958) wird die Herstellung verschiedener N-Benzyl-acetamido- und -benzamido-carbonsäuren beschrieben. – In der GB-A-1 130 480 werden verzweigtkettige Amidocarbonsäuren mit oberflächenaktiven Eigenschaften beschrieben.

Es wurde nun eine neue Klasse von Acylhydrocarbylaminoalkansäuren synthetisiert, die weder in den erwähnten Publikationen genannt, noch durch sie nahegelegt wird. Ferner wurde gefunden, daß diese Acylhydrocarbylaminoalkansäuren interessante, besonders vorteilhafte pharmakologische Eigenschaften aufweisen.

Gegenstand der Erfindung sind Acylhydrocarbylaminoalkansäuren der allgemeinen Formel I

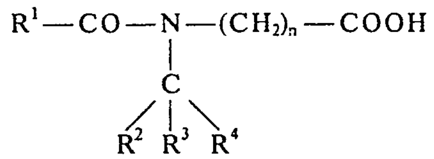

(I)

worin

$R^1$ $C_1-C_7$ Alkyl, $C_2-C_7$ Alkenyl, $C_2-C_7$ Alkinyl, $C_3-C_{10}$ Cycloalkyl oder Phenyl der allgemeinen Formel

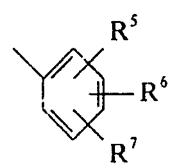

$R^2$ Wasserstoff, $C_1-C_7$ Alkyl, $C_2-C_7$ Alkenyl oder $C_2-C_7$ Alkinyl,

$R^3$ Wasserstoff, $C_1-C_7$ Alkyl, $C_3-C_{10}$ Cycloalkyl oder Phenyl der allgemeinen Formel

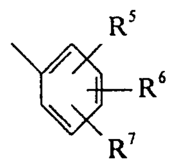

und

$R^4$ $C_1-C_7$ Alkyl, $C_3-C_{10}$ Cycloalkyl, Phenyl der allgemeinen Formel

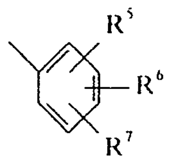

oder Phenylalkyl mit $C_1-C_4$ Alkyl und Phenyl der allgemeinen Formel

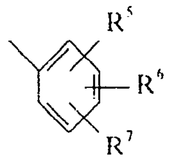

bedeuten, wobei die Summe der Kohlenstoffatome der Alkylsubstituenten $R^2$, $R^3$ und $R^4$ wenigstens 3 beträgt und wobei die Substituenten $R^2$, $R^3$ und $R^4$ gemeinsam mit dem Kohlenstoffatom, an das sie gebunden sind, kein geradkettiges oder verzweigtes Alkyl oder Alkenyl darstellen, wenn $R^1$ Alkyl oder Alkenyl ist und n 3 bedeutet, oder

$R^3$ und $R^4$ gemeinsam $C_2-C_8$ Alkylen oder

$R^2$, $R^3$ und $R^4$ unter Einschluß des benachbarten Kohlenstoffatoms Adamantyl,

n 3, 4 oder 5 bedeuten und

$R^5$, $R^6$ und $R^7$ gleich oder verschieden sind und Wasserstoff, Halogen, $C_1-C_4$ Alkyl, $C_1-C_4$ Alkoxy, $C_1-C_3$ Alkylmercapto, $C_2-C_5$ Alkanoyloxy, Amino, Nitro, Trifluormetnyl, Trifluormethoxy, Hydroxy oder Trifluormethylmercapto bedeuten, wobei $R^5$, $R^6$ und $R^7$ nicht gleichzeitig Wasserstoff bedeuten wenn $R^1$ und $R^4$ einen Phenylrest und $R^2$ und $R^3$ ein Wasserstoffatom darstellen,

sowie ihrer Salze mit anorganischen und organischen Basen.

Als Alkylreste mit 1 bis 7 Kohlenstoffatomen kommen geradkettige oder verzweigte in Frage. Geradkettige Alkylreste sind der Methyl-, Ethyl-, Propyl-, Butyl-, Pentyl-, Hexyl- oder Heptylrest, von denen die mit 1 bis 5, vor allem mit 1 bis 2, Kohlenstoffatomen bevorzugt sind. Verzweigte Alkylreste mit 3 bis 7 Kohlenstoffatomen sind z. B. der Isopropyl-, Isobutyl-, sek. Butyl- oder tert.Butylrest, von denen die mit 3 bis 5 Kohlenstoffatomen bevorzugt sind. Alkenyl- und Alkinylreste mit 2 bis 7 Kohlenstoffatomen sind beispielsweise der Ethenyl-, der Ethinyl-, der 1-Propenyl-, 1,3-Butadienyl-, 2-Butinylrest. Cycloalkylreste mit 3 bis 10 Kohlenstoffatomen sind beispielsweise der Cyclopropyl-, Cyclobutyl-, Cyclopentyl-, Cyclohexyl-, Cycloheptyl- oder Cyclooctylrest, von denen die mit 5 bis 8 Kohlenstoffatomen bevorzugt sind.

Als Halogenatome $R^5$, $R^6$ und $R^7$ kommen Fluor, Chlor oder Brom, bevorzugt Fluor und Chlor, insbesondere Chlor, in Betracht. Von den Alkylgruppen bzw. Alkoxygruppen bzw. Alkylmercaptogruppen $R^5$, $R^6$ und $R^7$ sind die mit 1 bis 3, vor allem die mit 1 Kohlenstoffatom(en) bevorzugt. Von den Alkanoyloxygruppen mit 2 bis 5 Kohlenstoffatomen ist die Acetoxygruppe bevorzugt.

Stellen $R^3$ und $R^4$ gemeinsam Alkylenguppen dar, so handelt es sich vorzugsweise um solche mit 4 bis 7 Kohlenstoffatomen. Stellen $R^2$, $R^3$ und $R^4$ unter Einschluß des benachbarten Kohlenstoffatoms einen Adamantylrest dar, so wird darunter der Adamantyl-(1)-Rest verstanden.

Als Phenylalkylgruppen seien beispielsweise genannt die Benzyl-, Phenethyl- und Phenylpropylgruppe, von denen die Benzylgruppe bevorzugt ist. Unter den substituierten Phenylalkylgruppen sind die im Phenylrest mono- oder disubstituierten bevorzugt. Beispielsweise genannt seien die p-Chlorbenzyl-, die m-Chlorbenzyl-, die p-Brombenzyl-, die o-Fluorbenzyl-, die p-Fluorbenzyl-, die p-Methylbenzyl-, die p-Methoxybenzylgruppe, die 3,4-Dimethoxybenzylgruppe.

Als Salze kommen Salze mit anorganischen und organischen Basen in Betracht. Pharmakologisch nicht verträgliche Salze werden nach an sich bekannten Methoden in pharmakologisch, d. h. biologisch, verträgliche Salze übergeführt, die unter den erfindungsgemäßen Salzen bevorzugt sind. Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker, basische Aminsäuren etc. zur Anwendung.

Beispielsweise seien die Salze von Lithium, Natrium, Kalium, Magnesium, Calcium, Aluminium, Ethylendiamin, Dimethylamin, Diethylamin, Morpholin, Piperidin, Piperazin, N-Niedrigalkylpiperazin (z. B. N-Methylpiperazin), Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methylglucamin, Glucosamin, N-Methylglucosamin, Lysin, Ornithin, Arginin, Chinolin genannt.

Bei den Ausgestaltungen der Erfindung werden die eingeschränkten Bedeutungen der einzelnen Substituentensymbole durch einen oder mehrere Sternchen gekennzeichnet.

Eine Ausgestaltung der Erfindung sind Acylhydrocarbylaminoalkansäuren der allgemeinen Formel

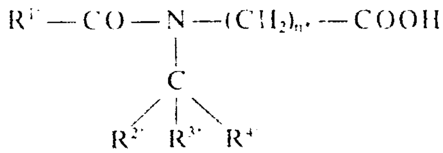

(I*)

worin

$R^{1*}$ $C_1-C_5$ Alkyl, $C_2-C_5$ Alkenyl, $C_2-C_5$ Alkinyl, $C_5-C_7$ Cycloalkyl oder Phenyl der allgemeinen Formel

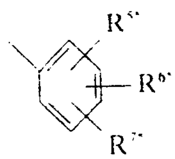

$R^{2*}$ Wasserstoff, $C_1-C_5$ Alkyl, $C_2-C_5$ Alkenyl oder $C_2-C_5$ Alkinyl,
$R^{3*}$ Wasserstoff, oder $C_1-C_5$ Alkyl und
$R^{4*}$ $C_1-C_5$ Alkyl bedeutet, wobei die Summe der Kohlenstoffatome der Alkylsubstituenten $R^{2*}$, $R^{3*}$ und $R^{4*}$ wenigstens 3 beträgt und wobei die Substituenten $R^{2*}$, $R^{3*}$ und $R^{4*}$ gemeinsam mit dem Kohlenstoffatom, an das sie gebunden sind, kein geradkettiges oder verzweigtes Alkyl oder Alkenyl darstellen, wenn $R^{1*}$ Alkyl oder Alkenyl ist und $n^*$ 3 bedeutet,
$n^*$ 3, 4 oder 5 bedeutet,
$R^{5*}$, $R^{6*}$ und $R^{7*}$ gleich oder verschieden sind und Wasserstoff, Halogen, $C_1-C_4$ Alkyl, $C_1-C_4$ Alkoxy, $C_2-C_5$ Alkanoyloxy, Amino, Nitro, Hydroxy oder Trifluormethyl bedeuten,

und ihre Salze mit anorganischen und organischen Basen.

Bevorzugte Vertreter der Ausgestaltung I* sind solche, in denen $R^{1*}$ $C_1-C_5$ Alkyl, $C_2-C_5$ Alkenyl oder Alkinyl oder durch $R^{5*}$, $R^{6*}$ und $R^{7*}$ substituiertes Phenyl, $R^{2*}$ Wasserstoff, $C_1-C_4$ Alkyl oder Ethinyl, $R^{3*}$ Wasserstoff oder $C_1-C_3$ Alkyl, $R^{4*}$ $C_1-C_5$ Alkyl (wobei die Summe der Kohlenstoffatome der Alkylsubstituenten $R^{2*}$, $R^{3*}$ und $R^{4*}$ wenigstens 3 beträgt und wobei die Substituenten $R^{2*}$, $R^{3*}$ und $R^{4*}$ gemeinsam mit dem Kohlenstoffatom, an das sie gebunden sind, kein geradkettiges oder verzweigtes Alkyl oder Alkenyl darstellen, wenn $R^{1*}$ Alkyl oder Alkenyl ist und $n^*$ 3 bedeutet), $R^{5*}$ Wasserstoff und $n^*$ 3, 4 oder 5 bedeuten, $R^{6*}$ und $R^{7*}$ gleich oder verschieden sind und Wasserstoff, Halogen, Methyl, Methoxy, Amino oder Trifluormethyl darstellen, und ihre Salze mit anorganischen oder organischen Basen.

Besonders bevorzugte Vertreter der Ausgestaltung I* sind solche, in denen $R^{1*}$ $C_1-C_5$ Alkyl, $C_2-C_5$ Alkenyl oder Alkinyl oder durch $R^{5*}$, $R^{6*}$ und $R^{7*}$ substituiertes Phenyl, $R^{2*}$ Wasserstoff, Methyl oder Ethinyl, $R^{3*}$ Wasserstoff oder Methyl, $R^{4*}$ $C_1-C_5$ Alkyl, (wobei die Summe der Kohlenstoffatome der Alkylsubstituenten $R^{2*}$, $R^{3*}$ und $R^{4*}$ wenigstens 3 beträgt und wobei die Substituenten $R^{2*}$, $R^{3*}$ und $R^{4*}$ gemeinsam mit dem Kohlenstoffatom, an das sie gebunden sind, kein geradkettiges oder verzweigtes Alkyl oder Alkenyl darstellen, wenn $R^{1*}$ Alkyl oder Alkenyl ist und $n^*$ 3 bedeutet), $R^{5*}$ Wasserstoff, $R^{6*}$ Wasserstoff, Chlor, Methoxy oder Trifluormethyl, $R^7$ Wasserstoff, Chlor oder Methoxy, $n^*$ 4 oder 5, vorzugsweise 3 bedeuten, und ihre Salze mit anorganischen oder organischen Basen.

Eine andere Ausgestaltung der Erfindung sind Acylhydrocarbylaminoalkansäuren der allgemeinen Formel

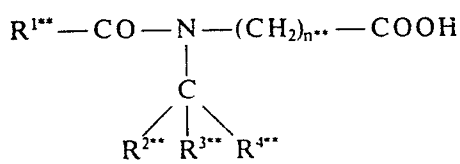

(I**)

worin

$R^{1**}$ die Bedeutung von $R^{1*}$ hat,
$R^{2**}$ die Bedeutung von $R^{2*}$ hat,
$R^{3**}$ Wasserstoff, $C_1-C_5$ Alkyl oder $C_5-C_7$ Cycloalkyl,
$R^{4**}$ $C_5-C_7$ Cycloalkyl oder
$R^{3**}$ und $R^{4**}$ gemeinsam Alkylen $-(CH_2)_{q^{**}}-$
oder
$R^{2**}$, $R^{3**}$ und $R^{4**}$ unter Einschluß des benachbarten Kohlenstoffatoms Adamantyl,
$n^{**}$ 3, 4 oder 5 und
$q^{**}$ 4, 5, 6 oder 7 bedeuten,
$R^{5**}$, $R^{6**}$ und $R^{7**}$ gleich oder verschieden sind und Wasserstoff, Halogen, $C_1-C_4$ Alkyl, $C_1-C_4$ Alkoxy, $C_2-C_5$ Alkanoyloxy, Amino, Nitro, Hydroxy oder Trifluormethyl bedeuten,

und ihre Salze mit anorganischen und organischen Basen.

Bevorzugte Vertreter der Ausgestaltung I** sind solche, in denen $R^{1**}$ $C_1-C_5$ Alkyl, $C_2-C_5$ Alkenyl oder Alkinyl oder durch $R^{5**}$, $R^{6**}$ und $R^{7**}$ substituiertes Phenyl, $R^{2**}$ Wasserstoff, $C_1-C_4$ Alkyl oder Ethinyl, $R^{3**}$ Wasserstoff, Methyl oder Cyclohexyl, $R^{4**}$ Cyclohexyl oder $R^{3**}$ und $R^{4**}$ gemeinsam Alkylen $-(CH_2)_{q^{**}}$ oder $R^{2**}$, $R^{3**}$ und $R^{4**}$ unter Einschluß des benachbarten Kohlenstoffatoms Adamantyl-(1), $n^{**}$ 3, 4 oder 5, $q^{**}$ 4, 5, 6 oder 7, $R^{5**}$ Wasserstoff bedeuten, $R^{6**}$ und $R^{7**}$ gleich oder verschieden sind und Wasserstoff, Halogen, Methyl, Methoxy, Amino oder Trifluormethyl darstellen, und ihre Salze mit anorganischen oder organischen Basen.

Besonders bevorzugte Vertreter der Ausgestaltung I** sind solche, in denen $R^{1**}$ $C_1-C_5$ Alkyl, $C_2-C_5$ Alkenyl oder Alkinyl oder durch $R^{5**}$, $R^{6**}$ und $R^{7**}$ substituiertes Phenyl, $R^{2**}$ Wasserstoff, Methyl oder Ethinyl, $R^{3**}$ und $R^{4**}$ gemeinsam Alkylen $-(CH_2)_{q^{**}}$ oder $R^{2**}$, $R^{3**}$ und $R^{4**}$ unter Einschluß des benachbarten Kohlenstoffatoms Adamantyl-(1), $n^{**}$ 4 oder 5, vorzugsweise 3, $q^{**}$ 5 oder 7, $R^{5**}$ Wasserstoff, $R^{6**}$ Wasserstoff, Chlor, Methoxy oder Trifluormethyl, $R^{7**}$ Wasserstoff, Chlor oder Methoxy bedeuten, und ihre Salze mit anorganischen und organischen Basen.

Eine weitere Ausgestaltung der Erfindung sind Acylhydrocarbylaminoalkansäuren der allgemeinen Formel

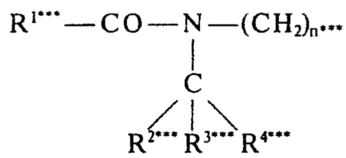

(I***)

worin

$R^{1***}$ die Bedeutung von $R^{1*}$ hat,
$R^{2***}$ Wasserstoff oder $C_1-C_5$ Alkyl,
$R^{3***}$ Wasserstoff, $C_1-C_5$ Alkyl oder Phenyl der allgemeinen Formel

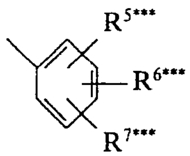

$R^{4***}$ Phenyl der allgemeinen Formel

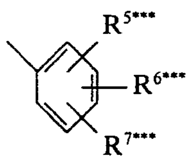

oder Phenylalkyl der allgemeinen Formel

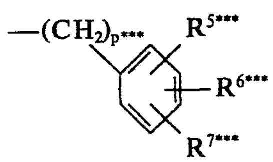

$n^{***}$ 3, 4 oder 5 und
$p^{***}$ 1, 2, 3 oder 4 bedeuten,
$R^{5***}$, $R^{6***}$ und $R^{7***}$ gleich oder verschieden sind und Wasserstoff, Halogen, $C_1-C_4$ Alkyl, $C_1-C_4$ Alkoxy, $C_2-C_5$ Alkanoyloxy, Amino, Nitro, Hydroxy oder Trifluormethyl bedeuten, wobei $R^{5***}$, $R^{6***}$ und $R^{7***}$ nicht gleichzeitig Wasserstoff bedeuten wenn $R^{1***}$ und $R^{4***}$ einen Phenylrest und $R^{2***}$ und $R^{3***}$ ein Wasserstoffatom darstellen,

und ihre Salze mit anorganischen und organischen Basen.

Bevorzugte Vertreter der Ausgestaltung I*** sind solche, in denen $R^{1***}$ $C_1-C_5$ Alkyl, $C_2-C_5$ Alkenyl oder Alkinyl oder durch $R^{5***}$, $R^{6***}$ und $R^{7***}$ substituiertes Phenyl, $R^{2***}$ Wasserstoff oder $C_1-C_4$ Alkyl, $R^{3***}$ Wasserstoff, $C_1-C_3$ Alkyl oder durch $R^{7***}$ substituiertes Phenyl, $R^{4***}$ durch $R^{7***}$ substituiertes Phenyl oder durch $R^{7***}$ substituiertes Benzyl, $R^{5***}$ Wasserstoff und $n^{***}$ 3, 4 oder 5 bedeuten, $R^{6***}$ und $R^{7***}$ gleich oder verschieden sind und Wasserstoff, Halogen, Methyl, Methoxy, Amino oder Trifluormethyl darstellen, wobei $R^{5***}$, $R^{6***}$ und $R^{7***}$ nicht gleichzeitig Wasserstoff bedeuten wenn $R^{1***}$ und $R^{4***}$ einen Phenylrest und $R^{2***}$ und $R^{3***}$ ein Wasserstoffatom darstellen, und ihre Salze mit anorganischen und organischen Basen.

Besonders bevorzugte Vertreter der Ausgestaltung I*** sind solche, in denen $R^{1***}$ $C_1-C_5$ Alkyl, $C_2-C_5$ Alkenyl oder Alkinyl oder durch $R^{5***}$, $R^{6***}$ und $R^{7***}$ substituiertes Phenyl, $R^{2***}$ Wasserstoff, $R^{3***}$ Wasserstoff, Methyl oder durch $R^{7***}$ substituiertes Phenyl, $R^{4***}$ durch $R^{7***}$ substituiertes Phenyl oder durch $R^{7***}$ substituiertes Benzyl, $R^{5***}$ Wasserstoff, $R^{6***}$ Wasserstoff, Chlor, Methoxy oder Trifluormethyl, $R^{7***}$ Wasserstoff, Chlor oder Methoxy darstellen, wobei $R^{5***}$, $R^{6***}$ und $R^{7***}$ nicht gleichzeitig Wasserstoff bedeuten wenn $R^{1***}$ und $R^{4***}$ einen Phenylrest und $R^{2***}$ und $R^{3***}$ ein Wasserstoffatom darstellen, n*** 4 oder 5, vorzugsweise 3 bedeutet, und ihre Salze mit anorganischen oder organischen Basen.

Eine alternative Ausgestaltung der Erfindung sind Acylhydrocarbylaminoalkansäuren der allgemeinen Formel

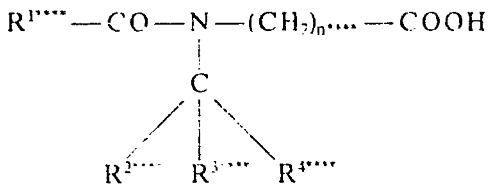

(I****)

worin

$R^{1****}$ die Bedeutung von $R^{1*}$ hat,
$R^{2****}$ $C_2-C_5$ Alkenyl oder $C_2-C_5$ Alkinyl,
$R^{3****}$ Wasserstoff oder $C_1-C_5$ Alkyl,
$R^{4****}$ Phenyl der allgemeinen Formel

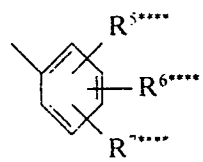

**oder Phenylalkyl der allgemeinen Formel**

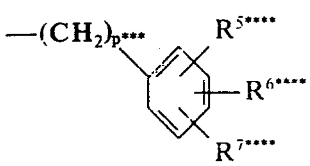

n**** 3, 4 oder 5 und
p**** 1, 2, 3 oder 4 bedeuten,
$R^{5****}$, $R^{6****}$ und $R^{7****}$ gleich oder verschieden sind und Wasserstoff, Halogen, $C_1-C_4$ Alkyl, $C_1-C_4$ Alkoxy, $C_2-C_5$ Alkanoyloxy, Amino, Nitro, Hydroxy oder Trifluormethyl bedeuten,

und ihre Salze mit anorganischen oder organischen Basen.

Bevorzugte Vertreter der Ausgestaltung I**** sind solche, in denen $R^{1****}$ $C_1-C_5$ Alkyl, $C_2-C_5$ Alkenyl oder Alkinyl, oder durch $R^{5****}$, $R^{6****}$ und $R^{7****}$ substituiertes Phenyl, $R^{2****}$ Ethinyl, $R^{3****}$ Wasserstoff oder $C_1-C_3$ Alkyl, $R^{4****}$ durch $R^{7****}$ substituiertes Phenyl oder durch $R^{7****}$ substituiertes Benzyl, $R^{5****}$ Wasserstoff und n**** 3, 4 oder 5 bedeuten, $R^{6****}$ und $R^{7****}$ gleich oder verschieden sind und Wasserstoff, Halogen, Methyl, Methoxy, Amino oder Trifluormethyl darstellen, und ihre Salze mit anorganischen und organischen Basen.

Besonders bevorzugte Vertreter der Ausgestaltung I**** sind solche, in denen $R^1$ $C_1-C_5$ Alkyl, $C_2-C_5$ Alkenyl oder Alkinyl oder durch $R^{5****}$, $R^{6****}$ und $R^{7****}$ substituiertes Phenyl, $R^{2****}$ Ethinyl, $R^{3****}$ Wasserstoff oder Methyl, $R^{4****}$ durch $R^{7****}$ substituiertes Phenyl oder durch $R^{7****}$ substituiertes Benzyl, $R^{5****}$ Wasserstoff, $R^{6****}$ Wasserstoff, Chlor, Methoxy oder Trifluormethyl, $R^{7****}$ Wasserstoff, Chlor oder Methoxy, n**** 4 oder 5, vorzugsweise 3, bedeuten, und ihre Salze mit anorganischen und organischen Basen.

**Die Ausgestaltung I*** ist gegenüber den übrigen Ausgestaltungen bevorzugt. Die Ausgestaltungen I* und I** sind gegenüber der Ausgestaltung I**** bevorzugt.**

**Als durch die allgemeine Formel I erfaßte Verbindungen seien beispielsweise genannt**

N-(2,6-Dimethylbenzoyl)-4-[(3-ethyl-1-pentin-3-yl)-amino]-buttersäure,
N-(3,4-Dichlorbenzoyl)-5-[(1-ethinyl-cyclohexyl-1)-amino]-valeriansäure,
N-(3-Chlor-4-fluorbenzoyl)-4-[(1-n-butyl-cyclopentyl-1)-amino]-buttersäure,
N-Isobutyryl-4-(tert.-butylamino)-buttersäure,
N-Propionyl-6-[2-(3,4-dimethoxyphenyl)-ethyl-amino]-capronsäure,
N-n-Hexanoyl-4-[adamantyl-(1)-amino]-buttersäure,
N-n-Heptanoyl-4-(n-butylamino)-buttersäure,
N-Crotonoyl-4-[(1-propyl-cyclohexyl-1)-amino]-buttersäure,
N-(3-Nitrobenzoyl)-4-[(4-chlorbenzyl)-amino]-buttersäure,
N-(2-Acetoxybenzoyl)-4-[(1,1,3,3-tetramethylbutyl)-amino]-buttersäure,
N-(2-Brombenzoyl)-4-[(3-ethyl-1-pentin-3-yl)-amino]-valeriansäure,
N-(3-Chlorbenzoyl)-4-[(1-ethinyl-cyclohexyl-1)-amino]-buttersäure,
N-(Cyclohexylcarbonyl)-4-[(1-n-butyl-cyclopentyl-1)-amino]-capronsäure,
N-(2-Chlorbenzoyl)-4-[(1-propyl-cyclohexyl-1)-amino]-buttersäure,
N-(2-Fluorbenzoyl)-4-[(α-methylbenzyl)-amino]-buttersäure,
N-(3-Nitrobenzoyl)-4-[(1,2-diphenylethyl)-amino]-buttersäure,
N-(4-Nitrobenzoyl)-4-[(3,4-dimethoxybenzyl)-amino]-buttersäure,
N-(3-Trifluormethylbenzoyl)-6-[(α-methylbenzyl)-amino]-capronsäure,
N-(3-Toluoyl)-4-[benzhydrylamino]-buttersäure,
N-Isovaleroyl-5-[(α-methylbenzyl)-amino]-valeriansäure.

Bevorzugte erfindungsgemäße Verbindungen sind

N-p-Chlorbenzoyl-4-(1-phenylethyl-amino)-buttersäure,
N-Acetyl-4-benzhydrylamino-buttersäure,
N-p-Chlorbenzoyl-4-benzhydrylamino-buttersäure,
N-p-Chlorbenzoyl-4-benzylamino-buttersäure und ihre Salze.

Die Acylhydrocarbylaminoalkansäuren der allgemeinen Formel I bzw. der Ausgestaltungen I*, I**, I*** und I**** besitzen an dem Kohlenstoffatom, an das $R^2$, $R^3$ und $R^4$ (bzw. die entsprechenden Substituenten der Ausgestaltungen) gebunden sind, ein Chiralitätszentrum, wenn $R^2$, $R^3$ und $R^4$ (bzw. die entsprechenden Substituenten der Ausgestaltungen) voneinander verschieden sind. Die Erfindung betrifft daher sowohl die Racemate als auch die Enantiomeren und ihre Gemische.

Die erfindungsgemäßen Verbindungen weisen wertvolle Eigenschaften auf, die sie gewerblich verwertbar machen. Sie entfalten an Warmblütlern eine Schutzwirkung für Magen und Leber, daneben bewirken sie eine Steigerung der Sekretion von Pankreas und Leber (Galle).

Aufgrund ihrer vorteilhaften Wirksamkeit sind die Acylhydrocarbylaminoalkansäuren zur Behandlung und Prophylaxe von Krankheiten, die auf Erkrankungen des Magens oder Darms oder auf Minderleistungen von Pankreas, Galle und/oder Leber beruhen, geeignet. Beispielsweise werden behandelt Magen- oder Darmulcera, Billroth II, Pankreas-Insuffizienz, Sprue, Maldigestionen und Malabsorptionen verschiedener Genese, akute und chronische Pankreatitis, indirekte Störungen der Pankreasfunktion (Unterstützung der Sekretin- und Pankreozymin-Produktion), daneben Gallenblasen- und Gallenwegsentzündungen, Gallenflußstörungen, Motilitätsstörungen der Gallenwege, Völlegefühl, Blähungen, Obstipationen, Oberbauchbeschwerden, hepato-biläre Funktionsstörungen, akute und chronische Hepatitis, Leberintoxikationen, Fettleber.

Ein weiterer Gegenstand der Erfindung sind Arzneimittel, die eine oder mehrere der Acylhydrocarbylaminoalkansäuren der allgemeinen Formel I

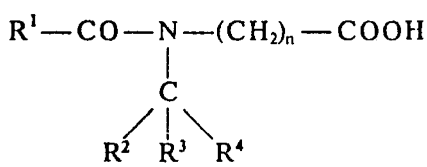

(I)

worin

$R^1$ $C_1$–$C_7$ Alkyl, $C_1$–$C_7$ Alkenyl, $C_2$–$C_7$ Alkinyl, $C_3$–$C_{10}$ Cycloalkyl oder Phenyl der allgemeinen Formel

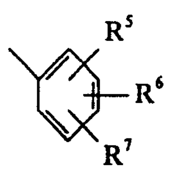

$R^2$ Wasserstoff, $C_1-C_7$ Alkyl, $C_2-C_7$ Alkenyl oder $C_2-C_7$ Alkinyl,
$R^3$ Wasserstoff, $C_1-C_7$ Alkyl, $C_3-C_{10}$ Cycloalkyl oder Phenyl der allgemeinen Formel

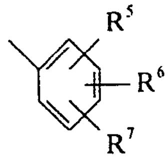

und
$R^4$ $C_1-C_7$ Alkyl, $C_3-C_{10}$ Cycloalkyl, Phenyl der allgemeinen Formel

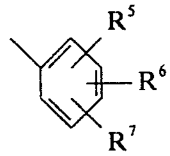

oder Phenylalkyl mit $C_1-C_4$ Alkyl und Phenyl der allgemeinen Formel

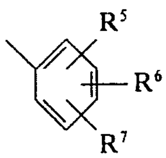

bedeuten, wobei die Summe der Kohlenstoffatome der Alkylsubstituenten $R^2$, $R^3$ und $R^4$ wenigstens 3 beträgt und wobei die Substituenten $R^2$, $R^3$ und $R^4$ gemeinsam mit dem Kohlenstoffatom, an das sie gebunden sind, kein geradkettiges oder verzweigtes Alkyl oder Alkenyl darstellen, wenn $R^1$ Alkyl oder Alkenyl ist und n 3 bedeutet, oder
$R^3$ und $R^4$ gemeinsam $C_2-C_8$ Alkylen oder
$R^2$, $R^3$ und $R^4$ unter Einschluß des benachbarten Kohlenstoffatoms Adamantyl,
n 3, 4 oder 5 bedeuten und
$R^5$, $R^6$ und $R^7$ gleich oder verschieden sind und Wasserstoff, Halogen, $C_1-C_4$ Alkyl, $C_1-C_4$ Alkoxy, $C_1-C_4$ Alkylmercapto, $C_2-C_5$ Alkanoyloxy, Amino, Nitro Trifluormethyl, Trifluormethoxy, Hydroxy oder Trifluormethylmercapto bedeuten, wobei $R^5$, $R^6$ und $R^7$ nicht gleichzeitig Wasserstoff bedeuten wenn $R^1$ und $R^4$ einen Phenylrest und $R^2$ und $R^3$ ein Wasserstoffatom darstellen,

und/oder ihre pharmakologisch verträglichen Salze mit anorganischen oder organischen Basen, enthalten.

Ausgestaltungen der Arzneimittel sind solche, die Acylhydrocarbylaminoalkansäuren der Formeln I*, I**, I***, I**** oder ihre bevorzugten Vertreter und/oder ihre pharmakologisch verträglichen Salze mit anorganischen oder organischen Basen enthalten.

Die Arzneimittel werden nach an sich bekannten Verfahren hergestellt. Als Arzneimittel können die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 1 bis 95, vorzugsweise 15 bis 85 Gewichtsprozent der Gesamtmischung.

Die Wirkstoffe können im humän- und veterinärmedizinischen Bereich in jeder beliebigen Form, z. B. systemisch, angewandt werden unter der Voraussetzung, daß die Ausbildung bzw. Aufrechterhaltung von ausreichenden Blut- oder Gewebsspiegeln oder Lokalkonzentrationen an Wirkstoffen gewährleistet ist. Das kann entweder durch orale, rektale oder parenterale Gabe in geeigneten Dosen erreicht werden. Vorteilhafterweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion, einer Suspension, eines Sols oder eines Gels sein.

Unter »Einheitsdosis« wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachem einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel der Einheitsdosis benötigt wird, ist die Einheitsosis vorteilhafterweise teilbar, z. B. in Form einer Tablette mit

Bruchkerbe.

Die pharmazeutischen Zubereitungen enthalten, wenn sie in Einheitsdosen vorliegen und für die Applikation z. B. am Menschen bestimmt sind, etwa 0,5 bis 1000 mg, vorteilhafterweise 1 bis 500 mg und insbesondere 5 bis 400 mg Wirkstoff.

Im allgemeinen hat es sich sowohl in der Human- als auch in der Veterinärmedizin als vorteilhaft erwiesen, den oder die Wirkstoffe bei oraler Gabe in einer Tagesdosis von etwa 0,01 bis etwa 40, vorzugsweise 0,1 bis 30, insbesondere 0,2 bis 20 mg/kg Körpergewicht, gegebenenfalls in Form mehrerer, vorzugsweise 2 bis 3 Einzelgaben zur Erzielung der gewünschten Ergebnisse zu verabreichen. Eine Einzelgabe enthält den oder die Wirkstoffe in Mengen von etwa 0,01 bis etwa 20, vorzugsweise 0,1 bis 15, insbesondere 0,2 bis 10 mg/kg Körpergewicht.

Bei einer parenteralen Behandlung, z. B. einer intramuskulären oder intravenöser Applikation, können ähnliche Dosierungen zur Anwendung kommen. Bei dieser Therapie werden etwa 50 bis 1000 mg Wirkstoff appliziert.

Die therapeutische Verabreichung der pharmazeutischen Zubereitung erfolgt bei Dauermedikation im allgemeinen zu festgelegten Zeitpunkten, wie 1 bis 4 mal am Tage, z. B. jeweils nach den Mahlzeiten und/oder am Abend. Bei akuten Anlässen erfolgt die Medikation zu variierendem Zeitpunkt. Unter bestimmten Gegebenheiten kann es erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben ausgeführte Wirkstoffmenge überschritten werden muß. Die Festlegung der jeweils erforderlichen optimalen Dosierung und Applikationsart der Wirkstoffe kann der Fachmann aufgrund seines Fachwissens jederzeit vornehmen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z. B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z. B. Tabletten, Dragées, harte und weiche Kapseln, z. B. aus Gelatine, dispergierbare Pulver, Granulate, wäßrige und ölige Suspensionen, Emulsionen, Lösungen oder Sirupe kommen.

Tabletten können inerte Verdünnungsmittel, z. B. Calciumcarbonat, Calciumphosphat, Natriumphosphat oder Lactose; Granulierungs- und Verteilungsmittel, z. B. Maisstärke oder Alginate; Bindemittel, z. B. Stärke, Gelatine oder Akaziengummi; und Gleitmittel, z. B. Aluminium- oder Magnesiumstearat, Talkum oder Silikonöl, enthalten. Sie können zusätzlich mit einem Überzug versehen sein, der auch so beschaffen sein kann, daß eine verzögerte Auflösung und Resorption des Arzneimittels im Gastrointestinaltrakt und damit z. B. eine verbesserte Verträglichkeit, Protrahierung oder eine Retardierung erreicht wird. Gelatinekapseln können den Arzneistoff vermischt mit einem festen, z. B. Calciumcarbonat oder Kaolin, oder einem öligen, z. B. Olivenöl-, Erdnuß- oder Paraffinöl, Verdünnungsmittel enthalten.

Wäßrige Suspensionen können Suspendiermittel, z. B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z. B. Polyoxyethylenstearat, Heptadecaethylenoxycetanol, Polyoxyethylensorbitolmonooleat, Polyoxyethylensorbitanmonooleat oder Lecithin; Konservierungsmittel, z. B. Methyl- oder Propylhydroxybenzoate; Geschmacksmittel; Süßungsmittel, z. B. Saccharose, Lactose, Natriumcyclamat, Dextrose, Invertzuckersirup, enthalten.

Ölige Suspensionen können z. B. Erdnuß-, Oliven-, Sesam-, Kokos- oder Paraffinöl und Verdickungsmittel, wie z. B. Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischung mit Dispergier-, Benetzungs- und Suspendiermitteln, z. B. den obengenannten, sowie mit Süßungsmitteln, Geschmacksmitteln und Farbstoffen enthalten.

Emulsionen können z. B. Oliven-, Erdnuß- oder Paraffinöl neben Emulgiermitteln, wie z. B. Akaziengummi, Traganthgummi, Phosphatiden, Sorbitanmonooleat, Polyoxyethylensorbitanmonooleat, und Süßungs- und Geschmacksmitteln enthalten.

Zur rektalen Anwendung der Arzneistoffe gelangen Suppositorien, die mit Hilfe von bei Rektaltemperatur schmelzenden Bindemitteln, beispielsweise Kakaobutter oder Polyethylenglykolen, hergestellt werden.

Zur parenteralen Anwendung der Arzneistoffe dienen steril injizierbare wäßrige Suspensionen, isotonische Salzlösungen oder sonstige Lösungen, die Dispergier- oder Benetzungsmittel und/oder pharmakologisch verträgliche Verdünnungsmittel, z. B. Propylen- oder Butylenglykol, enthalten können.

Der oder die Wirkstoffe können gegebenenfalls mit einem oder mehreren der angegebenen Träger-oder Zusatzstoffe auch in mikroverkapselter Form formuliert werden.

Neben den erfindungsgemäßen Acylhydrocarbylaminoalkansäuren und/oder ihren Salzen können die pharmazeutischen Zubereitungen einen oder mehrere andere pharmakologisch aktive Bestandteile anderer Arzneimittelgruppen, wie Antacida, beispielsweise Aluminiumhydroxid, Magnesiumaluminat; Tranquilizer, wie Benzodiazepine, beispielsweise Diazepam; Spasmolytika, wie z. B. Bietamiverin, Camylofin, Anticholinergika, wie z. B. Oxyphencyclimin, Phencarbamid; Entschäumungsmittel, beispielsweise Dimethylpolysiloxan; Laxantien, beispielsweise Bisacodyl, Quellmittel; gegebenenfalls auch Fermente, Gallensäuren, Antibiotika, Vitamine, Aminosäuren, Fettsäurengemische etc. enthalten.

Ein weiterer Gegenstand der Erfindung ist ein Verfahren zur Herstellung der Acylhydrocarbylaminoalkansäuren der allgemeinen Formel I sowie ihrer Salze mit anorganischen oder organischen Basen, das dadurch gekennzeichnet ist, daß man

a) eine Hydrocarbylaminoalkansäure der allgemeinen Formel II

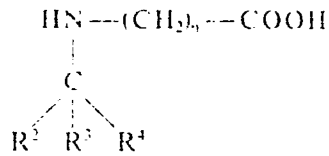

(II)

worin $R^2$, $R^3$, $R^4$, und n die oben angegebene Bedeutung haben, gegebenenfalls unter Schutz der Carboxylgruppe, mit einem Acylderivat der allgemeinen Formel III

$$R^1—CO—R^8 \qquad (III)$$

worin $R^8$ eine Fluchtgruppe (= Abgangsgruppe) oder eine $R^1-CO-O$-Gruppe darstellt und $R^1$ die oben angegebene Bedeutung hat, acyliert und gegebenenfalls anschließend in die Salze überführt oder

b) eine Hydrocarbylaminoalkensäure der allgemeinen Formel IV

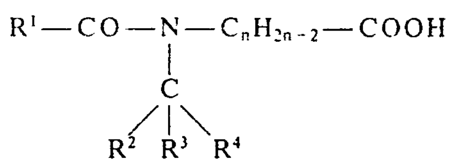

(IV)

worin $R^1$, $R^2$, $R^3$, $R^4$ und n die oben angegebene Bedeutung haben, gegebenenfalls unter Schutz der Carboxylgruppe, hydriert und gegebenenfalls anschließend in die Salze überführt oder

c) eine Acylaminoalkansäure der allgemeinen Formel V

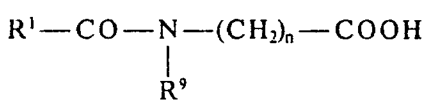

(V)

worin $R^1$ und n die oben angegebene Bedeutung haben und $R^9$ ein Wasserstoffatom oder ein Metallatom eines Erdalkalimetalls, vorzugsweise eines Alkalimetalls, bedeutet, gegebenenfalls unter Schutz der Carboxylgruppe, mit einem Hydrocarbylderivat der allgemeinen Formel VI

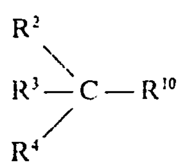

(VI)

worin $R^2$, $R^3$ und $R^4$ die oben angegebene Bedeutung haben und $R^{10}$ eine Fluchtgruppe darstellt, umsetzt und gegebenenfalls anschließend in die Salze überführt oder

d) eine funktionelles Acylhydrocarbylaminoalkansäurederivat der allgemeinen Formel VII

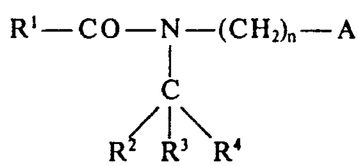

(VII)

worin $R^1$, $R^2$, $R^3$, $R^4$ und n die oben angegebene Bedeutung haben und A ein funktionelles Derivat einer Carboxylgruppe bedeutet, solvolysiert und gegebenenfalls anschließend in die Salze überführt.

Werden die Hydrocarbylaminoalkansäuren der Formel II unter Schutz der Carboxylgruppe zur Reaktion gebracht, so gelangen solche Vertreter zum Einsatz, deren Schutzgruppen nicht mit den Acylderivaten III reagieren. Geeignete Vertreter sind beispielsweise Salze anorganischer oder organischer Basen, wie Alkali- oder Erdalkalimetallsalze, Ammoniumsalze, Salze mit tertiären Basen (z. B. Triethylamin, Pyridin) oder quartiäre Ammoniumsalze, oder Ester von Alkanolen oder Phenalkanolen, wie Methyl-, Propyl-, Butyl-, Benzyl- oder Phenethylester.

In den Acylderivaten III ist eine Fluchtgruppe $R^8$ beispielsweise eine Hydroxygruppe, ein Halogenatom, vorzugsweise ein Chlor- oder Bromatom, eine Alkylsulfonyloxy- oder Benzolsulfonyloxygruppe, wie eine Mesyloxy- oder p-Tolylsulfonyloxygruppe, eine Alkoxygruppe, vorzugsweise eine Methoxy- oder Ethoxygruppe, eine Alkylmercaptogruppe, wie eine Methylmercapto- oder Ethylmercaptogruppe.

Die Reaktion der Hydrocarbylaminoalkansäuren II mit den Acylderivaten III wird nach an sich bekannten Verfahren durchgeführt. Die Umsetzung wird in geeigneten Lösungsmitteln, wie Wasser oder Kohlenwasserstoffen, z. B. Benzol, Toluol, Xylol, oder Ethern, z. B. Tetrahydrofuran, Dioxan, 1,2-Dimethoxythan, oder Ketonen, z. B. Ethylmethylketon, oder Amiden, z. B. Dimethylformamid, oder Sulfoxiden, z. B. Dimethylsulfoxid, durchgeführt. Zweckmäßigerweise wird die Acylierung, wenn $R^8$ eine Halogenfluchtgruppe bedeutet, in Gegenwart eines säurebindenden Mittels (Protonenacceptors) vorgenommen. Als solche sind beispielsweise geeignet Alkalimetallhydroxide, wie Natriumhydroxid, Kaliumhydroxid, oder Alkalimetallcarbonate, wie Natriumcarbonat, Kaliumcarbonat, oder tertiäre Amine, wie Pyridin, Triethylamin, Ethyldiisopropylamin. Stellen die Acylderivate III Säureanhydride dar, d. h. bedeutet $R^8$ eine $R^1$–CO–O-Gruppe, so genügt auch das Erhitzen der Verbindungen II und III in einem inerten Lösungsmittel.

Die Reaktionstemperatur kann in weiten Grenzen variiert werden, z. B. – 20 bis + 100°C, wobei Temperaturen um Raumtemperatur (10 bis 30°C) bevorzugt sind. Werden die Verbindungen II unter Schutz der Carboxylgruppe acyliert, so wird nach der Acylierung die Schutzgruppe in üblicher Weise wieder abgespalten. Werden Salze als Schutzgruppe eingesetzt, so erfolgt die Freisetzung der erhaltenen Säuren I durch Umsetzung mit einer geeigneten Mineralsäure, wie Salzsäure, Schwefelsäure etc. Werden Ester als Schutzgruppen eingesetzt, so erfolgt auf die Acylierung die Verseifung des Reaktionsproduktes zu Verbindungen der Formel I. Die Verseifung wird vorzugsweise mit einer alkoholischen (z. B. ethanolischen) Alkalimetallhydroxid-(z. B. Kaliumhydroxid-)Lösung bei Raumtemperatur vorgenommen, gegebenenfalls unter Zusatz eines inerten Verdünnungsmittels, wie Dioxan oder Benzol.

Die Ausgangsverbindungen der Formel II werden nach verschiedenen an sich bekannten Verfahren hergestellt. So werden sie durch Umsetzung von Halogenalkansäuren der Formel VIII, gegebenenfalls unter Schutz der Carboxylgruppe als Estergruppe, mit einem primären Amin der Formel IX

$$R^{11}—(CH_2)_n—COOH$$

(VIII)

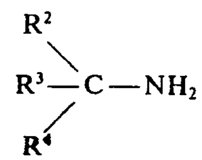

(IX)

worin n, $R^2$, $R^3$ und $R^4$ die oben angegebene Bedeutung haben und $R^{11}$ ein Halogenatom, vorzugsweise ein Chlor- oder Bromatom, darstellt, erhalten. Die Reaktion wird zweckmäßig in einem inerten Lösungsmittel, z. B. Benzol, Cyclohexan, Diethylether, unter Zusatz eines Protonenacceptors durchgeführt. Als solcher dient vorzugsweise ein Überschuß des zur Reaktion eingesetzten Amins IX, welches auch als Lösungsmittel dienen kann. Gewünschtenfalls kann jedoch auch ein anderer Protonenacceptor hinzugefügt werden.

Die Vorprodukte II werden auch durch Umsetzung einer Aminosäure X

$$H_2N—(CH_2)_n—COOH \qquad (X)$$

worin n die oben angegebene Bedeutung hat, vorzugsweise unter Schutz der Carboxylgruppe, mit einem Hydrocarbylderivat VI erhalten. Geeignete Fluchtgruppen $R^{10}$ sind Halogenatome, vorzugsweise Chlor- oder Bromatome, Trichlormethyl- oder Tribrommethylgruppen.

Die Reaktion wird zweckmäßig in Gegenwart eines inerten Lösungsmittels, wie Kohlenwasserstoffen, z. B. Benzol, Toluol, Xylol, oder Ethern, z. B. Tetrahydrofuran, Dioxan, 1,2-Dimethoxyethan, oder Ketonen, z. B. Ethylmethylketon, oder Amiden, z. B. Dimethylformamid, oder Sulfoxiden, z. B. Dimethylsulfoxid, durchgeführt. Zweckmäßigerweise wird ein Protonenacceptor eingesetzt. Als solche sind beispielsweise geeignet Alkalimetallhydroxide, wie Natriumhydroxid, Kaliumhydroxid oder Alkalimetallcarbonate, wie Natriumcarbonat, Kaliumcarbonat, oder tertiäre Amine, wie Pyridin, Triethylamin, Ethyldiisopropylamin. Erfolgt die Herstellung von II aus Aminosäuren X, deren Carboxylgruppe geschützt ist,

so wird die Schutzgruppe zweckmäßigerweise erst nach Umsetzung der in geschützter Form erhaltenen Zwischenprodukte II mit den Acylderivaten III unter Erhalt der Endprodukte I abgespalten.

Die Ausgangsverbindungen II werden ferner durch Solvolyse von funktionellen Hydrocarbylaminoalkansäurederivaten der allgemeinen Formel XI

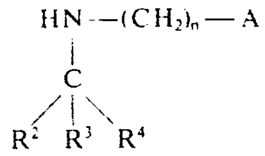

(XI)

worin n, $R^2$, $R^3$, $R^4$ und A die oben angegebene Bedeutung haben, nach dem Fachmann bekannten Verfahren erhalten. Zweckmäßige Ausführungsformen werden unter der Verfahrensvariante d) beschrieben.

Die Vorprodukte II werden weiterhin durch Solvolyse von Lactamen der allgemeinen Formel XII

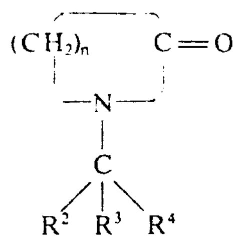

(XII)

worin $R^2$, $R^3$, $R^4$ und n die oben angegebene Bedeutung haben, in an sich bekannter Weise erhalten. Die Solvolyse erfolgt beispielsweise als Hydrolyse durch Erhitzen von XII auf Temperaturen von 80 bis 110°C mit wäßrigen oder wäßrig-alkoholischen Alkalimetallhydroxid-(z. B. Natriumhydroxid-)Lösungen, oder als Alkoholyse durch Erhitzen von XII mit Alkoholen, wie Methanol, Ethanol, in Gegenwart von Mineralsäuren, wie Schwefelsäure.

Die Zwischenprodukte II werden alternativ durch Hydrierung von Hydrocarbyliminoalkansäuren XIII oder Alkylidenaminoalkansäuren XIV

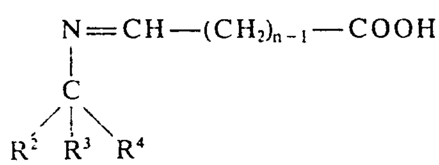

(XIII)

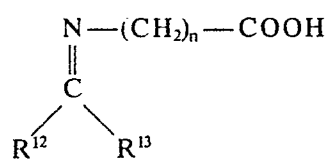

(XIV)

worin $R^2$, $R^3$, $R^4$ und n die oben angegebene Bedeutung haben und $R^{12}$ ein Wasserstoffatom, eine Alkylgruppe oder einen gegebenenfalls substituierten Phenylrest und $R^{13}$ eine Alkylgruppe, eine Alkinylgruppe, eine gegebenenfalls substituierte Phenylgruppe oder eine gegebenenfalls substituierte Phenylalkylgruppe oder $R^{12}$ und $R^{13}$ unter Einschluß des benachbarten Kohlenstoffatoms eine Cycloalkylgruppe darstellen, gegebenenfalls unter Schutz der Carboxylgruppe, in an sich bekannter Weise erhalten. Die Hydrierung erfolgt beispielsweise mit Raney-Nickel unter Wasserstoffdrücken von 1 bis 250 atm bei Raumtemperatur in abs. Ethanol.

Die Säuren XIII sind durch Umsetzung der Amine IX mit Oxosäureestern XV

$$O{=}CH{-}(CH_2)_{n-1}{-}CO{-}O{-}R^{14} \qquad (XV)$$

worin n die oben angegebene Bedeutung hat und $R^{14}$ eine Alkylgruppe mit 1 bis 5 Kohlenstoffatomen oder eine Benzylgruppe darstellt, zugänglich. Die Säuren XIV werden durch Umsetzung der Aminosäuren X, gegebenenfalls unter Schutz der Carboxylgruppe, mit den Oxoverbindungen XVI

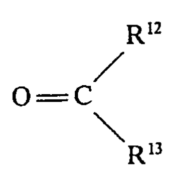

(XVI)

worin $R^{12}$ und $R^{13}$ die oben angegebene Bedeutung haben, erhalten.

Die Hydrierung gemäß Verfahrensvariante b) erfolgt nach Methoden, wie sie dem Fachmann bekannt

sind. So werden die Hydrocarbylaminoalkensäuren IV mit Wasserstoff in Gegenwart eines Übergangsmetall- oder Edelmetall-Katalysators oder der entsprechenden Oxide oder Komplexe in inerten Lösungsmitteln hydriert. Geeignete Metalle sind z. B. Platin, Palladium, Iridium, Rhodium. Eine Übersicht über die Hydrierungsverfahren findet sich u. a. in Kirk-Othmer 11, 418–462; Ullmann 10, 109–114, 541–555; 14, 630–649. Die Abspaltung einer gegebenenfalls vorhandenen Schutzgruppe erfolgt in üblicher Weise.

Die Alkensäuren IV werden beispielsweise aus den Halogenalkensäureestern XVII

$$R^{15}—C_nH_{2n-2}—CO—O—R^{14} \qquad (XVII)$$

worin $R^{14}$ und n die oben angegebene Bedeutung haben und $R^{15}$ ein Halogenatom, vorzugsweise ein Bromatom dedeutet, durch Aminierung mit einem Amin der allgemeinen Formel IX, Acylierung mit einem Acylderivat III und gegebenenfalls anschließende Verseifung gewonnen. Die Herstellung erfolgt nach an sich bekannten Verfahren, Halogenierung und Aminierung z. B. analog J. Heterocycl. Chem. 8 (1971) 21; Acylierung und Verseifung werden u. a. in der vorliegenden Anmeldung beschrieben.

Die Hydrocarbylierung der Acylaminoalkansäuren V (Verfahrensvariante c) erfolgt in üblicher Weise. Beispielsweise werden die Verbindungen V in einem geeigneten inerten, wasserfreien Lösungsmittel, wie Benzol, Toluol, Xylol, Tetrahydrofuran, Dimethylglykol, Dimethylformamid, Dimethylsulfoxid, mit einem Alkalimetallhydrid oder -amid, wie Natriumhydrid oder -amid, deprotoniert und dann mit dem Hydrocarbylderivat VI, worin $R^{10}$ ein Halogenatom, vorzugsweise ein Chlor- oder Bromatom, eine Alkylsulfonyloxy- oder Benzolsulfonyloxygruppe, wie eine Mesyloxy- oder p-Tolylsulfonyloxygruppe, bedeutet, behandelt. Bei Verwendung von Dimethylsulfoxid kann auch Kaliumhydroxid als Deprotonierungsmittel eingesetzt werden (vgl. Isele u. Lüttringhaus, Synthesis 1971, 266).

Die Halogenalkansäuren VIII und Lactame XII sind bekannte Verbindungen oder werden nach Analogierverfahren hergestellt; beispielsweise sind die Halogenalkansäuren VIII durch Solvolyse, wie Hydrolyse oder Alkoholyse, der entsprechenden Lactone und anschließende Halogenierung bzw. die Lactame XII durch N-Hydrocarbylierung entsprechender N-unsubstituierter Lactame zugänglich.

Die Solvolyse gemäß Verfahrensvariante d) erfolgt nach dem Fachmann bekannten Verfahren. Unter einem funktionellen Säurederivat wird hierbei ein Abkömmling verstanden, dessen funktionelle Gruppe A durch Solvolyse in die freie Carboxylgruppe überführbar ist. Typische Vertreter sind beispielsweise solche, worin A eine – CN-Gruppe oder eine

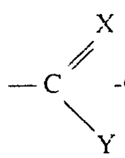 -Gruppe

bedeutet, worin

X ein Sauerstoff- oder ein Schwefelatom oder ein substituiertes Stickstoffatom, insbesondere eine Imino-, Alkylimino- oder Hydroxyiminogruppe darstellt und

Y eine Hydroxygruppe oder einen monovalenten eliminierbaren elektrophilen Rest, insbesondere eine freie oder substituierte Aminogruppe, vorzugsweise eine Monalkyl- oder Dialkyl- oder Arylaminogruppe, eine Hydroxyamino- oder Hydrazinogruppe, eine Hydrazobenzolgruppe, eine 2-Hydroxyethylaminogruppe, eine Morpholinogruppe oder eine Piperidinogruppe, eine freie oder substituierte Mercaptogruppe, vorzugsweise eine Alkylthiogruppe, eine substituierte Hydroxygruppe, vorzugsweise eine Alkoxygruppe, einen Azido-, einen Chlor- oder Bromrest, bedeutet, wobei Y nicht eine Hydroxygruppe ist, wenn X ein Sauerstoffatom darstellt.

Unter einem Alkylrest einer Alkylimino-, einer Monoalkylamino-, einer Dialkylamino-, einer Alkylthio- und einer Alkoxygruppe wird ein Alkylrest mit bis zu 6 Kohlenstoffatomen, unter einem Arylrest einer Arylaminogruppe wird ein Arylrest bis zu 10 Kohlenstoffatomen verstanden.

Bevorzugte Vertreter der Säurederivate VII sind solche, in denen A eine – CN-Gruppe eine

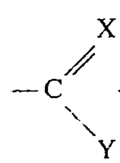 -Gruppe

darstellt, worin

X ein Sauerstoffatom, ein Schwefelatom oder eine Iminogruppe und

Y einen Amino-, Monoalkylamino-, Dialkylamino-, Phenylamino-, Alkoxy-, Alkylthio-, Chlor- oder Bromrest

bedeutet.

Besonders bevorzugte Vertreter der Säurederivate VII sind die entsprechenden Säureamide, Säurealkylester und Nitrile.

Zur Solvolyse der funktionellen Carbonsäurederivate VII verwendet man ein wasserabgebendes Medium, das ganz oder teilweise aus Wasser bzw. aus bei den Hydrolysebedingungen wasserabspaltenden Mitteln besteht. Die Reaktion kann als Homogenreaktion geführt werden, in welchem Fall man meist in Gegenwart eines polaren organischen Lösungsmittels oder eines Lösungsvermittlers arbeitet. Vorteilhafterweise werden als Lösungsmittel beispielsweise niedermolekulare Alkohole, Dioxan, Aceton, niedermolekulare Carbonsäuren, N-Methylpyrrolidon, Sulfolan oder Dimethylsulfoxid verwendet. Man kann aber die Hydrolyse auch als Heterogenreaktion führen. Der pH-Wert des wasserabgebenden Mediums richtet sich nach der chemischen Natur des eingesetzten Säurederivates, aber auch nach der Natur der gewünschten Verbindung der allgemeinen Formel I, er kann daher neutral, sauer oder basisch sein. Er wird mit Säuren, Basen oder Puffern auf den gewünschten Wert eingestellt.

Die Hydrolysetemperaturen liegen zwischen 0°C und dem Siedepunkt des wasserabgebenden Mediums, im allgemeinen 0° und 150°C, insbesondere zwischen 20° uns 120°C. Die Hydrolysetemperaturen hängen im einzelnen auch davon ab, ob unter Druck oder ohne Druck gearbeitet wird. Die Reaktionszeiten liegen je nach Ansatz, Reaktionstemperaturen und übrigen Reaktionsparametern zwischen 10 Minuten und 20 Stunden. Nach beendeter Hydrolyse werden die Säuren I nach üblichen Methoden, z. B. durch Umkristallisation oder durch Ansäuern ihrer Lösungen, gegebenenfalls unter Einengung ihrer Lösungen, isoliert. Zu ihrer Reinigung kann deren alkalische Lösung mit einem organischen Lösungsmittel, das mit der alkalischen Lösung nicht mischbar ist, beispielsweise Diethylether, Benzol, Chlorbenzol, Chloroform oder Methylenchlorid, extrahiert werden.

Die Carbonsäurederivate VII werden nach dem Fachmann geläufigen Methoden erhalten. Beispielsweise werden sie durch Reaktion von funktionellen Halogenalkansäurederivaten XVIII

$$R^{11}—(CH_2)_n—A \qquad (XVIII)$$

worin $R^{11}$, n und A die oben angegebene Bedeutung haben, mit Aminen IX und anschließende Acylierung mit Acylderivaten III erhalten. Alternativ können sie durch Umsetzung der Acylaminoalkansäurederivate XIX

$$R^1—CO—\underset{\substack{|\\ R^9}}{N}—(CH_2)_n—A \qquad (XIX)$$

in denen $R^1$, $R^9$, A und n die oben angegebene Bedeutung haben, mit den Hydrocarbylderivaten VI hergestellt werden.

Zur Herstellung der Verbindungen der Ausgestaltungen I*, I**, I*** bzw. I**** werden entsprechende Ausgangsmaterialien II*, II**, II***, II**** bzw. III*, III**, III***, III**** bzw. IV*, IV**, IV***, IV**** bzw. V*, V**, V***, V**** bzw. VI*, VI**, VI***, VI**** bzw. VII*, VII**, VII***, VII****, worin die Substituenten die entsprechende Bedeutung haben, umgesetzt.

Die Überführung der Säuren der allgemeinen Formel I oder der Ausgestaltungen I*, I**, I***, I**** in ihre Salze kann durch direkte alkalische Hydrolyse der Säurederivate der allgemeinen Formel VII erfolgen. Als alkalischer Reaktionspartner wird diejenige anorganische oder organische Base verwendet, deren Salz gewünscht wird. Man erhält die Salze aber auch, indem man die Säuren der allgemeinen Formel I mit dem stöchiometrischem Äquivalent an entsprechender Base, z. B. Natriumhydroxid oder Natriumalkoholat, umsetzt, oder leicht lösliche Salze durch doppelte Umsetzung in schwer lösliche Salze überführt, oder beliebige Salze in pharmakologisch verträgliche Salze überführt.

Die folgenden Beispiele erläutern die Erfindung näher, ohne sie einzuschränken. Die Abkürzung F. bzw. Sdp. bedeuten Schmelzpunkt bzw. Siedepunkt.

## Beispiele

### Beispiel 1

N-p-Chlorbenzoyl-4-[(1.1.3.3-tetramethylbutyl)-amino]-buttersäure

$R^1$ = p-Chlorphenyl, $R^2 = R^3 = CH_3$, $R^4 = -CH_2-C(CH_3)_3$, n = 3

a) N-p-Chlorbenzoyl-4-[(1.1.3.3-tetramethylbutyl)-amino]-buttersäure-ethylester

19,5 g 4-Brombuttersäure-ethylester, 25,9 g 1.1.3.3-Tetramethylbutylamin und 20 ml Cyclohexan werden 14 Tage bei Raumtemperatur gerührt. Das ausgeschiedene Salz [4-(1.1.3.3-Tetramethylbutyl-

amino)-buttersäure-ethylester-hydrobromid] wird abfiltriert und mit Cyclohexan gewaschen. Die Filtrate werden eingedampft, vom Rückstand werden bei einem Druck von ca. 0,5 Torr und einer Badtemperatur von maximal 50° alle flüchtigen Bestandteile abgezogen.

Der Rückstand wird in 30 ml Benzol gelöst, nach Zugabe von 8,8 g Ethyldiisopropylamin werden bei Raumtemperatur 12,0 g p-Chlorbenzoylchlorid unter Rühren zugetropft. Nach einer halben Stunde wird das ausgeschiedene Salz abfiltriert, das Filtrat eingedampft und der Eindampfungsrückstand aus Cyclohexan umkristallisiert. Es werden 22,0 g (57,6%d. Th.) der Titelverbindung vom F. 79—81° erhalten.

b) N-p-Chlorbenzoyl-4-[(1.1.3.3-tetramethylbutyl)-amino]-buttersäure

Eine Mischung von 17,0 g N-p-Chlorbenzoyl-4-[(1.1.3.3-tetramethylbutyl)-amino]-buttersäure-ethylester in 100 ml Benzol wird mit einer Lösung von 3,5 g Kaliumhydroxid in 20 ml Ethanol vermischt. Die Mischung wird 20 Stunden bei Raumtemperatur gerührt. Das Lösungsmittel wird bei einer Badtemperatur von maximal 50° im Vakuum abgedampft und der Rückstand in Wasser gelöst. Die wäßrige Lösung wird einmal mit Diethylether extrahiert, um Verunreinigungen und nicht umgesetztes Ausgangsmaterial zu entfernen, und dann mit verdünnter Salzsäure angesäuert. Der entstandene Niederschlag wird abfiltriert, getrocknet und aus Benzol-Leichtbenzin umkristallisiert. Es werden 13,3 g (83,6% d Th) der Titelverbindung vom F. 141—143° erhalten.

Beispiel 2

N-p-Fluorbenzoyl-4-[(1.1.3.3-tetramethylbutyl)-amino]-buttersäure

$R^1$ = p-Fluorphenyl, $R^2 = R^3 = CH_3$, $R^4 = -CH_2-C(CH_3)_3$, n = 3

Analog Beispiel 1 wird N-p-Fluorbenzoyl-4-[(1.1.3.3-tetramethylbutyl)-amino]-buttersäure-ethylester als zähes Öl durch Umsetzung des Reaktionsproduktes aus 4-Brombuttersäurethylester und 1.1.3.3-Tetramethylbutylamin mit p-Fluorbenzoylchlorid erhalten, dessen Verseifung die Titelverbindung (F. 114—117°) ergibt.

Beispiel 3

N-p-Chlorbenzoyl-4-(tert.-butylamino)-buttersäure

$R^1$ = p-Chlorphenyl, $R^2 = R^3 = R^4 = CH_3$, n = 3

Analog Beispiel 1 wird N-p-Chlorbenzoyl-4-(tert.-butylamino)-buttersäure-ethylester (F. 62—63°) durch um Setzung des Reaktionsproduktes aus 4-Brombuttersäure-ethylester und tert.-Butylamin mit p-Chlorbenzoylchlorid erhalten, dessen Verseifung die Titelverbindung (F. 126—127°) ergibt.

Beispiel 4

N-3,4,5-Trimethoxybenzoyl-6-(tert.-butylamino)-capronsäure

$R^1$ = 3,4,5-Trimethoxyphenyl, $R^2 = R^3 = R^4 = CH_3$, n = 5

Analog Beispiel 1 wird N-3,4,5-Trimethylbenzoyl-6-(tert.-butylamino)-capronsäure-ethylester (zähes, nicht unzersetzt destillierbares Öl) durch Umsetzung des Reaktionsproduktes aus 6-Bromcapronsäurethylester und tert.-Butylamin mit 3,4,5-Trimethoxybenzoylchlorid erhalten, dessen Verseifung die Titelverbindung (F. 83—85°) ergibt.

Beispiel 5

N-p-Chlorbenzoyl-4-[(1.1-dimethylpropyl)-amino]-buttersäure

$R^1$ = p-Chlorphenyl, $R^2 = R^3 = CH_3$, $R^4 = C_2H_5$, n = 3

Analog Beispiel 1 wird N-p-Chlorbenzoyl-4-[(1.1-dimethylpropyl)-amino]-buttersäure-ethylester (F. 65—67°) durch Umsetzung des Reaktionsproduktes aus 4-Brombuttersäure-ethylester und 1.1-Dimethylpropylamin mit p-Chlorbenzoylchlorid erhalten, dessen Verseifung die Titelverbindung (F. 79—81°) ergibt.

Beispiel 6

N-2,4-Dichlorbenzoyl-4-[(1.1-dimethylpropyl)-amino]-buttersäure

$R^1$ = 2,4-Dichlorphenyl, $R^2 = R^3 = CH_3$, $R^4 = C_2H_5$, n = 3

Analog Beispiel 1 wird N-2,4-Dichlorbenzoyl-4-[(1.1-dimethylpropyl)-amino]-buttersäure-ethylester (F. 75—77°) durch Umsetzung des Reaktionsproduktes aus 4-Brombuttersäure-ethylester und 1.1-Dimethylpropylamin mit 2,4-Dichlorbenzoylchlorid erhalten, dessen Verseifung die Titelverbindung (F. 124—126°) ergibt.

Beispiel 7

N-n-Butyryl-4-[(1.1-dimethylpropyl)-amino]-buttersäure

$R^1$ = N-$C_3H_7$, $R^2 = R^3 = CH_3$, $R^4 = C_2H_5$, n = 3

19,5 g 4-Brombuttersäure-ethylester, 26,1 g 1.1-Dimethylpropylamin und 20 ml Cyclohexan werden 10 Tage bei Raumtemperatur gerührt. Der entstandene Niederschlag wird abfiltriert und das Filtrat eingedampft. Nachdem bei einer Badtemperatur von 50° und einem Druck von 0,5 Torr alle flüchtigen Bestandteile entfernt wurden, wird der Rückstand mit 41 g n-Buttersäure-anhydrid, 33,4 ml Pyridin und 100 ml Benzol 5 Stunden bei Raumtemperatur gerührt. Das Gemisch wird bei einem Druck von 10 Torr und einer Badtemperatur von 90° eingedampft. Der in 100 ml Benzol gelöste Rückstand wird mit einer Lösung von 1,7 g Kaliumhydroxid in 20 ml Ethanol vermischt. Nach 20stündigem Stehen bei Raumtemperatur wird das Lösungsmittel bei vermindertem Druck verdampft und der Rückstand in Wasser gelöst; die wässerige Lösung wird einmal mit Diethylether gewaschen und dann mit verdünnter Salzsäure angesäuert. Der sich zunächst ölig abscheidende Niederschlag wird abfiltriert, getrocknet und aus Essigsäureethylester/Cyclohexan umkristallisiert. Es werden 8,9 g (36,6% d. Th) der Titelverbindung (F. 70—72°) erhalten.

Beispiel 8

N-p-Chlorbenzoyl-4-[(2-methyl-3-butin-2-yl)-amino]-buttersäure

$R^1$ = p-Chlorphenyl, $R^2 = -C{\equiv}CH$, $R^3 = R^4 = CH_3$, n = 3

Analog Beispiel 1 wird N-p-Chlorbenzoyl-4-[(2-methyl-3-butin-2-yl)-amino]-buttersäure-ethylester (F. 68—70°) durch Umsetzung des Reaktionsproduktes aus 4-Brombuttersäure-ethylester und 2-Methyl-3-butin 2-yl-amin mit p-Chlorbenzoylchlorid erhalten, dessen Verseifung die Titelverbindung (F. 102—104°) ergibt.

Beispiel 9

N-p-Chlorbenzoyl-4-[(3-ethyl-1-pentin-3-yl)-amino]-buttersäure

$R^1$ = p-Chlorphenyl, $R^2 = -C{\equiv}CH$, $R^3 = R^4 = C_2H_5$, n = 3

Analog Beispiel 1 wird N-p-Chlorbenzoyl-4-[(3-ethyl-1-pentin-3-yl)-amino]-butteräure-ethylester (F. 73—75°) durch Umsetzung des Reaktionsproduktes aus 4-Brombuttersäure-ethylester und 3-Ethyl-1-pentin-3-yl-amin mit p-Chlorbenzoylchlorid erhalten, dessen Verseifung die Titelverbindung (F. 92—94°) ergibt.

Beispiel 10

N-p-Chlorbenzoyl-4-[(1-ethinyl-cyclohexyl-1)-amino]-buttersäure

$R^1$ = p-Chlorphenyl, $R^2 = -C{\equiv}CH$, $R^3$ und $R^4 = -(CH_2)_5-$, n = 3

Analog Beispiel 1 wird N-p-Chlorbenzoyl-4-[(1-ethinyl-cyclohexyl-1)-amino]-buttersäure-ethylester (F. 84—86°) durch Umsetzung des Reaktionsproduktes aus 4-Brombuttersäure-ethylester und 1-Ethinyl-cyclohexylamin mit p-Chlorbenzoylchlorid erhalten, dessen Verseifung die Titelverbindung (F. 120—122°) ergibt.

Beispiel 11

N-Acetyl-4-[(1-ethinyl-cyclohexyl-1)-amino]-buttersäure

$R^1 = CH_3$, $R^2 = -C{\equiv}CH$, $R^3$ und $R^4 = -(CH_2)_5-$, n = 3

Analog Beispiel 1 wird N-Acetyl-4-[(1-ethinyl-cyclohexyl-1-)-amino]-buttersäure-ethylester (F. 73—75°) durch Umsetzung des Reaktionsproduktes aus 4-Brombuttersäure-ethylester und 1-Ethinyl-cyclohexylamin mit Acetylchlorid erhalten, dessen Verseifung die Titelverbindung (F. 103—105°) ergibt.

Beispiel 12

N-p-Chlorbenzoyl-4-[(1-n-propyl-cyclohexyl-1)-amino]-buttersäure

$R^1$ = p-Chlorphenyl, $R^2 = n\text{-}C_3H_7$, $R_3$ und $R_4 = -(CH_2)_5-$, n = 3

Analog Beispiel 1 wird N-p-Chlorbenzoyl-4-[(1-n-propyl-cyclohexyl-1)-amino]-buttersäure-ethylester (zähes nicht unzersetzt destillierbares Öl) durch Umsetzung des Reaktionsproduktes aus 4-Brombuttersäureethylester und 1-n-Propylcyclohexylamin mit p-Chlorbenzoylchlorid erhalten, dessen Verseifung die Titelverbindung (F. 110—112°) ergibt.

Beispiel 13

N-p-Chlorbenzoyl-4-[(1-n-butyl-cyclopentyl-1)-amino]-buttersäure

$R^1$ = p-Chlorphenyl, $R^2 = n\text{-}C_4H_9$, $R^3 + R^4 = -(CH_2)_4-$, n = 3

Analog Beispiel 1 wird N-p-Chlorbenzoyl-4-[(1-n-butyl-cyclopentyl-1)-amino]-buttersäure-ethylester (F. 85—87°) durch Umsetzung des Reaktionsproduktes aus 4-Brombuttersäure-ethylester und 1-n-Butyl-cyclopentylamin mit p-Chlorbenzoylchlorid erhalten, dessen Verseifung die Titelverbindung (F. 91—93°) ergibt.

Beispiel 14

N-p-Chlorbenzoyl-4-(1-adamantyl)-aminobuttersäure

$R^1$ = p-Chlorphenyl, $R^2$ und $R^3$ und $R^4$ = 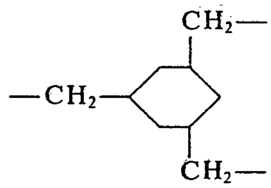 n = 3

Analog Beispiel 1 wird N-p-Chlorbenzoyl-4-[(1-adamantyl)-amino]-buttersäure-ethylester (F. 103—105°) durch Umsetzung des Reaktionsproduktes aus 4-Brombuttersäureethylester und 1-Aminoadamantan mit p-Chlorbenzoylchlorid erhalten, dessen Verseifung die Titelverbindung (F. 164—166°) ergibt.

Beispiel 15

N-p-Chlorbezoyl-4-cyclooctylamino-buttersäure

$R^1$ = p-Chlorphenyl, $R^2$ = H, $R^3$ und $R^4$ = $-(CH_2)_7-$, n = 3

Analog Beispiel 1 wird N-p-Chlorbenzoyl-4-cyclooctylamino-buttersäure-ethylester (nicht unzersetzt destillierbares Öl) durch Umsetzung des Reaktionsproduktes aus 4-Brombuttersäure-ethylester und Cyclooctylamin mit p-Chlorbenzoylchlorid erhalten, dessen Verseifung die Titelverbindung (F. 109—110°) ergibt.

Beispiel 16

N-Benzoyl-4-n-butylamino-buttersäure

15,5 g n-Butylpyrrolidon werden in einer Mischung von 15 g Natriumhydroxid in 100 ml Wasser 20 Stunden unter Rückfluß erhitzt. In die auf 0° gekühlte und stark gerührte Lösung werden 23,1 g Bezoylchlorid langsam eingetropft, die dann noch weitere 5 Stunden gerührt wird. Unter weiterer Kühlung wird 5 n Salzsäure bis zur sauren Reaktion zugegeben. Der ausgefallene Niederschlag, der noch Benzoesäure enthält, wird abgetrennt und mehrfach aus Leichtbenzin umkristallisiert. Es werden 13,6 g (47% d. Th.) der Titelverbindung (F. 62—64°) erhalten.

Beispiel 17

N-p-Chlorbenzoyl-5-(n-butylamino)-valeriansäure

$R^1$ = p-Chlorphenyl, $R^2$ = $R^3$ H, $R^4$ = n-$C_3H_7$, n = 4

a) 1-n-Butyl-$\delta$-valerolactam

Zu einer Lösung von 49,6 g $\delta$-Valerolactam in 300 ml wasserfreiem Dimethylsulfoxid gibt man 33,6 g feingepulvertes Kaliumhydroxid und läßt anschließend unter Rühren und gelegentlicher Kühlung 82,2 g 1-Brombutan innerhalb 1 Stunde zutropfen. Die Mischung wird noch 3 Stunden 70° gerührt, dann gekühlt, mit 1,5 l Wasser versetzt und anschließend mit Diethylether extrahiert. Die Etherphase wird mit Wasser gewaschen, getrocknet und eingedampft. Der Eindampfungsrückstand wird im Vakuum destilliert, wobei 42,5 g (54,7%d. Th.) der Titelverbindung (Sdp. 122°/13 Torr) erhalten werden.

b) N-p-Chlorbenzoyl-5-(n-butylamino)-valeriansäure

18,0 g 1-n-Butyl-$\delta$-valerolactam, 14,0 g Natriumhydroxid und 280 ml Wasser werden zusammen 8 Stunden unter Rückfluß zum Sieden erhitzt. Die Lösung wird dann gekühlt und mit verdünnter Salzsäure auf einen pH-Wert von 7,5 eingestellt. Anschließend werden unter Rühren und laufender pH-Kontrolle gleichzeitig 22,3 g p-Chlorbenzoylchlorid und 22,0 g 25%ige Natriumhydroxidlösung zugetropft, wobei der pH-Wert zwischen 7 und 8 gehalten wird. Nachdem nicht umgesetztes Ausgangsmaterial sowie Verunreinigungen durch Extraktion mit Diethylether entfernt worden sind, wird die wäßrige Lösung mit verdünnter Salzsäure angesäuert und der sich abscheidene ölige Niederschlag in Methylenchlorid aufgenommen. Der nach Trocknen und Abdestillieren des Methylenchlorids verbleibende Rückstand wird aus Essigsäureethylester-Petrolether umkristallisiert. Es werden 22,4 g (62% d. Th.) der Titelverbindung (F. 64,5—65,5°) erhalten.

Beispiel 18

N-p-Chlorbenzoyl-4-benzylamino-buttersäure

$R^1$ = p-Chlorphenyl, $R^2$ = $R^3$ = H, $R^4$ = Phenyl, n = 3

27,0 g N-Benzylpyrrolidon werden mit 350 ml 5%iger Natriumhydroxid-Lösung 30 Stunden unter Rückfluß zum Sieden erhitzt. Nach dem Abkühlen wird die Lösung mit verdünnter Salzsäure auf pH 8 eingestellt und nicht umgesetztes Ausgangsmaterial mit Diethylether extrahiert. In die klare wäßrige Lösung werden unter Rühren langsam 19,7 g p-Chlorbenzoylchlorid eingetropft unter gleichzeitiger

Zugabe von verdünnter Natronlauge, um den pH-Wert zwischen 7 und 8 zu halten. Nach Beendigung der Säurechloridzugabe wird die Lösung noch 30 Minuten bei pH 8 gerührt und dann mit verdünnter Salzsäure bis pH 3 angesäuert. Der ausgefallene Niederschlag wird in Essigsäureethylester aufgenommen; die Essigesterphase wird mit Magnesiumsulfat getrocknet und eingeengt. Nach Zugabe von Petrolether und unter Kühlung kristallisiert das Produkt aus. Man erhält 31 g (61%d. Th.) der Titelverbindung (F. 101–102°).

Beispiel 19

N-p-Chlorbenzoyl-4-benzhydrylamino-buttersäure

$R^1$ = p-Chlorphenyl, $R^2$ = H, $R^3$ = $R^4$ = Phenyl, n = 3

a) 4-Benzhydrylamino-buttersäure-ethylester

19,5 g 4-Brombuttersäureethylester, 55 g Benzhydrylamin und 30 ml Cyclohexan werden 12 Tage bei Raumtemperatur gerührt. Der entstandene Kristallbrei wird mit Diethylether verdünnt, abgesaugt und das Filtrat eingedampft. Der Eindampfungsrückstand wird im Vakuum destilliert. Als Vorlauf destilliert bei 100—110° (0,02 Torr) überschüssiges Benzhydrylamin über. Als Hauptlauf erhält man 21,1 g (71% d. Th.) der Titelverbindung [Sdp. 150—155° (0,02 Torr)].

b) N-p-Chlorbenzoyl-4-benzhydrylamino-buttersäure-ethylester

6,8 g p-Chlorbenzoylchlorid werden zu einer Lösung von 10,5 g 4-Benzhydrylamino-buttersäure-ethylester und 5 g Ethyldiisopropylamin in 100 ml Benzol unter Kühlung und Rühren zugetropft. Nach weiteren 2 Stunden wird der Niederschlag abfiltriert, das Filtrat eingedampft und der Eindampfungsrückstand aus Leichtbenzin umkristallisiert. Es werden 9,5 g (61,7%d. Th.) der Titelverbindung (F. 68—69°) erhalten.

c) N-p-Chlorbenzoyl-4-benzhydrylamino-buttersäure

9,1 g N-p-Chlorbenzoyl-4-benzhydrylamino-buttersäure-ethylester werden in 90 ml Benzol gelöst und nach Zugabe einer Lösung von 1,8 g Kaliumhydroxid in 20 ml Ethanol 20 Stunden bei Raumtemperatur gerührt. Das Lösungsmittel wird dann im Vakuum abdestilliert und der Rückstand in Wasser gelöst. Die wässerige alkalische Lösung wird einmal mit Diethylether gewaschen und dann mit verdünnter Salzsäure angesäuert. Das ausgefallene Produkt wird abfiltriert, getrocknet und aus Leichtbenzin umkristallisiert. Es werden 8,0 g (94% d. Th.) der Titelverbindung (F. 110—111°) erhalten.

Beispiel 20

N-Acetyl-4-benzhydrylamino-buttersäure

$R^1$ = $CH_3$, $R^2$ = H, $R^3$ = Phenyl, n = 3

Analog Beispiel 19 werden 10,8 g 4-Benzhydrylamino-buttersäure-ethylester und 5,2 g Ethyldiisopropylamin in 100 ml Benzol gelöst und mit 3,1 g Acetylchlorid umgesetzt. Als Reaktionsprodukt werden 11,4 g (92,5%d. Th.) N-Acetyl-4-benzhydrylamino-buttersäure-ethylester als zähes, nicht destillierbares Öl erhalten. Die Verseifung dieses Esters ergibt 9,2 g (88% d. Th.) der Titelverbindung (F. 173—174°).

Beispiel 21

N-p-Chlorbenzoyl-4-(1-phenylethyl-amino)-buttersäure

$R^1$ = p-Chlorphenyl, $R^2$ = H, $R^3$ = $CH_3$, $R^4$ = Phenyl, n = 3

Analog Beispiel 1 wird N-p-Chlorbenzoyl-4-(1-phenylethylamino)-buttersäure-ethylester (zähes, nicht destillierbares Öl) durch Umsetzung des Reaktionsproduktes aus 4-Brombuttersäure-ethylester und dl-1-Phenylethylamin mit p-Chlorbenzoylchlorid erhalten, dessen Verseifung die Titelverbindung (F. 110—112°) ergibt.

Beispiel 22

N-p-Chlorbenzoyl-6-(1-phenylethylamino)-capronsäure

$R^1$ = p-Chlorphenyl, $R^2$ = H, $R^3$ = $CH_3$, $R^4$ = Phenyl, n = 5

Analog Beispiel 1 wird N-p-Chlorbenzoyl-6-(1-phenylethylamino)-capronsäure-ethylester (zähes, nicht destillierbares Öl) durch Umsetzung des Reaktionsproduktes aus 6-Bromcapronsäureethylester und dl-1-Phenylethylamin mit p-Chlorbenzoylchlorid erhalten, dessen Verseifung die Titelverbindung (F. 132—133°) ergibt.

Beispiel 23

N-p-Chlorbenzoyl-4-homoveratrylamino-buttersäure

$R^1$ = p-Chlorphenyl, $R^2$ = $R^3$ = H, $R^4$ = 3,4-Dimethoxybenzyl, n = 3

Analog Beispiel 1 wird N-p-Chlorbenzoyl-4-homoveratrylamino-buttersäure-ethylester (zähes nicht destillierbares Öl) durch Umsetzung des Reaktionsproduktes aus 4-Brombuttersäure-ethylester und Homoveratrylamin mit p-Chlorbenzoylchlorid erhalten, dessen Verseifung die Titelverbindung (F. 101—103°) ergibt.

Beispiel 24

N-p-Chlorbenzoyl-4-[(1,2-diphenylethyl)-amino]-buttersäure

$R^1$ = p-Chlorphenyl, $R^2$ = H, $R^3$ = Phenyl, $R^4$ = Benzyl, n = 3

Analog Beispiel 1 wird N-p-Chlorbenzoyl-4-[(1,2diphenylethyl)-amino]-buttersäure-ethylester (zähes, nicht destillierbares Öl) durch Umsetzung des Reaktionsproduktes aus 4-Brombuttersäure-ethylester und 1,2-Diphenyl-ethylamin mit p-Chlorbenzoylchlorid erhalten, dessen Verseifung die Titelverbindung (F. 121—122°) ergibt.

Beispiel 25

Natriumsalz der N-p-Chlorbenzoyl-4-[(1.1.3.3-tetramethylbutyl)-amino]-buttersäure

3,5 g N-p-Chlorbenzoyl-4-[(1.1.3.3-tetramethylbutyl)-amino]-buttersäure werden in 35 ml siedendem Isopropylalkohol gelöst, dazu wird eine heiße Auflösung von 0,23 g Natriummetall in 17,5 ml Isopropylalkohol zugegeben. Nach dem Abkkühlen wird die Lösung mit dem gleichen Volumen Diethylether vermischt. Nach mehreren Stunden wird die kristalline Titelverbindung abfiltriert, mit Isopropylalkohol/Ether gewaschen und getrocknet. Die Ausbeute ist fast quantitativ. Das Salz schmilzt unscharf bei 187—190°.

Beispiel 26

N-p-Chlorbenzoyl-4-benzylamino-buttersäure

a) 4-Benzylamino-butyronitril

32,2 g Benzylamin, 14,8 g 4-Brombutyronitril und 30 ml Cyclohexan werden 12 Stunden bei Raumtemperatur gerührt. Nach Zugabe von Diethylether wird das abgeschiedene Benzylamin-hydrobromid abfiltriert; die Etherphase wird mit Wasser gewaschen und eingedampft. Der Rückstand wird im Vakuum destilliert. Es werden 11,5 g (66% d. Th.) der Titelverbindung (Sdp. 114—120°/0,05 Torr) erhalten.

b) N-p-Chlorbenzoyl-4-benzylamino-butyronitril

Zu einer gut gerührten Mischung von 9,0 g 4-Benzylamino-butyronitril, 6,7 g Ethyldiisopropylamin und 60 ml Benzol werden bei Raumtemperatur 9,0 g p-Chlorbenzoylchlorid zugetropft. Nach Beendigung der Zugabe wird 2 Stunden weitergerührt. Das Reaktionsgemisch wird mit Diethylether versetzt und in Eiswasser gekühlt; das ausgeschiedene Salz wird abfiltriert. Das Filtrat wird eingedampft und der Rückstand aus Essigsäureethylester/Petrolether umkristallisiert. Es werden 10,0 g (62% d. Th.) der Titelverbindung (F. 48—50°) erhalten.

c) N-p-Chlorbenzoyl-4-benzoylamino-buttersäure

Eine Lösung von 0,5 g N-p-Chlorbenzoyl-4-benzylamino-butyronitril in 5 ml absolutem Ethanol wird bei 0° mit Chlorwasserstoffgas gesättigt. Die Lösung wird 30 Minuten auf 70° erwärmt, dann eingeengt; der Rückstand wird mit Wasser versetzt und mit Diethylether extrahiert. Der Rückstand des Etherextraktes wird in 3 ml Benzol gelöst und mit einer Lösung von 0,2 g Kaliumhydroxid in 4 ml Ethanol vermischt. Nach zweitägigem Stehen wird die klare Lösung eingeengt und der Rückstand in Wasser gelöst. Die wässerige Lösung wird zunächst mit Diethylester extrahiert, um Verunreinigungen zu entfernen, und dann mit verdünnter Salzsäure auf pH 3 angesäuert. Der ausgefallene Niederschlag wird mit Methylenchlorid extrahiert und nach dem Abdestillieren des Methylenchlorids aus Essigsäureethylester/Petrolether umkristallisiert. Es werden 0,4 g der Titelverbindung (F. 101—102°) erhalten, deren IR- und NMR-Spektren mit denen der nach Beispiel 18 hergestellten Substanz identisch sind.

Beispiel 27

N-p-Chlorbenzoyl-4-benzylamino-buttersäure

a) N-p-Chlorbenzoyl-4-aminobuttersäure

Zu einer Lösung von 10,3 g 4-Aminobuttersäure und 4,0 g Natriumhydroxid in 150 ml Wasser werden bei Raumtemperatur 17,5 g p-Chlorbenzoylchlorid langsam unter Rühren zugetropft. Durch gleichzeitige Zugabe von verdünnter Natronlauge wird ein pH von 7 bis 8 eingehalten. Die wäßrige Lösung wird einmal mit Diethylether gewaschen und dann mit verdünnter Salzsäure angesäuert. Der entstandene Niederschlag wird in Methylenchlorid aufgenommen; der nach dem Abdampfen des Lösungsmittels verbleibende Rückstand wird mit Diethylether gewaschen. Man erhält 21,5 g (89% d. Th.) der Titelverbindung (F. 107—108°).

b) N-p-Chlorbenzoyl-4-benzylamino-buttersäure-benzylester

In einer Lösung von 7,25 g N-p-Chlorbenzoyl-4-aminobuttersäure und 30 ml Dimethylsulfoxid werden 4,0 g Kaliumhydroxid-Pulver suspendiert. In diese Suspension werden unter Rühren 9,1 g Benzylchlorid langsam eingetropft. Das Gemisch wird weitere 7 Stunden bei Raumtemperatur gerührt und dann mit 100 ml Wasser versetzt. Das Reaktionsprodukt wird mit Diethylether extrahiert; die Etherphase wird mit Wasser gewaschen, getrocknet und eingedampft. Das Rohprodukt wird durch Chromatographie an einer Kieselgel-Säule, Laufmittel Methylenchlorid, gereinigt. Es werden 5,8 g (46%d. Th.) der Titelverbindung als farbloses zähes Öl erhalten. Das NMR-Spektrum bestätigt die Struktur.

c) N-p-Chlorbenzoyl-4-benzylamino-buttersäure (cis-Form)

Eine Lösung von 2,0 g N-p-Chlorbenzoyl-4-benzylamino-buttersäure-benzylester in 20 ml Benzol wird mit einer Lösung von 0,4 g Kaliumhydroxid in 5 ml Ethanol vermischt. Nach 20stündigem Stehen bei Raumtemperatur wird das Lösungsmittel abgedampft und der Rückstand in Wasser gelöst. Die wäßrige Lösung wird mit Diethylether gewaschen und dann mit verdünnter Salzsäure auf pH 3 angesäuert. Der Niederschlag wird abfiltriert und aus Essigsäureethylester/Leichtbenzin umkristallisiert. Es werden 1,35 g (86% d. Th.) der Titelverbindung (F. 111—112°) erhalten.

Es handelt sich hierbei um die cis-Form der Titelverbindung. Erhitzt man diese einige Minuten auf 230° und kristallisiert dann nochmals aus Essigester/Leichtbenzin um, so erhält man Kristalle vom F. 101—102°), die mit den nach Beispiel 18 und Beispiel 26 hergestellten Verbindungen identisch sind.

Beispiel 28

N-p-Chlorbenzoyl-4-(tert.-butylamino)-buttersäure

a) N-p-Chlorbenzoyl-4-(tert.-butylamino)-crotonsäure-ethylester

Zu einer Suspension von 8,4 g 4-(tert.-Butylamino)-crotonsäure-ethylester-hydrochlorid in 70 ml Benzol werden unter Kühlung 11,7 g Ethyldiisopropylamin und anschließend 7,9 g p-Chlorbenzoylchlorid zugetropft. Die Mischung wird weitere 5 Stunden bei Raumtemperatur gerührt, filtriert und das Filtrat eingedampft. Der Rückstand wird aus Leichtbenzin umkristallisiert. Man erhält 10,0 g der Titelverbindung (F. 77—78 °).

b) N-p-Chlorbenzoyl-4-(tert.-butylamino)-crotonsäure

Eine Lösung von 8,0 g N-p-Chlorbenzoyl-4-(tert.-butylamino)-crotonsäure-ethylester in 20 ml Benzol wird mit einer Lösung von 2,0 g Kaliumhydroxid in 15 ml Ethanol vermischt. Die Mischung wird 20 Stunden bei Raumtemperatur aufbewahrt und dann am Rotationsverdampfer eingeengt. Der Eindampfungsrückstand wird in Wasser gelöst; die wäßrige Lösung wird mit Diethylether gewaschen und anschließend mit verdünnter Salzsäure angesäuert. Der ausgefallene Niederschlag wird aus Cyclohexan umkristallisiert. Man erhält 6,1 g der Titelverbindung (F. 113—114°).

c) N-p-Chlorbenzoyl-4-(tert.-butylamino)-buttersäure

5,0 g der N-p-Chlorbenzoyl-4-(tert.-butylamino)-crotonsäure werden in 100 ml Tetrahydrofuran gelöst und nach Zugabe von 3,0 g Palladiumkohle (5% Pd) in einer Hydrierapparatur mit Wasserstoff behandelt. Nach Beendigung der Wasserstoffaufnahme wird vom Katalysator abfiltriert und das Lösungsmittel verdampft. Der Rückstand wird aus Ethanol/Wasser (1 : 1) umkristallisiert. Man erhält 4,9 g der Titelverbindung (F. 126—127°). Der Mischschmelzpunkt mit der nach Beispiel 3 hergestellten Substanz zeigt keine Depression.

Beispiel 29

N-p-Chlorbenzoyl-4-[(p-methoxybenzyl)-amino]-buttersäure

$R^1$ = p-Chlorphenyl, $R^2 = R^3$ = H, $R^4$ = p-Methoxyphenyl, n = 3

Analog Beispiel 18 wird 1-p-Methoxybenzylpyrrolidon mit Natriumhydroxid-Lösung erhitzt und anschließend mit p-Chlorbenzoylchlorid umgesetzt. Man erhält die Titelverbindung vom F. 128,5—129,5°.

Beispiel 30

N-p-Chlorbenzoyl-5-benzylamino-valerinsäure

$R^1$ = p-Chlorphenyl, $R^2 = R^3$ = H, $R^4$ = Phenyl, n = 4

Analog Beispiel 18 wird 1-Benzyl-$\delta$-valerolactam mit Natriumhydroxid-Lösung erhitzt und anschließend mit p-Chlorbenzoylchlorid umgesetzt. Man erhält die Titelverbindung vom F. 93—94°.

Beispiel 31

N-m-Trifluormethyl-benzoyl-4-[(1.1.3.3-tetramethylbutyl)-amino]-buttersäure

$R^1$ = $\alpha,\alpha,\alpha$-Trifluor-m-tolyl, $R^2 = R^3 = CH_3$, $R^4 = -CH_2-C(CH_3)_3$, n = 3

Analog Beispiel 1 wird N-m-Trifluormethyl-benzoyl-4-[(1.1.3.3-methylbutyl)-amino]-buttersäure-ethylester als zähes Öl durch Umsetzung des Reaktionsproduktes aus 4-Brombuttersäure-ethylester und 1.1.3.3-Tetramethylbutylamin mit m-Trifluormethyl-benzoylchlorid erhalten, dessen Verseifung die Titelverbindung (F. 86—87°) ergibt.

Beispiel 32

N-Crotonoyl-4-[(1.1.3.3-tetramethylbutyl)amino]-buttersäure

$R^1 = -CH=CH-CH_3$, $R^2 = R^3 = CH_3$, $R^4 = -CH_2-C(CH_3)_3$, n = 3

Analog Beispiel 1 wird N-Crotonoyl-4-[(1.1.3.3-tetramethylbutyl)-amino]-buttersäure-ethylester als zähes Öl durch Umsetzung des Reaktionsproduktes aus 4-Brombuttersäure-ethylester und 1.1.3.3-Tetramethylbutylamin mit Crotonsäurechlorid erhalten, dessen Verseifung die Titelverbindung (F. 92—93°) ergibt.

Beispiel 33

N-Propionyl-4-benzhydrylamino-buttersäure

$R^1 = C_2H_5$, $R^2 = H$, $R^3 = R^4 =$ Phenyl, n = 3

a) N-Propionyl-4-benzhydrylamino-buttersäure-ethylester

Analog Beispiel 19 werden 9,0 g 4-Benzhydrylamino-buttersäure-ethylester und 4,3 g Diisopropylethylamin in 100 ml Benzol mit 3,1 g Propionylchlorid umgesetzt. Das Reaktionsprodukt wird aus Essig-ester-Leichtbenzin (1 : 1) umkristallisiert. Es werden 9,9 g (92,5% d. Th.) der Titelverbindung (F. 83—85°) erhalten.

b) N-Propionyl-4-benzhydrylamino-buttersäure

Die Verseifung von 9,3 g N-Propionyl-4-benzhydrylamino-buttersäure-ethylester in 100 ml Benzol mit einer Lösung von 2,2 g Kaliumhydroxid in 20 ml Ethanol analog Beispiel 19 ergibt nach Umkristallisieren des Reaktionsproduktes aus Essigester-Leichtbenzin (1 : 1) 7,8 g (91% d. Th.) die Titelverbindung vom F. 151,5—152,5°.

Beispiel 34

N-(5-Chlor-2-methoxy-benzoyl)-4-benzhydrylamino-buttersäure

$R^1 =$ 5-Chlor-2-methoxy-phenyl, $R^2 = H$, $R^3 = R^4 =$ Phenyl, n = 3

Analog Beispiel 19 werden 8,9 g 4-Benzhydrylamino-buttersäure-ethylester und 4,3 g Ethyl-diisopropylamin in 80 ml Benzol gelöst und mit einer Lösung von 6,8 g 5-Chlor-2-methoxy-benzoesäure-chlorid in 20 ml Benzol umgesetzt. Als Reaktionsprodukt werden 13,8 g (99% d. Th.) N-(5-Chlor-2-methoxy-benzoyl)-4-benzhydrylamino-buttersäure-ethylester als zähes, nicht destillierbares Öl erhalten. Die Verseifung dieses Esters ergibt 11,1 g (85,6% d. Th.) der Titelverbindung (F. 176—178°C).

Beispiel 35

N-Acetyl-6-benzhydrylamino-capronsäure

$R^1 = CH_3$, $R^2 = H$, $R^3 = R^4 =$ Phenyl, n = 5

a) 6-Benzhydrylamino-capronsäure-ethylester

22,3 g 6-Bromcapronsäure-ethylester, 55 g Benzhydrylamin und 30 ml Cyclohexan werden 20 Tage bei Raumtemperatur gerührt. Die Aufarbeitung analog Beispiel 19 ergibt 22,5 g (69% d. Th.) der Titelverbindung [Sdp. 162—167° (0,02 Torr)].

b) N-Acetyl-6-benzhydrylamino-capronsäure

Analog Beispiel 19 werden 8 g 6-Benzhydrylamino-capronsäure-ethylester und 3,5 Ethyldiisopropylamin in 100 ml Benzol gelöst und mit 2,1 g Acetylchlorid umgesetzt. Als Reaktionsprodukt werden 9 g N-Acetyl-6-benz-hydrylamino-capronsäure-ethylester als zähes, nicht destillierbares Öl erhalten. Die Verseifung dieses Esters ergibt 7,3 g (87,5%) der Titelverbindung (F. 119—120°C).

Beispiel 36

N-Isobutyryl-6-benzhydrylamino-capronsäure

$R^1 = CH(CH_3)_2$, $R^2 = H$, $R^3 = R^4 =$ Phenyl, $n = 5$

Analog Beispiel 19 werden 7 g 6-Benzhyarylamino-capronsäure-ethylester und 3,1 g Ethyldiisopropylamin in 100 ml Benzol gelöst und mit 2,5 g Isobutyrylchlorid umgesetzt. Als Reaktionsprodukt werden 7,8 g N-Isobutyryl-6-benzhydrylamino-capronsäure-ethylester als zähes, nicht destillierbares Öl erhalten. Die Verseifung dieses Esters ergibt 6,1 g (77% d. Th.) der Titelverbindung (F. 106–107°).

Beispiel 37

N-Acetyl-5-benzhydrylamino-valeriansäure

$R^1 = CH_3$, $R^2 = H$, $R^3 = R^4 =$ Phenyl, $n = 4$

a) 5-Benzhydrylamino-valeriansäure-ethylester

25,1 g 5-Bromvaleriansäure-ethylester, 66 g Benzhydrylamin und 30 ml Cyclohexan werden 15 Tage bei Raumtemperatur gerührt. Die Aufarbeitung analog Beispiel 19 ergibt 23,4 g (62,6%d. Th.) der Titelverbindung [Sdp. 158–163° (0,01 Torr)].

b) N-Acetyl-5-benzhydrylamino-valeriansäure

Analog Beispiel 19 werden 9 g 5-Benzhydrylamino-valeriansäure-ethylester und 4,1 g Ethyldiisopropylamin in 100 ml Benzol gelöst und mit 2,5 g Acetylchlorid umgesetzt. Als Reaktionsprodukt werden 10 g N-Acetyl-5-benzhydrylamino-valeriansäure-ethylester als zähes, nicht destillierbares Öl erhalten. Die Verseifung dieses Esters ergibt 7,4 g (78,7% d. Th.) der Titelverbindung (F. 135–136°).

Beispiel 38

N-Crotonoyl-5-benzhydrylamino-valeriansäure

$R^1 = CH{=}CH{-}CH_3$, $R^2 = H$, $R^3 = R^4 =$ Phenyl, $n = 4$

Analog Beispiel 19 werden 7 g 5-Benzhydrylamino-valeriansäure-ethylester und 3,2 g Ethyldiisopropylamin in 100 ml Benzol gelöst und mit 2,6 g Crotonoylchlorid umgesetzt. Als Reaktionsprodukt werden 8,5 g N-Crotonoyl-5-benzhydrylamino-valeriansäure-ethylester als zähes, nicht destillierbares Öl erhalten. Die Verseifung dieses Esters ergibt 6 g (76% d. Th.) der Titelverbindung (F. 88–89°).

Beispiel 39

N-p-Chlorbenzoyl-4-[L(–)-(α-methylbenzyl)-amino]-buttersäure

$R^1 =$ p-Chlorphenyl, $R^2 = H$, $R^3$ $CH_3$, $R^4 =$ Phenyl, $n = 3$

Analog Beispiel 1 wird N-p-Chlorbenzoyl-4-L(–)-(α-methylbenzyl-amino)-buttersäure-ethylester (zähes, nicht destillierbares Öl) durch Umsetzung des Reaktionsproduktes aus 4-Brombuttersäure-ethylester und L(–)-α-Methylbenylamin mit p-Chlorbenzoylchlorid erhalten, dessen Verseifung die Titelverbindung (F. 93–94°, $[\alpha]_D$ – 142,5°) ergibt.

Beispiel 40

N-Salicyloyl-4-benzhydrylamino-buttersäure

$R^1 =$ o-Hydroxyphenyl, $R^2 = H$, $R^3 = R^4 =$ Phenyl, $n = 3$

13,3 g 4-Benzhydrylamino-buttersäure-ethylester (Beispiel 19a) und 6,4 g Ethyldiisopropylamin werden in 80 ml Benzol gelöst, dazu wird eine Lösung von 9,8 g o-Acetylsalicylsäurechlorid in 20 ml

Benzol unter Kühlung und Rühren zugetropft. Nach weiteren 4 Stunden wird das Reaktionsgemisch mit Diethylether versetzt, das ausgeschiedene Salz abfiltriert und das Filtrat eingedampft. Der Eindampfrückstand wird durch Chromatographie an einer Kieselgel-Säule, Laufmittel Methylenchlorid, gereinigt. Es werden 18,6 g (90,5%d. Th.) N-o-Acetoxy-benzoyl-4-benzhydrylamino-buttersäure-ethylester als farbloses, zähes, nicht destillierbares Öl erhalten. Dieses wird in 200 ml Benzol gelöst und eine Lösung von 5,7 g Kaliumhydroxid in 30 ml Ethanol zugefügt. Nach 20stündigem Stehen bei Raumtemperatur wird das Reaktionsgemisch mit Diethylether verdünnt und dann mit Wasser ausgeschüttelt. Die wäßrige Phase wird mit verdünnter Salzsäure angesäuert, der Niederschlag abfiltriert, getrocknet und aus Essigester/Leichtbenzin umkristallisiert. Es werden 13,0 g (82,5%d. Th.) der Titelverbindung (F. 168—169°) erhalten.

## Beispiel 41

Ampullen mit 600 mg N-(p-Chlorbenzoyl)-4-(1-phenylethylamino)-buttersäure, Chargengröße 250 kg

25 kg 1,2-Propylenglykol und 150 kg Aqua bidestillata werden vorgelegt, 15 kg N-(p-Chlorbenzoyl)-4-(1-phenylethyl-amino)-buttersäure werden zugegeben und anschließend werden langsam, unter Rühren, ca. 17 kg Natronlauge (10 Gew.-% NaOH) zugegeben. Wenn sich alles gelöst hat, wird mit Natronlauge der pH auf 7,5—8,0 eingestellt. 0,0625 kg Natriumpyrosulfit werden zugegeben, und die Mischung wird gerührt, bis sich alles gelöst hat. Mit Aqua bidestillata wird auf 250 kg aufgefüllt. Die Lösung wird in 10-ml-Ampullen abgefüllt und bei 120° 30 Minuten im Autoklaven sterilisiert.

## Beispiel 42

Ampullen mit 600 g N-(p-Chlorbenzoyl)-4-(benzhydrylamino)-buttersäure, Chargengröße 250 kg

50 kg 1,2-Propylenglykol und 150 kg Aqua bidestillata werden vorgelegt. Dann werden unter Rühren 15 kg N-(p-Chlorbenzoyl)-4-(benzhydrylamino)-buttersäure zugegeben. Anschließend werden zunächst 15 kg Natronlauge hinzugefügt, die Mischung wird dann auf einen pH von 8,0 eingestellt. Mit Aqua bidestillata wird auf 250 kg ergänzt. Die Lösung wird in 10-ml-Ampullen abgefüllt und 30 Minuten bei 120° im Autoklaven sterilisiert.

## Beispiel 43

Tabletten mit 50 mg N-(p-Chlorbenzoyl)-4-(benzylamino)-buttersäure

25 kg N-(p-Chlorbenzoyl)-4-(benzylamino)-buttersäure, 35 kg Milchzucker und 26 kg Maisstärke werden mit 2,5 kg Polyvinylpyrrolidon (Molekulargewicht ca. 25 000) in ungefähr 6 l Wasser granuliert. Das Granulat wird durch ein Sieb von 1,25 mm Maschenweite gesiebt, und nach dem Trocknen werden 8 kg Carboxymethylcellulose, 2,5 kg Talkum und 1 kg Magnesiumstearat zugegeben. Man verpreßt das trockene Granulat zu Tabletten von 8 mm Durchmesser, 250 mg Gewicht und einer Härte von 5—6 kg.

In ähnlicher Weise werden Tabletten mit N-Acetyl-4-benzhydrylamino-buttersäure bzw. N-(p-Chlorbenzoyl)-4-(1-phenylethyl-amino)-buttersäure hergestellt.

## Beispiel 44

Tabletten mit 100 mg N-(p-Chlorbenzoyl)-4-[(1,2-diphenylethyl)-amino]-buttersäure

40 kg N-(p-Chlorbenzoyl)-4-[(1,2-diphenylethyl)-amino]-buttersäure, 24 kg Milchzucker und 16 kg Maisstärke werden mit 4 kg Polyvinylpyrrolidon (Molekulargewicht ca. 25 000) in ungefähr 5,5 l Wasser granuliert und durch ein Sieb von 1,25 mm Maschenweite gepreßt. Nach dem Trocknen werden 10 kg Carboxymethylcellulose, 4 kg Talkum und 2 kg Magnesiumstearat zugegeben. Auf einer Exzentermaschine wird das Granulat zu Tabletten von 9 mm Durchmesser, 250 mg Gewicht und einer Härte von 4—5 kg verpreßt.

In ähnlicher Weise werden N-(p-Chlorbenzoyl)-6-(1-phenylethylamino)-capronsäure bzw. N-(p-Chlorbenzoyl)-4-(1-adamantyl)-aminobuttersäure zu Tabletten mit einem Wirkstoffgehalt von 100 mg verpreßt.

Beispiel 45

Tabletten mit 300 mg N-(p-Chlorbenzoyl)-4-(cyclooctylamino)-buttersäure

60 kg N-(p-Chlorbenzoyl)-4-(cyclooctylamino)-buttersäure, 12 kg Milchzucker und 8 kg Maisstärke werden mit 4 kg Polyvinylpyrrolidon (Molekulargewicht ca. 25 000) in ungefähr 6 l Wasser granuliert und durch ein Sieb von 1,25 mm Maschenweite gepreßt. Nach dem Trocknen werden 10 kg Carboxymethylcellulose, 4 kg Talkum und 2 kg Magnesiumstearat zugegeben. Auf einem Rundläufer wird das Granulat zu Tabletten von 11 mm Durchmesser, 500 mg Gewicht und einer Härte von 6–7 kg verpreßt.

Beispiel 46

10 000 Kapseln mit einem Wirkstoffgehalt von 50 mg werden aus folgenden Bestandteilen hergestellt: 500 g N-(p-Chlorbenzoyl)-4-(benzhydrylamino)-buttersäure, 495 g mikrokristalline Cellulose und 5 g amorphe Kieselsäure werden gut gemischt und in Hartgelatinekapseln Größe 4 abgefüllt.

Pharmakologie

Die Acylhydrocarbylaminoalkansäuren sowie ihre Salze üben einen starken Einfluß auf die Pankreassekretion narkotisierter Ratten aus, daneben beeinflussen sie die Gallesekretion narkotisierter Ratten und entfalten eine antihepatotoxische Wirksamkeit an der wachen Ratte, wobei sie sich bekannten Handelspräparaten, z. B. Piprozolin überlegen erweisen.
In den anschließenden Tabellen werden die untersuchten Verbindungen durch eine laufende Nummer gekennzeichnet, die wie folgt zugeordnet ist:

| Lfd. Nr. | Name der Verbindung |
|---|---|
| 1 | Piprozolin |
| 2 | N-p-Chlorbenzoyl-4-(tert.-butylamino)-buttersäure |
| 3 | N-p-Chlorbenzoyl-4-[(2-methyl-3-butin-2-yl)-amino]-buttersäure |
| 4 | N-p-Chlorbenzoyl-4-[(3-ethyl-1-pentin-3-yl)-amino]-buttersäure |
| 5 | N-p-Chlorbenzoyl-4-[(1-ethinyl-cyclohexyl-1)-amino]-buttersäure |
| 6 | N-p-Chlorbenzoyl-4-(1-adamantyl)-aminobuttersäure |
| 7 | N-p-Chlorbenzoyl-4-[(1.1.3.3-tetramethyl-butyl)-amino]-buttersäure |
| 8 | N-Acetyl-4-[(1-ethinyl-cyclohexyl-1)-amino]-buttersäure |
| 9 | N-p-Chlorbenzoyl-4-homoveratrylamino-buttersäure |
| 10 | N-p-Chlorbenzoyl-4-(1-phenylethyl-amino)-buttersäure |
| 11 | N-2,4-Dichlorbenzoyl-4-[(1.1-dimethyl-propyl)-amino]-buttersäure |
| 12 | N-p-Chlorbenzoyl-4-benzhydrylamino-buttersäure |
| 13 | N-p-Chlorbenzoyl-6-(1-phenethylamino)-capronsäure |
| 14 | N-Acetyl-4-benzhydrylamino-buttersäure |
| 15 | N-p-Chlorbenzoyl-4-benzylamino-buttersäure |
| 16 | N-p-Fluorbenzoyl-4-[(1.1.3.3-tetramethyl-butyl)-amino]-buttersäure |
| 17 | N-p-Chlorbenzoyl-4-[(1.2-diphenylethyl)-amino]-buttersäure |
| 18 | N-p-Chlorbenzoyl-4-cyclooctylamino-buttersäure |

In Tabelle I werden Untersuchungen der Pankreassekretion von narkotisierten Ratten nach intraduodenaler Applikation ($ED_{50}$) und die letale Wirkung an der Maus ($LD_{50}$) nach intraperitonealer Applikation von Vertretern der erfindungsgemäßen Verbindungen sowie der therapeutische Quotient ($TQ = LD_{50}/ED_{50}$) wiedergegeben.

Tabelle I

Pankreassekretion, Toxizität und therapeutischer Quotient

| Lfd. Nr. | Toxizität $LD_{50}$ [mg/kg] (Maus, i.p.) | Pankreassekretion $ED_{50}$*) [mg/kg] (Ratte, i.d.) | TQ [$LD_{50}/ED_{50}$] |
|---|---|---|---|
| 1 | 1070**) | 35 | 31 |
| 5 | 190 | 2 | 95 |
| 6 | 200 | 1 | 200 |
| 7 | 220 | 2 | 110 |
| 8 | $\gg$1000 | 10 | $\gg$100 |
| 9 | 780 | 5 | 156 |
| 10 | 1200 | ~20 | ~60 |
| 12 | 250 | ~0,1 | ~2500 |
| 13 | 220 | 2 | 110 |
| 14 | 1100 | ~5 | ~220 |
| 15 | 350 | 1,5 | 233 |
| 16 | 400 | ~10 | ~40 |
| 17 | 160 | ~1 | ~160 |
| 18 | 150 | 2,5 | 60 |

*) $ED_{50}$ = Dosis, die eine Steigerung der Pankreassekretion (Flüssigkeitsvolumen; 30-Min.-Fraktion) um maximal 50% bewirkt.

**) $LD_{50}$ [p.o.], zit. aus Herrmann et al. Arzneim.-Forsch. 27 (1977) 467.

In Tabelle II werden Untersuchungen der Gallesekretion (Cholerese) von narkotisierten Ratten nach intraduodenaler Applikation ($ED_{50}$) und die letale Wirkung an der Maus ($LD_{50}$) nach intraperitonealer Applikation von Vertretern der erfindungsgemäßen Verbindungen sowie der therapeutische Quotient ($TQ = LD_{50}/ ED_{50}$) wiedergegeben.

Tabelle II

Gallesekretion, Toxizität und therapeutischer Quotient

| Lfd. Nr. | Toxizität $LD_{50}$ [mg/kg] (Maus, i.p.) | Gallesekretion $ED_{50}$***) [mg/kg] (Ratte, i.d.) | TQ [$LD_{50}/ED_{50}$] |
|---|---|---|---|
| 1 | 1070**) | 40 | 27 |
| 2 | 850 | 15 | 57 |
| 3 | >1000 | 25 | >40 |
| 4 | 200 | ~5 | ~40 |
| 5 | 190 | 6 | 32 |
| 9 | 780 | 18 | 43 |
| 10 | 1200 | ~15 | ~80 |
| 11 | 190 | 6 | 32 |
| 14 | 1100 | 5 | 220 |
| 16 | 400 | 7 | 57 |
| 17 | 160 | 5 | 32 |

*) $ED_{50}$ = Dosis, die eine Steigerung der Gallesekretion (Flüssigkeitsvolumen; 30-Min.-Fraktion) um maximal 50% bewirkt.

**) $LD_{50}$ [p.o.], zit. aus Herrmann et al. Arzneim.-Forsch. 27 (1977) 467.

In Tabelle III werden Untersuchungen der antihepatotoxischen Wirkung ($ED_{50}$) erfindungsgemäßer Verbindungen nach oraler Applikation an wachen Ratten und die letale Wirkung nach intraperitonealer Applikation an der Maus ($LD_{50}$) sowie der therapeutische Quotient (TQ = $LD_{50}/ED_{50}$) wiedergegeben.

Tabelle III

Antihepatotoxischer Effekt, Toxizität und therapeutischer Quotient

| Lfd. Nr. | Toxizität $LD_{50}$ [mg/kg] (Maus, i.p.) | Antihepatotoxischer Effekt $ED_{50}$****) [mg/kg] (Ratte, p.o.) | TQ $[LD_{50}/ED_{50}]$ |
|---|---|---|---|
| 1 | 1070**) | >300 | <3,6 |
| 3 | >1000 | 100 | >10 |
| 9 | 780 | 100 | 7,8 |
| 10 | 1200 | ~10 | ~120 |
| 12 | 250 | 10 | 25 |
| 14 | 1100 | 10 | 110 |
| 17 | 160 | 10 | 16 |
| 18 | 150 | 8 | 18,8 |

****) $ED_{50}$ = Dosis, die für $CCl_4$-lebergeschädigte Ratten die Hexobarbital-Narkose um 50% verkürzt.

**) $LD_{50}$ [p.o.], zit. aus Herrmann et al. Arzneim.-Forsch. 27 (1977) 467.

Die Ermittlung der pharmakologischen Eigenschaften erfolgte nach folgenden Methoden:

### Einfluß auf die Pankreas- und Gallesekretion der narkotisierten Ratte

### Versuchsdurchführung

Männliche Sprague-Dawley-Ratten (250—300 g Körpergewicht) werden mit 1,2 g/kg Urethan i.m. narkotisiert. Dann wird die Bauchhöhle medial geöffnet, der Ductus choledochus kurz oberhalb seiner Mündung in das Duodenum sowie nahe der Leberpforte ligiert, beide Gangabschnitte werden leberwärts katheterisiert.

Da bei der Ratte alle Pankreaskanäle in den mittleren Abschnitt des Ductus choledochus münden, lassen sich auf diese Weise aus dem distalen (ligierten) Gangabschnitt das Pankreassekret und aus dem proximalen Teil des Ductus choledochus die Galle getrennt ableiten.

Die sezernierten Mengen an Pankreas- und Gallensaft werden in 30-Min.-Abständen während 2 Stunden vor bis 3 Stunden nach der intraduodenalen (V. jugularis externa) Verabreichung der zu prüfenden Verbindungen (verabreichtes Flüssigkeitsvolumen 5 ml/kg) gemessen.

Die Körpertemperatur der Tiere wird mittels Heizkissen und Bestrahlung auf 36—38°C gehalten; die Temperaturkontrolle erfolgt rectal.

### Auswertung

Die Flüssigkeitsvolumina der 30-Min.-Fraktionen nach der Substanzgabe werden jeweils auf die vor der Substanzapplikation ausgeschiedene Galle- bzw. Pankreassaftmenge (= 100%, Mittelwert aus den beiden letzten Messungen) bezogen. Die maximale prozentuale Steigerung der Pankreas- bzw. Gallesekretion wird in Abhängigkeit von der Dosis dargestellt und daraus die $ED_{50}$ durch Interpolation ermittelt.

Prüfung auf antihepatotoxische Wirkung

Einfluß auf die Hexobarbitalschlafdauer der Ratte nach $CCl_4$-Leberschädigung

Versuchsdurchführung

In Anlehnung an Vogel et al. [Arzneim. Forsch. 25 (1975) 82] wird bei nüchternen weiblichen Sprague-Dawley-Ratten (190 ± 10 g Körpergewicht, 10 Tiere/Dosis pro Versuchsansatz) durch orale Verabreichung von Tetrachlorkohlenstoff (0,15 mg/kg $CCl_4$ in 2,5 ml/kg Olivenöl) ein Leberzellenschaden erzeugt, dessen Ausmaß an der Verlängerung der Hexobarbitalnatrium-Schlafdauer (50 mg/ml/kg i.v.; Schwanzvene, Injektionsdauer 45–60 sec) 47 Stunden nach der $CCl_4$-Gabe bestimmt wird. Die zu prüfenden Verbindungen werden eine Stunde vor der $CCl_4$-Gabe oral in einem Flüssigkeitsvolumen von 10 ml/kg verabreicht.

Auswertung

Der antihepatotoxische Effekt der zu prüfenden Verbindungen (Natrium-Salze in wäßriger Lösung) wird an der prozentualen Verkürzung der durch die $CCl_4$-Leberzellschädigung bedingten Schlafdauerverlängerung bei den behandelten Gruppen gegenüber der Schlafdauerverlängerung der $CCl_4$-Kontrollgruppe (= 100%) gemessen. Die $ED_{50}$ wurde durch Interpolation aus der Dosis-Wirkungskurve ermittelt.

Bestimmung der Toxizität

Die Toxizitätsuntersuchungen werden an weiblichen NMRI-Mäusen (Körpergewicht 22–26 g) durchgeführt. Die Tiere (5 Tiere pro Dosis) erhalten Futter und Wasser ad libitum. Verschiedene Dosierungen der Substanzen werden intraperitoneal verabreicht. Die Beobachtungsdauer beträgt 14 Tage. Die $DL_{50}$, d. h. die Dosis, bei der 50% der Tiere sterben, wird aus der Dosiswirkungskurve ermittelt.

**Patentansprüche**

1. Acylhydrocarbylaminoalkansäuren der allgemeinen Formel I

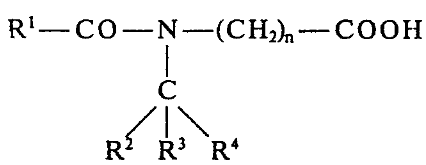

(I)

worin

$R^1$ $C_1$–$C_7$ Alkyl, $C_2$–$C_7$ Alkenyl, $C_2$–$C_7$ Alkinyl, $C_3$–$C_{10}$ Cycloalkyl oder Phenyl der allgemeinen Formel

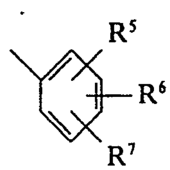

$R^2$ Wasserstoff, $C_1$–$C_7$ Alkyl, $C_2$–$C_7$ Alkenyl oder $C_2$–$C_7$ Alkinyl,
$R^3$ Wasserstoff, $C_1$–$C_7$ Alkyl, $C_3$–$C_{10}$ Cycloalkyl oder Phenyl der allgemeinen Formel

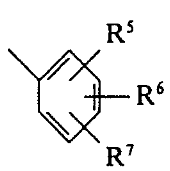

und
$R^4$ $C_1$–$C_7$ Alkyl, $C_3$–$C_{10}$ Cycloalkyl, Phenyl der allgemeinen Formel

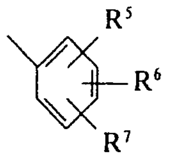

oder Phenylalkyl mit $C_1-C_4$ Alkyl und Phenyl der allgemeinen Formel

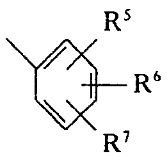

bedeuten, wobei die Summe der Kohlenstoffatome der Alkylsubstituenten $R^2$, $R^3$ und $R^4$ wenigstens 3 beträgt und wobei die Substituenten $R^2$, $R^3$ und $R^4$ gemeinsam mit dem Kohlenstoffatom, an das sie gebunden sind, kein geradkettiges oder verzweigtes Alkyl oder Alkenyl darstellen, wenn $R^1$ Alkyl oder Alkenyl ist und n 3 bedeutet, oder

$R^3$ und $R^4$ gemeinsam $C_2-C_8$ Alkylen oder
$R^2$, $R^3$ und $R^4$ unter Einschluß des benachbarten Kohlenstoffatoms Adamantyl,
n 3, 4 oder 5 bedeuten und
$R^5$, $R^6$ und $R^7$ gleich oder verschieden sind und Wasserstoff, Halogen, $C_1-C_4$ Alkyl, $C_1-C_4$ Alkoxy, $C_1-C_4$ Alkylmercapto, $C_2-C_5$ Alkanoyloxy, Amino, Nitro, Trifluormethyl, Trifluormethoxy, Hydroxy oder Trifluormethylmercapto bedeuten, wobei $R^5$, $R^6$ und $R^7$ nicht gleichzeitig Wasserstoff bedeuten wenn $R^1$ und $R^4$ einen Phenylrest und $R^2$ und $R^3$ ein Wasserstoffatom darstellen,

sowie ihre Salze mit anorganischen und organischen Basen.

2. Acylhydrocarbylaminoalkansäuren nach Anspruch 1, gekennzeichnet durch die allgemeine Formel I*

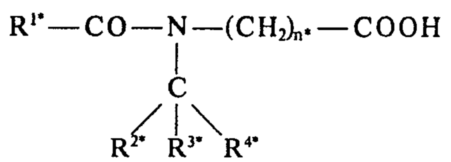

(I*)

worin

$R^{1*}$ $C_1-C_5$ Alkyl, $C_2-C_5$ Alkenyl, $C_2-C_5$ Alkinyl, $C_5-C_7$ Cycloalkyl oder Phenyl der allgemeinen Formel

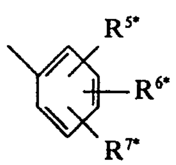

$R^{2*}$ Wasserstoff, $C_1-C_5$ Alkyl, $C_2-C_5$ Alkenyl oder $C_2-C_5$ Alkinyl,
$R^{3*}$ Wasserstoff oder $C_1-C_5$ Alkyl und
$R^{4*}$ $C_1-C_5$ Alkyl bedeutet, wobei die Summe der Kohlenstoffatome der Alkylsubstituenten $R^{2*}$, $R^{3*}$ und $R^{4*}$ wenigstens 3 beträgt und wobei die Substituenten $R^{2*}$, $R^{3*}$ und $R^{4*}$ gemeinsam mit dem Kohlenstoffatom, an das sie gebunden sind, kein geradkettiges oder verzweigtes Alkyl oder Alkenyl darstellen, wenn $R^{1*}$ Alkyl oder Alkenyl ist und n* 3 bedeutet,
n* 3, 4 oder 5 bedeutet,
$R^{5*}$, $R^{6*}$ und $R^{7*}$ gleich oder verschieden sind und Wasserstoff, Halogen, $C_1-C_4$ Alkyl, $C_1-C_4$ Alkoxy, $C_2-C_5$ Alkanoyloxy, Amino, Nitro, Hydroxy oder Trifluormethyl bedeuten,

und ihre Salze mit anorgansichen und organischen Basen.

3. Verbindungen nach Anspruch 2, in denen $R^{1*}$ $C_1-C_5$ Alkyl, $C_2-C_5$ Alkenyl oder Alkinyl oder durch $R^{5*}$, $R^{6*}$ und $R^{7*}$ substituiertes Phenyl, $R^{2*}$ Wasserstoff, $C_1-C_4$ Alkyl oder Ethinyl, $R^{3*}$ Wasserstoff oder $C_1-C_3$ Alkyl, $R^{4*}$ $C_1-C_5$ Alkyl (wobei die Summe der Kohlenstoffatome der Alkylsubstituenten $R^{2*}$, $R^{3*}$ und $R^{4*}$ wenigstens 3 beträgt und wobei die Substituenten $R^{2*}$, $R^{3*}$ und $R^{4*}$ gemeinsam mit dem Koh-

lenstoffatom, an das sie gebunden sind, kein geradkettiges oder verzweigtes Alkyl oder Alkenyl darstellen, wenn $R^{1*}$ Alkyl oder Alkenyl ist und n* 3 bedeutet), $R^{5*}$ Wasserstoff und n* 3, 4 oder 5 bedeuten, $R^{6*}$ und $R^{7*}$ gleich oder verschieden sind und Wasserstoff, Halogen, Methyl, Methoxy, Amino oder Trifluormethyl darstellen, und ihre Salze mit anorganischen oder organischen Basen.

4. Acylhydrocarbylaminoalkansäuren nach Anspruch 1, gekennzeichnet durch die allgemeine Formel I**

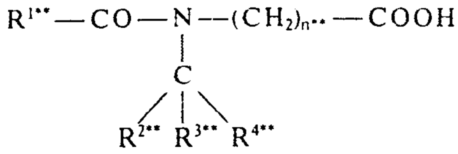

(I**)

worin

$R^{1**}$ die Bedeutung von $R^{1*}$ hat,
$R^{2**}$ die Bedeutung von $R^{2*}$ hat,
$R^{3**}$ Wasserstoff, $C_1-C_5$ Alkyl oder $C_5-C_7$ Cycloalkyl,
$R^{4**}$ $C_5-C_7$ Cycloalkyl oder
$R^{3**}$ und $R^{4**}$ gemeinsam Alkylen $-(CH_2)_{q^{**}}-$
oder
$R^{2**}$, $R^{3**}$ und $R^{4**}$ unter Einschluß des benachbarten Kohlenstoffatoms Adamantyl,
n** 3, 4 oder 5 und
q** 4, 5, 6 oder 7 bedeuten,
$R^{5**}$, $R^{6**}$ und $R^{7**}$ gleich oder verschieden sind und Wasserstoff, Halogen, $C_1-C_4$ Alkyl, $C_1-C_4$ Alkoxy, $C_2-C_5$ Alkanoyloxy, Amino, Nitro, Hydroxy oder Trifluormethyl bedeuten,

und ihre Salze mit anorganischen und organischen Basen.

5. Verbindungen nach Anspruch 4, in denen $R^{1**}$ $C_1-C_5$ Alkyl, $C_2-C_5$ Alkenyl oder Alkinyl oder durch $R^{5**}$, $R^{6**}$ und $R^{7**}$ substituiertes Phenyl, $R^{2**}$ Wasserstoff, $C_1-C_4$ Alkyl oder Ethinyl, $R^{3**}$ Wasserstoff, Methyl oder Cyclohexyl, $R^{4*}$ Cyclohexyl oder $R^{3**}$ und $R^{4**}$ gemeinsam Alkylen $-(CH_2)_{q^{**}}$ oder $R^{2**}$, $R^{3**}$ und $R^{4**}$ unter Einschluß des benachbarten Kohlenstoffatoms Adamantyl-(1), n** 3, 4 oder 5, q** 4, 5, 6 oder 7, $R^{5**}$ Wasserstoff bedeuten, $R^{6**}$ und $R^{7**}$ gleich oder verschieden sind und Wasserstoff, Halogen, Methyl, Methoxy, Amino oder Trifluormethyl darstellen, und ihre Salze mit anorganischen oder organischen Basen.

6. Acylhydrocarbylaminoalkansäuren nach Anspruch 1, gekennzeichnet durch die allgemeine Formel I***

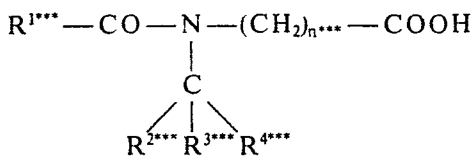

(I***)

worin

$R^{1***}$ die Bedeutung von $R^{1*}$ hat,
$R^{2***}$ Wasserstoff oder $C_1-C_5$ Alkyl,
$R^{3***}$ Wasserstoff, $C_1-C_5$ Alkyl oder Phenyl der allgemeinen Formel

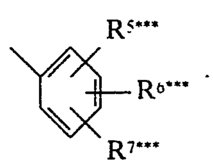

$R^{4***}$ Phenyl der allgemeinen Formel

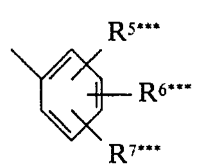

oder Phenylalkyl der allgemeinen Formel

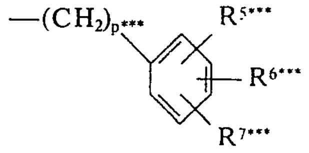

$n^{***}$ 3, 4 oder 5 und
$p^{***}$ 1, 2, 3 oder 4 bedeuten,
$R^{5***}$, $R^{6***}$ und $R^{7***}$ gleich oder verschieden sind und Wasserstoff, Halogen, $C_1$—$C_4$ Alkyl, $C_1$—$C_4$ Alkoxy, $C_2$—$C_5$ Alkanoyloxy, Amino, Nitro, Hydroxy oder Trifluormethyl bedeuten, wobei $R^{5***}$, $R^{6***}$ und $R^{7***}$ nicht gleichzeitig Wasserstoff bedeuten, wenn $R^{1***}$ und $R^{4***}$ einen Phenylrest und $R^{2***}$ und $R^{3***}$ ein Wasserstoffatom darstellen,

und ihre Salze mit anorganischen und organischen Basen.

7. Verbindungen nach Anspruch 6, in denen $R^{1***}$ $C_1$—$C_5$ Alkyl, $C_2$—$C_5$ Alkenyl oder Alkinyl oder durch $R^{5***}$, $R^{6***}$ und $R^{7***}$ substituiertes Phenyl, $R^{2***}$ Wasserstoff oder $C_1$—$C_4$ Alkyl, $R^{3***}$ Wasserstoff, $C_1$—$C_3$ Alkyl oder durch $R^{7***}$ substituiertes Phenyl, $R^{4***}$ durch $R^{7***}$ substituiertes Phenyl oder durch $R^{7***}$ substituiertes Benzyl, $R^{5***}$ Wasserstoff und $n^{***}$ 3, 4 oder 5 bedeuten, $R^{6***}$ und $R^{7***}$ gleich oder verschieden sind und Wasserstoff, Halogen, Methyl, Methoxy, Amino oder Trifluormethyl darstellen, wobei $R^{5***}$, $R^{6***}$ und $R^{7***}$ nicht gleichzeitig Wasserstoff bedeuten wenn $R^{1***}$ und $R^{4***}$ einen Phenylrest und $R^{2***}$ und $R^{3***}$ ein Wasserstoffatom darstellen, und ihre Salze mit anorganischen und organischen Basen.

8. Acylhydrocarbylaminoalkansäuren nach Anspruch 1, gekennzeichnet durch die allgemeine Formel $I^{****}$

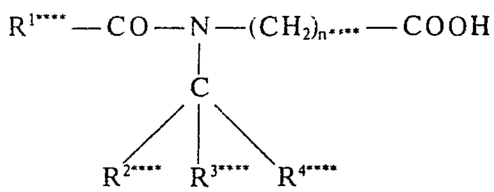

(I****)

worin

$R^{1****}$ die Bedeutung von $R^{1*}$ hat,
$R^{2****}$ $C_2$–$C_5$ Alkenyl oder $C_2$–$C_5$ Alkinyl,
$R^{3****}$ Wasserstoff oder $C_1$–$C_5$ Alkyl,
$R^{4****}$ Phenyl der allgemeinen Formel

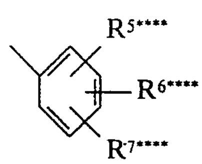

**oder Phenylalkyl der allgemeinen Formel**

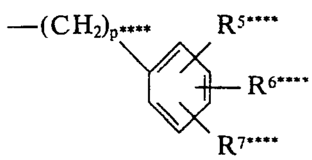

$n^{****}$ 3, 4 oder 5 und
$p^{****}$ 1, 2, 3 oder 4 bedeuten,
$R^{5****}$, $R^{6****}$ und $R^{7****}$ gleich oder verschieden sind und Wasserstoff, Halogen, $C_1$—$C_4$ Alkyl, $C_1$—$C_4$ Alkoxy, $C_2$—$C_5$ Alkanoyloxy, Amino, Nitro, Hydroxy oder Trifluormethyl bedeuten,

und ihre Salze mit anorgischen oder organischen Basen.

9. Verbindungen nach Anspruch 8, in denen $R^{1****}$ $C_1$–$C_5$ Alkyl, $C_2$–$C_5$ Alkenyl oder Alkinyl, oder durch $R^{5****}$, $R^{6****}$ und $R^{7****}$ substituiertes Phenyl, $R^{2****}$ Ethinyl, $R^{3****}$ Wasserstoff oder $C_1$–$C_3$ Alkyl, $R^{4****}$ durch $R^{7****}$ substituiertes Phenyl oder durch $R^{7****}$ substituiertes Benzyl, $R^{5****}$ Wasserstoff und $n^{****}$ 3, 4 oder 5 bedeuten, $R^{6****}$ und $R^{7****}$ gleich oder verschieden sind und Wasserstoff, Halogen, Methyl, Methoxy, Amino oder Trifluormethyl darstellen, und ihre Salze mit anorganischen und organischen Basen.

10. Arzneimittel, enthaltend eine oder mehrere Verbindungen gemäß einem oder mehreren der Ansprüche 1 bis 9.

11. Verfahren zur Herstellung der Acylhydrocarbylaminoalkansäuren der allgemeinen Formel I

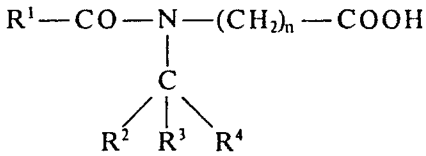
(I)

sowie ihrer Salze mit anorganischen oder organischen Basen, dadurch gekennzeichnet, daß man

a) eine Hydrocarbylaminoalkansäure der allgemeinen Formel II

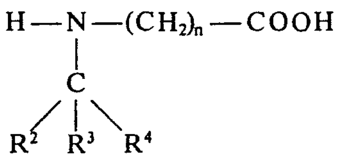
(II)

gegebenenfalls unter Schutz der Carboxylgruppe, mit einem Acylderivat der allgemeinen Formel III

$$R^1—CO—R^8 \qquad (III)$$

worin $R^8$ eine Fluchtgruppe oder eine $R^1$–CO–O-Gruppe darstellt, acyliert und gegebenenfalls anschließend in die Salze überführt oder

b) eine Hydrocarbylaminoalkensäure der allgemeinen Formel IV

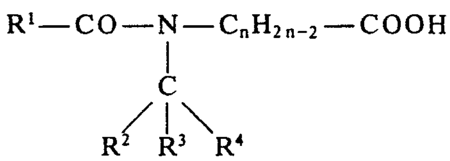
(IV)

gegebenenfalls unter Schutz der Carboxylgruppe, hydriert und gegebenenfalls anschließend in die Salze überführt oder

c) eine Acylaminoalkansäure der allgemeinen Formel V

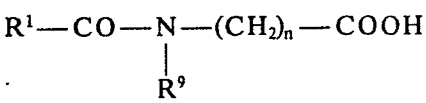
(V)

worin $R^9$ ein Wasserstoffatom oder ein Metallatom eines Alkali- oder Erdalkalimetalls bedeutet, gegebenenfalls unter Schutz der Carboxylgruppe, mit einem Hydrocarbylderivat der allgemeinen Formel VI

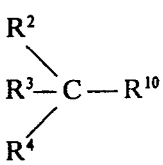
(VI)

worin $R^{10}$ eine Fluchtgruppe darstellt, umsetzt und gegebenenfalls anschließend in die Salze überführt oder

d) ein funktionelles Acylhydrocarbylaminoalkansäurederivat der allgemeinen Formel VII

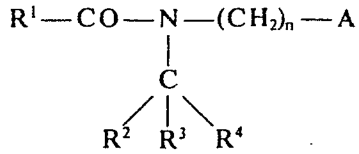

(VII)

worin A ein funktionelles Derivat einer Carboxylgruppe bedeutet, solvolysiert und gegebenenfalls anschließend in die Salze überführt, wobei die Substituenten $R^1$, $R^2$, $R^3$, $R^4$ und n in den Formeln I–VII die in Anspruch 1 angegebene Bedeutung haben.

12. Verfahren nach Anspruch 11 zur Herstellung der Acylhydrocarbylaminoalkansäuren der allgemeinen Formel I*

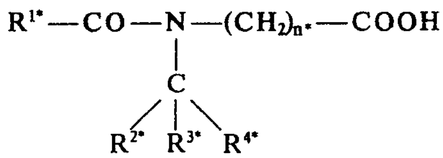

(I*)

und ihrer Salze mit anorganischen oder organischen Basen, dadurch gekennzeichnet, daß man

a) eine Hydrocarbylaminoalkansäure der allgemeinen Formel II*

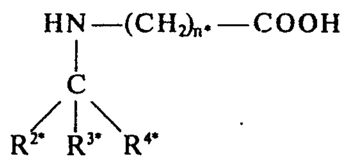

(II*)

gegebenenfalls unter Schutz der Carboxylgruppe, mit einem Acylderivat der allgemeinen Formel III*

$$R^{1*}—CO—R^{8*} \qquad (III^*)$$

worin $R^{8*}$ eine Fluchtgruppe oder eine $R^{1*}$–CO–O-Gruppe darstellt, acyliert und gegebenenfalls anschließend in die Salze überführt oder

b) eine Hydrocarbylaminoalkensäure der allgemeinen Formel IV*

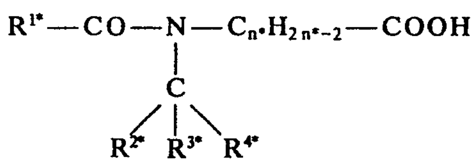

(IV*)

gegebenenfalls unter Schutz der Carboxylgruppe, hydriert und gegebenenfalls anschließend in die Salze überführt oder

c) eine Acylaminoalkansäure der allgemeinen Formel V*

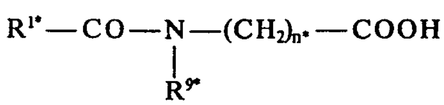

(V*)

worin $R^{9*}$ ein Wasserstoffatom oder ein Metallatom eines Alkalimetalls bedeutet, gegebenenfalls unter Schutz der Carboxylgruppe, mit einem Hydrocarbylderivat der allgemeinen Formel VI*

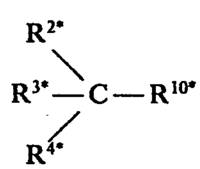

(VI*)

worin $R^{10*}$ eine Fluchtgruppe darstellt, umsetzt und gegebenenfalls anschließend in die Salze überführt oder

d) ein funktionelles Acylhydrocarbylaminoalkansäurederivat der allgemeinen Formel VII*

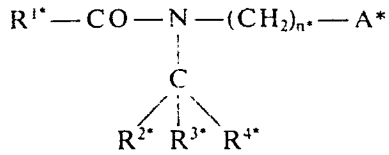

(VII*)

worin A* ein funktionelles Derivat einer Carboxylgruppe bedeutet, solvolysiert und gegebenenfalls anschließend in die Salze überführt, wobei die Substituenten $R^{1*}$, $R^{2*}$, $R^{3*}$, $R^{4*}$ und n* in den Formeln I*–VII* die in Anspruch 2 angegebene Bedeutung haben.

13. Verfahren nach Anspruch 11 zur Herstellung der Acylhydrocarbylaminoalkansäuren der allgemeinen Formel I**

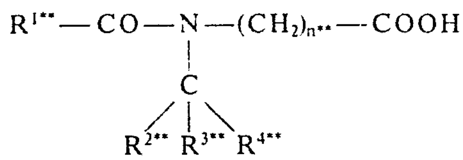

(I**)

und ihrer Salze mit anorganischen oder organischen Basen, dadurch gekennzeichnet, daß man

a) eine Hydrocarbylaminoalkansäure der allgemeinen Formel II**

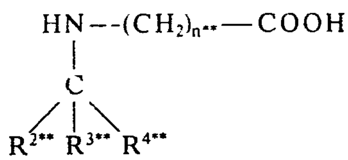

(II**)

gegebenenfalls unter Schutz der Carboxylgruppe, mit einem Acylderivat der allgemeinen Formel III**

$$R^{1**}—CO—R^{8**} \qquad (III^*)$$

worin $R^{8**}$ eine Fluchtgruppe oder eine $R^{1**}-CO-O$-Gruppe darstellt, acyliert und gegebenenfalls anschließend in die Salze überführt oder

b) eine Hydrocarbylaminoalkensäure der allgemeinen Formel IV**

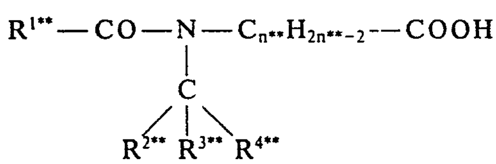

(IV**)

gegebenenfalls unter Schutz der Carboxylgruppe, hydriert und gegebenenfalls anschließend in die Salze überführt oder

c) eine Acylaminoalkansäure der allgemeinen Formel V**

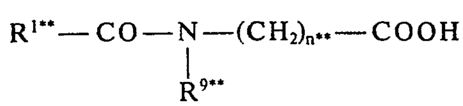

(V**)

worin $R^{9**}$ ein Wasserstoffatom oder ein Metallatom eines Alkalimetalls bedeutet, gegebenenfalls unter Schutz der Carboxylgruppe, mit einem Hydrocarbylderivat der allgemeinen Formel VI**

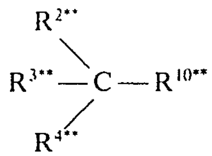

(VI**)

worin $R^{10**}$ eine Fluchtgruppe darstellt, umsetzt und gegebenenfalls anschließend in die Salze überführt oder

d) ein funktionelles Acylhydrocarbylaminoalkansäurederivat der allgemeinen Formel VII**

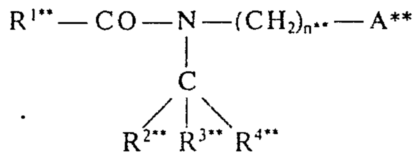

(VII**)

worin A** ein funktionelles Derivat einer Carboxylgruppe bedeutet, solvolysiert und gegebenenfalls anschließend in die Salze überführt, wobei die Substituenten $R^{1**}$, $R^{2**}$, $R^{3**}$, $R^{4**}$ und n** in den Formeln I**–VII** die in Anspruch 4 angegebene Bedeutung haben.

14. Verfahren nach Anspruch 11 zur Herstellung der Acylhydrocarbylaminoalkansäuren der allgemeinen Formel I***

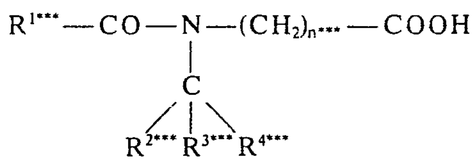

und ihrer Salze mit anorganischen oder organischen Basen, dadurch gekennzeichnet, daß man

a) eine Hydrocarbylaminoalkansäure der allgemeinen Formel II***

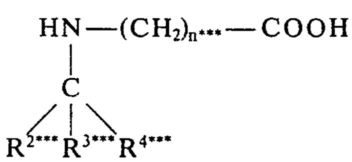

(II***)

gegebenenfalls unter Schutz der Carboxylgruppe, mit einem Acylderivat der allgemeinen Formel III***

$R^{1***}$—CO—$R^{8***}$ (III***)

worin $R^{8***}$ eine Fluchtgruppe oder eine $R^{1***}$—CO—O-Gruppe darstellt, acyliert und gegebenenfalls anschließend in die Salze überführt oder

b) eine Hydrocarbylaminoalkensäure der allgemeinen Formel IV***

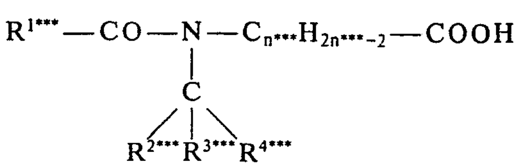

gegebenenfalls unter Schutz der Carboxylgruppe, hydriert und gegebenenfalls anschließend in die Salze überführt oder

c) eine Acylaminoalkansäure der allgemeinen Formel V***

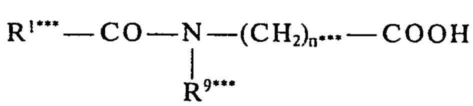

(V***)

worin $R^{9***}$ ein Wasserstoffatom oder ein Metallatom eines Alkalimetalls bedeutet, gegebenenfalls unter Schutz der Carboxylgruppe mit einem Hydrocarbylderivat der allgemeinen Formel VI***

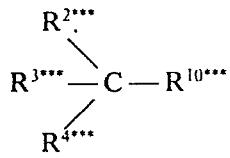

(VI***)

worin $R^{10***}$ eine Fluchtgruppe darstellt, umsetzt und gegebenenfalls anschließend in die Salze überführt oder

d) ein funktionelles Acylhydrocarbylaminoalkansäurederivat der allgemeinen Formel VII***

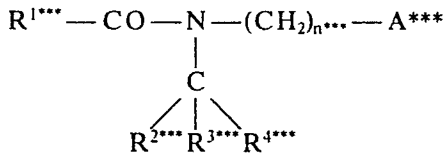

(VII***)

worin A*** ein funktionelles Derivat einer Carboxylgruppe bedeutet, solvolysiert und gegebenenfalls anschließend in die Salze überführt, wobei die Substituenten $R^{1***}$, $R^{2***}$, $R^{3***}$, $R^{4***}$ und n*** in den Formeln I***–VII*** die in Anspruch 6 angegebene Bedeutung haben.

15. Verfahren nach Anspruch 11 zur Herstellung der Acylhydrocarbylaminoalkansäuren der allgemeinen Formel I****

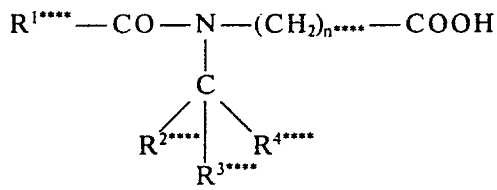

(I****)

und ihrer Salze mit anorganischen oder organischen Basen, dadurch gekennzeichnet, daß man

a) eine Hydrocarbylaminoalkansäure der allgemeinen Formel II****

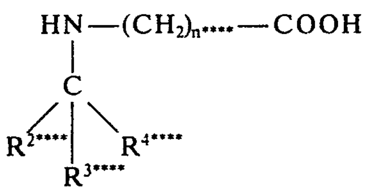

(II****)

gegebenenfalls unter Schutz der Carboxylgruppe, mit einem Acylderivat der allgemeinen Formel III****

$$R^{1****}—CO—R^{8****} \qquad (III^{****})$$

worin $R^{8****}$ eine Fluchtgruppe oder eine $R^{1****}$ – CO – O-Gruppe darstellt, acyliert und gegebenenfalls anschließend in die Salze überführt oder

b) eine Hydrocarbylaminoalkensäure der allgemeinen Formel IV****

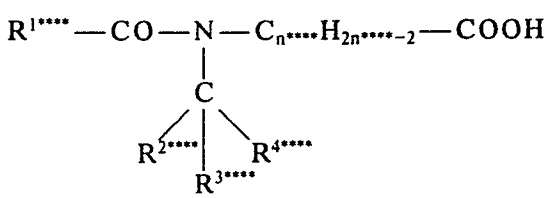

(IV****)

gegebenenfalls unter Schutz der Carboxylgruppe, hydriert und gegebenenfalls anschließend in die Salze überführt oder

c) eine Acylaminoalkansäure der allgemeinen Formel V****

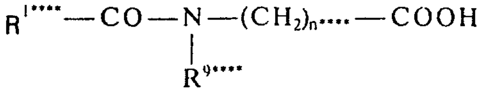

(V****)

worin R9**** ein Wasserstoffatom oder ein Metallatom eines Alkalimetalls bedeutet, gegebenenfalls unter Schutz der Carboxylgruppe mit einem Hydrocarbylderivat der allgemeinen Formel VI****

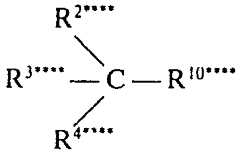

(VI****)

**worin R10**** eine Fluchtgruppe darstellt, umsetzt und gegebenenfalls anschließend in die Salze überführt oder**

d) ein funktionelles Acylhydrocarbylaminoalkansäurederivat der allgemeinen Formel VII****

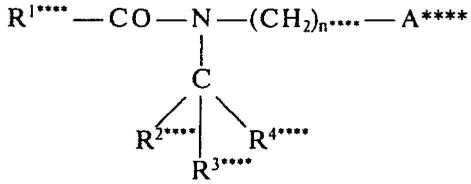

(VII****)

worin A**** ein funktionelles Derivat einer Carboxylgruppe bedeutet, solvolysiert und gegebenenfalls anschließend in die Salze überführt, wobei die Substituenten R1****, R2****, R3****, R4**** und n**** in den Formeln I****–VII**** die in Anspruch 8 angegebene Bedeutung haben.

16. Verbindungen gemäß einem oder mehreren der Ansprüche 1 bis 9 zur Behandlung oder Prophylaxe von Krankheiten, die auf Erkrankungen des Magens oder Darms oder auf Minderleistungen von Pankreas, Galle und/oder Leber beruhen.

**Claims**

Acylhydrocarbylaminoalkanoic acids of the general formula I

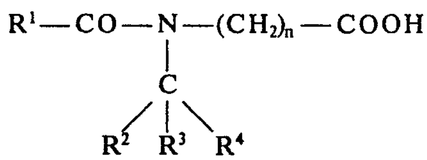

(I)

in which

$R^1$ signifies an alkyl group with 1 to 7 carbon atoms, an alkenyl or alkynyl radical with 2 to 7 carbon atoms, a cycloalkyl group with 3 to 10 carbon atoms or a phenyl radical

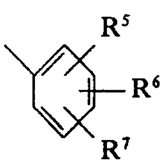

$R^2$ signifies a hydrogen atom, an alkyl group with 1 to 7 carbon atoms, an alkenyl or alkynyl radical with 2 to 7 carbon atoms,

$R^3$ signifies a hydrogen atom, an alkyl group with 1 to 7 carbon atoms, a cycloalkyl group with 3 to 10 carbon atoms or a phenyl radical

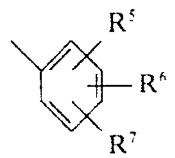

$R^4$ signifies an alkyl group with 1 to 7 carbon atoms, a cycloalkyl group with 3 to 10 carbon atoms, a phenyl radical

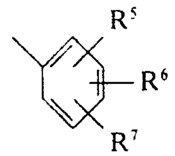

or a phenylalkyl radical with 1 to 4 carbon atoms in the alkyl group and a phenyl radical

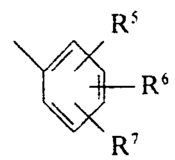

with the proviso that the sum of the carbon atoms of the alkyl groups $R^2$, $R^3$ and $R^4$ is at least 3 and with the proviso that $R^2$, $R^3$ and $R^4$ together by including the neighbouring carbon atom do not form a straight chain or branched alkyl or alkenyl radical if $R^1$ denotes an alkyl or alkenyl group and n denotes 3, or

$R^3$ and $R^4$ together constitute an alkylene group with 2 to 8 carbon atoms or

$R^2$, $R^3$ and $R^4$ by including the neighbouring carbon atom form an adamantyl radical,

n signifies 3, 4 or 5,

$R^5$, $R^6$ and $R^7$ are the same or different and signify a hydrogen atom, a halogen atom, an alkyl group with 1 to 4 carbon atoms, an alkoxy group with 1 to 4 carbon atoms, an alkylmercapto group with 1 to 4 carbon atoms, an alkanoyloxy group with 2 to 5 carbon atoms, an amino group, a nitro group, a trifluoromethyl group, a trifluoromethoxy group, a hydroxy group or a trifluoromethylmercypto group, with the proviso that $R^5$, $R^6$ and $R^7$ do not simultaneously denote hydrogen atoms when $R^1$ and $R^4$ signify phenyl radicals and $R^2$ and $R^3$ signify hydrogen atoms,

and also their salts of inorganic and organic bases.

2. Acylhydrocarbylaminoalkanoic acids according to claim 1 of the general formula I*

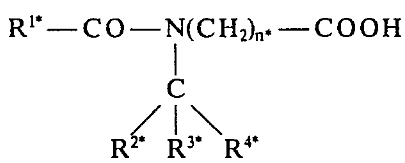

(I*)

in which

$R^{1*}$ signifies an alkyl group with 1 to 5 carbon atoms, an alkenyl or alkynyl radical with 2 to 5 carbon atoms, a cycloalkyl group with 5 to 7 carbon atoms or a phenyl radical

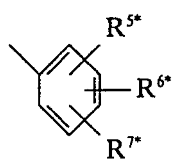

$R^{2*}$ signifies a hydrogen atom, an alkyl group with 1 to 5 carbon atoms, an alkenyl or an alkinyl radical with 2 to 5 carbon atoms,

$R^{3*}$ signifies a hydrogen atom or an alkyl group with 1 to 5 carbon atoms,

$R^{4*}$ signifies an alkyl group with 1 to 5 carbon atoms with the proviso that the sum of the carbon atoms of the alkyl groups $R^{2*}$, $R^{3*}$ and $R^{4*}$ is at least 3 and with the proviso that $R^{2*}$, $R^{3*}$ and $R^{4*}$ together by including the neighbouring carbon atom do not form a straight chain or branched alkyl or alkenyl radical if $R^{1*}$ denotes an alkyl or alkenyl group and $n^*$ denotes 3,

$n^*$ signifies 3, 4 or 5,

$R^{5*}$, $R^{6*}$ and $R^{7*}$ are the same or different and signify a hydrogen atom, a halogen atom, an alkyl group with 1 to 4 carbon atoms, an alkoxy group with 1 to 4 carbon atoms, an alkanoyloxy group with 2 to 5 carbon atoms, an amino group, a nitro group, a hydroxy group or a trifluoromethyl group

and their salts of inorganic or organic bases.

3. Compounds in accordance with Claim 2 in which $R^{1*}$ signifies an alkyl group with 1 to 5 carbon atoms, an alkenyl or alkynyl radical with 2 to 5 carbon atoms or a phenyl radical substituted with $R^{5*}$, $R^{6*}$ and $R^{7*}$, $R^{2*}$ signifies a hydrogen atom, an alkyl group with 1 to 4 carbon atoms or an ethynyl group, $R^{3*}$ signifies a hydrogen atom or an alkyl group with 1 to 3 carbon atoms, $R^{4*}$ signifies an alkyl group with 1 to 5 carbon atoms with the proviso that the sum of the carbon atoms of the alkyl groups $R^{2*}$, $R^{3*}$ and $R^{4*}$ is at least 3 and with the proviso that $R^{2*}$, $R^{3*}$ and $R^{4*}$ together by including the neighbouring carbon atom do not form a straight chain or branched alkyl or alkenyl radical if $R^{1*}$ denotes an alkyl or alkenyl group and $n^*$ denotes 3; $R^{5*}$ signifies a hydrogen atom and $n^*$ signifies 3, 4 or 5, $R^{6*}$ and $R^{7*}$ are the same or different and signify a hydrogen atom, a halogen atom, a methyl group, a methoxy group, an amino group or a trifluoromethyl group, and their salts of inorganic or organic bases.

4. Acylhydrocarbylaminoalkanoic acids according to claim 1 of the general formula I**

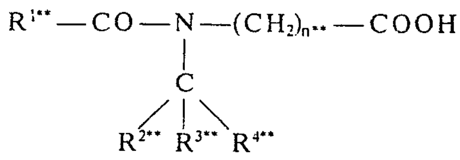

(I**)

in which

$R^{1**}$ has the meanining of $R^{1**}$,
$R^{2**}$ has the meaning of $R^{2**}$,
$R^{3**}$ signifies a hydrogen atom, an alkyl group with 1 to 5 carbon atoms, or a cycloalkyl group with 5 to 7 carbon atoms,
$R^{4**}$ signifies a cycloalkyl group with 5 to 7 carbon atoms or
$R^{3**}$ and $R^{4**}$ together form an alkylene group $-(CH_2)_{q^{**}}$ or
$R^{2**}$, $R^{r**}$ and $R^{4**}$ with the inclusion of the neighbouring carbon atom signify an adamantyl radical,
$n^{**}$ signifies 3, 4 or 5,
$q^{**}$ signifies 4, 5, 6 or 7
$R^{5**}$, $R^{6**}$ and $R^{7**}$ are the same or different and signify a hydrogen atom, a halogen atom, an alkyl group with 1 to 4 carbon atoms, an alkoxy group with 1 to 4 carbon atoms, an alkanoyloxy group with 2 to 5 carbon atoms, an amino group, a nitro group, a hydroxy group or a trifluoromethyl group

and their salts of inorganic or organic bases.

5. Compounds in accordance with Claim 4, in which $R^{1**}$ signifies an alkyl group with 1 to 5 carbon atoms or an alkenyl or alkynyl radical with 2 to 5 carbon atoms or a phenyl radical substituted with $R^{5**}$, $R^{6**}$ and $R^{7**}$, $R^{2**}$ signifies a hydrogen atom, an alkyl group with 1 to 4 carbon atoms or an ethinyl group, $R^{3**}$ signifies a hydrogen atom, a methyl group or a cyclohexyl group, $R^{4**}$ signifies a cyclohexyl group or $R^{3**}$ and $R^{4**}$ together form an alkylene group $-(CH_2)_{q^{**}}$ or else $R^{2**}$, $R^{3**}$ and $R^{4**}$ with the inclusion of the neighbouring carbon atom form an adamantyl-(1) radical, $n^{**}$ signifies 3, 4 or 5, $q^{**}$ signifies 4, 5, 6 or 7, $R^{5**}$ signifies a hydrogen atom, $R^{6**}$ and $R^{7**}$ are the same or different and signify a hydrogen atom, a halogen atom, a methyl group, a methoxy group, an amino group or a trifluoromethyl group, and their salts of inorganic or organic bases.

6. Acylhydrocarbylaminoalkanoic acids according to claim 1 of the general formula I***

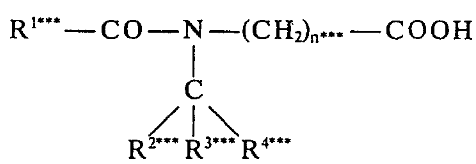

(I***)

in which

$R^{1***}$ has the meaning of $R^{1*}$,
$R^{2***}$ has the meaning of $R^{2*}$,
$R^{3***}$ signifies a hydrogen atom, an alkyl group with 1 to 5 carbon atoms or a phenyl radical

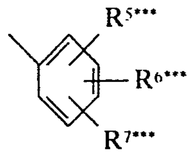

$R^{4***}$ signifies a phenyl radical

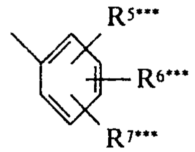

or a phenalkyl radical

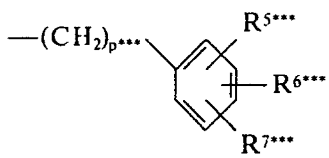

$n^{***}$ signifies 3, 4 or 5,
$p^{***}$ signifies 1, 2, 3 or 4,
$R^{5***}$, $R^{6***}$ and $R^{7***}$ are the same or different and signify a hydrogen atom, a halogen atom, an alkyl group with 1 to 4 carbon atoms, an alkoxy group with 1 to 4 carbon atoms, an alkanoyloxy group with 2 to 5 carbon atoms, an amino group, a nitro group, a hydroxy group or a trifluoromethyl group, with the proviso that $R^{5***}$, $R^{6***}$ and $R^{7***}$ do not simultaneously denote hydrogen atoms when $R^{1***}$ and $R^{4***}$ signify phenyl radicals and $R^{2***}$ and $R^{3***}$ signify hydrogen atoms,

and also their salts of inorganic and organic bases.

7. Compounds in accordance with claim 6, in which $R^{1***}$ signifies an alkyl group with 1 to 5 carbon atoms or a alkenyl or alkynyl radical with 2 to 5 carbon atoms or a phenyl radical substituted with $R^{5***}$, $R^{6***}$ and $R^{7***}$, $R^{2***}$ signifies a hydrogen atom or an alkyl group with 1 to 4 carbon atoms, $R^{3***}$ signifies a hydrogen atom, an alkyl group with 1 to 3 carbon atoms or a phenyl radical substituted with $R^{7***}$, $R^{4***}$ signifies a phenyl radical substituted with $R^{7***}$ or a benzyl radical substituted with $R^{7***}$, $R^{5***}$ signifies a hydrogen atom and $n^{***}$ signifies 3, 4 or 5, $R^{6***}$ and $R^{7***}$ are the same or different and signify a hydrogen atom, a halogen atom, a methyl group, a methoxy group, an amino group or a trifluoromethyl group, with the proviso that $R^{5***}$, $R^{6***}$ and $R^{7***}$ do not simultaneously denote hydrogen atoms when $R^{1***}$ and $R^{4***}$ signify phenyl radicals and $R^{2***}$ and $R^{3***}$ signify hydrogen atoms, and their salts of inorganic and organic bases.

8. Acylhydrocarbylaminoalkanoic acids according to claim 1 of the general formula $I^{****}$

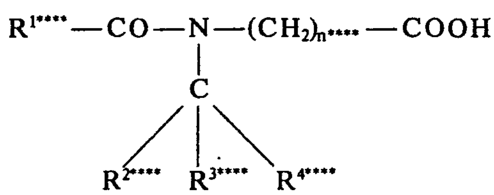

(I****)

in which

$R^{1****}$ has the meaning of $R^{1*}$,
$R^{2****}$ has the meaning of $R^{2*}$,
$R^{3****}$ signifies a hydrogen atom or an alkyl group with 1 to 5 carbon atoms,
$R^{4****}$ signifies a phenyl radical

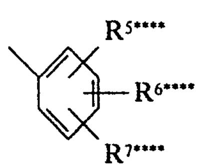

or a phenalkyl radical

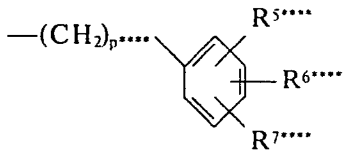

$n^{****}$ signifies 3, 4 or 5,

$p^{****}$ signifies 1, 2, 3 or 4,

$R^{5****}$, $R^{6****}$ and $R^{7****}$ are the same or different and signify a hydrogen atom, a halogen atom, an alkyl group with 1 to 4 carbon atoms, an alkoxy group with 1 to 4 carbon atoms, an alkanoyloxy group with 2 to 5 carbon atoms, an amino group, a nitro group, a hydroxy group or a trifluoromethyl group,

and their salts of inorganic or organic bases.

9. Compounds in accordance with Claim 8, in which $R^{1****}$ signifies an alkyl group with 1 to 5 carbon atoms or an alkenyl or alkynyl radical with 2 to 5 carbon atoms or a phenyl radical substituted with $R^{5****}$, $R^{6****}$ and $R^{7****}$, $R^{2****}$ signifies an ethynyl group, $R^{3****}$ signifies a hydrogen atom or an alkyl group with 1 to 3 carbon atoms, $R^{4****}$ signifies a phenyl radical substituted with $R^{7****}$ or a benzyl radical substituted with $R^{7****}$, $R^{5****}$ signifies a hydrogen atom and $n^{****}$ signifies 3, 4 or 5, $R^{6****}$ and $R^{7****}$ are the same or different and signify a hydrogen atom, a halogen atom, a methyl group, a methoxy group, an amino group or a trifluoromethyl group, and their salts of inorganic or organic bases.

10. Pharmaceutical products containing one or more compounds according to one or more of the claims 1 to 9.

11. Process for the production of acylhydrocarbylaminoalkanoic acids of the general formula I

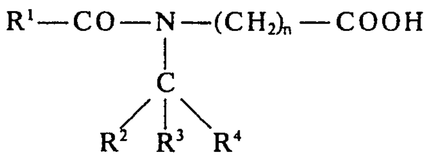
(I)

as well as their salts of inorganic or organic bases, characterized by the fact that

a) a hydrocarbylaminoalkanoic acid of the general formula II

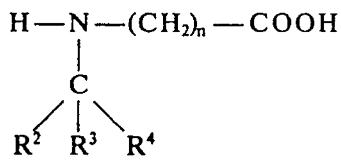
(II)

if desired with protection of the carboxyl group, is acylated with an acyl derivative of the general formula III

$$R^1—CO—R^8 \qquad (III)$$

in which $R^8$ signifies a leaving group or a $R^1—CO—O$ group, and if desired is then converted into the salts or else

b) a hydrocarbylaminoalkenoic acid of the general formula IV

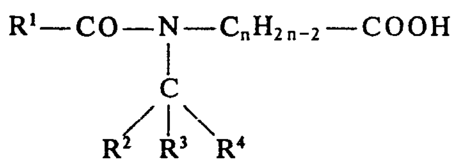
(IV)

if desired with protection of the carboxyl group, is hydrogenated and if desired is then converted into the salts or

c) an acylaminoalkanoic acid of the general formula V

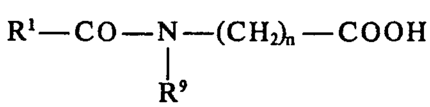
(V)

in which $R^9$ is a hydrogen atom or a metal atom of an alkali or alkaline earth metal, if desired with protection of the carboxyl group, is reacted with a hydrocarbyl derivative of the general formula VI

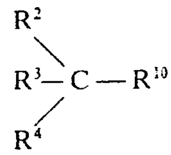

(VI)

in which $R^{10}$ represents a leaving group, and if desired is then converted into the salts or

d) a functional acylhydrocarbylaminoalkanoic acid derivative of the general formula VII

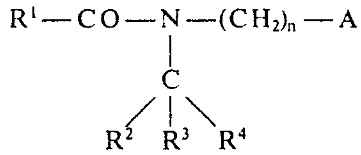

(VII)

in which A is a functional derivative of a carboxyl group, is solvolysed and if desired is then converted into the salts, the substituents $R^1$, $R^2$, $R^3$, $R^4$ and n in the formulae I to VII having the meaning given in claim 1.

12. Process according to claim 11 for the production of the acylhydrocarbylaminoalkanoic acids of the general formula I*

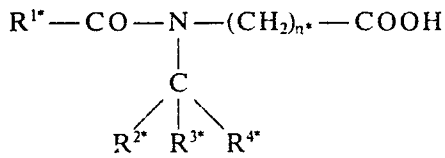

(I*)

and their salts of inorganic or organic bases, characterised by the fact that

a) a hydrocarbylaminoalkanoic acid of the general formula II*

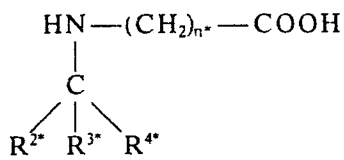

(II*)

if desired with protection of the carboxyl group, is acylated with an acyl derivative of the general formula III*

$$R^{1*}—CO—R^{8*} \qquad (III^*)$$

in which $R^{8*}$ signifies a leaving group or a $R^{1*}$–CO–O– group and if desired is then converted into the salts or

b) a hydrocarbylaminoalkenoic acid of the general formula IV*

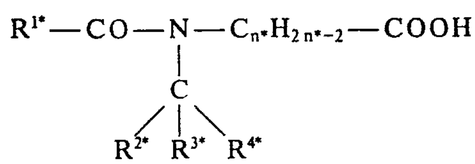

(IV*)

if desired with protection of the carboxyl group, is hydrogenated and if desired is then converted into the salts or

c) an acylaminoalkanoic acid of the general formula V*

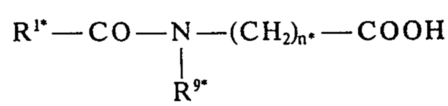

(V*)

in which $R^{9*}$ is a hydrogen atom or a metal atom of an alkali metal, if desired with protection of the carboxyl group, is reacted with a hydrocarbyl derivative of the general formula VI*

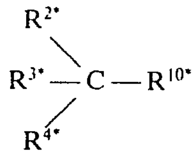

(VI*)

in which $R^{10^*}$ signifies a leaving group, and if desired is then converted into the salts or

d) a functional acylhydrocarbylaminoalkanoic acid derivative of the general formula VII*

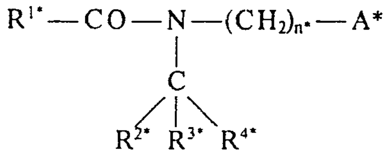

(VII*)

in which A* is a functional derivative of a carboxyl group, is solvolysed and if desired is then converted into the salts, the substituents $R^{1^*}$, $R^{2^*}$, $R^{3^*}$, $R^{4^*}$ and n* in the formulae I* to VII* having the meaning given in claim 2.

13. Process according to claim 11 for the production of the acylhydrocarbylaminoalkanoic acids of the general formula I**

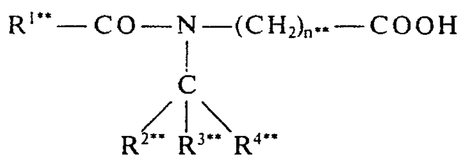

(I**)

and their salts of inorganic or organic bases, characterised by the fact that

a) a hydrocarbylaminoalkanoic acid of the general formula II**

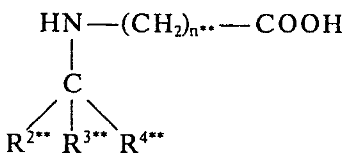

(II**)

if desired with protection of the carboxyl group, is acylated with an acyl derivative of the general formula III**

$$R^{1^{**}}—CO—R^{8^{**}} \qquad (III^{**})$$

in which $R^{8^{**}}$ is a leaving group or a $R^{1^{**}}$ –CO–O-group and if desired it is then converted into the salts or else

b) a hydrocarbylaminoalkenoic acid of the general formula IV**

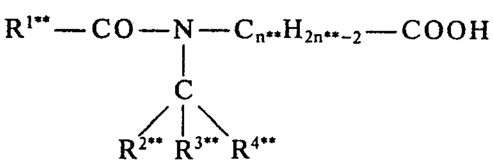

(IV**)

if desired with protection of the carboxyl group, is hydrogenated and if desired is then converted into the salts or else

c) an acylaminoalkanoic acid of the general formula V**

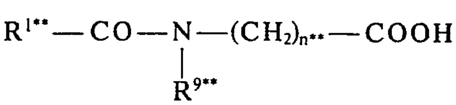

(V**)

in which $R^{9^{**}}$ signifies a hydrogen atom or a metal atom of an alkali metal, if desired with protection of the carboxyl group, is reacted with a hydrocarbyl derivative of the general formula VI**

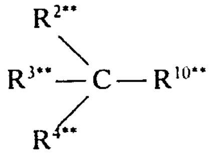

(VI**)

in which $R^{10**}$ represents a leaving group, and if desired is then converted into the salts or

d) a functional acylhydrocarbylaminoalkanoic acid derivative of the general formula VII**

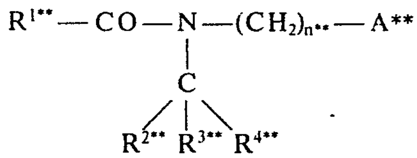

(VII**)

in which A** is a functional derivative of a carboxyl group, is solvolysed and if desired is then converted into salts, the substituents $R^{1**}$, $R^{2**}$, $R^{3**}$, $R^{4**}$ and n** in the formulae I** to VII** having the meaning given in claim 4.

14. Process according to Claim 11 for the production of the acylhydrocarbylaminoalkanoic acids of the general formula I***

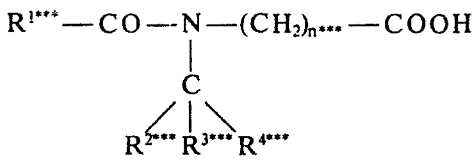

(I***)

and their salts of inorganic or organic basas, characterised by the fact that

a) a hydrocarbylaminoalkanoic acid of the general formula II***

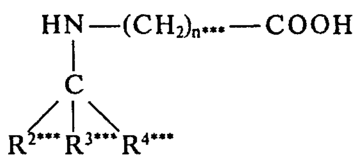

(II***)

if desired with protection of the carboxyl group, is acylated withe an acyl derivative of the general formula III***

$$R^{1***}—CO—R^{8***} \qquad (III^{***})$$

in which $R^{8***}$ is a leaving group or a $R^{***}-CO-O$-group and if desired is then converted into the salts or

b) a hydrocarbylaminoalkenoic acid of the general formula IV***

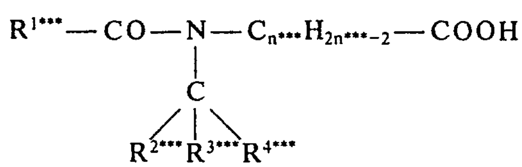

(IV***)

if desired with protection of the carboxyl group, is hydrogenated and if desired is then converted into the salts or

c) an acylaminoalkanoic acid of the general formula V***

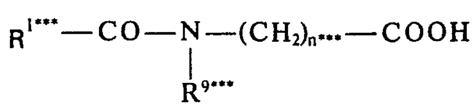

(V***)

in which $R^{9***}$ signifies a hydrogen atom or a metal atom of an alkali metal, if desired with protection of the carboxyl group, is reacted with a hydrocarbyl derivative of the general formula VI***

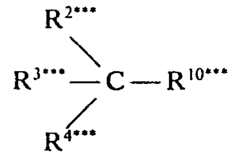

(VI***)

in which $R^{10***}$ represents a leaving group, and if desired is then converted into the salts or

d) a functional acylhydrocarbylaminoalkanoic acid derivative of the general formula VII***

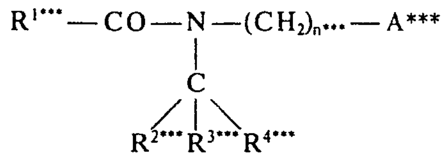

(VII***)

in which A*** signifies a functional derivative of a carboxyl group, is solvolysed and if desired is then converted into the salts, the substituents $R^{1***}$, $R^{2***}$, $R^{3***}$, $R^{4***}$ and n*** in the formulae I*** to VII*** having the meaning given in claim 6.

15. Process according to claim 11 for the production of the acylhydrocarbylaminoalkanoic acids of the general formula I***

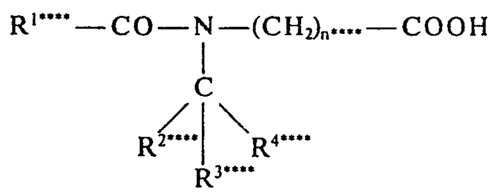

(I****)

and their salts of inorganic or organic bases, characterised by the fact that,

a) a hydrocarbylaminoalkanoic acid of the general formula II****

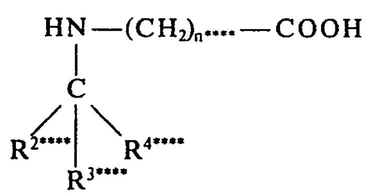

(II****)

if desired with protection of the carboxyl group, is acylated with an acyl derivative of the general formula III****

$$R^{1****}—CO—R^{8****} \qquad (III^{****})$$

in which $R^{8****}$ signifies a leaving group or a $R^{1****}-CO-O$-group and if desired is then converted into the salts or

b) a hydrocarbylaminoalkenoic acid of the general formula IV****

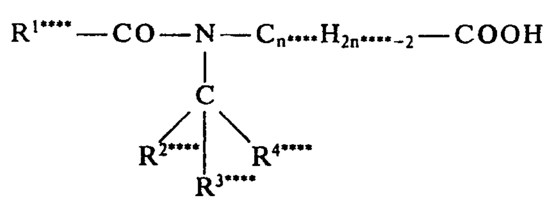

(IV****)

if desired with protection of the carboxyl group, is hydrogenated and if desired is then converted into the salts or

c) an acylaminoalkanoic acid of the general formula V****

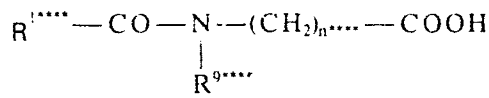

(V****)

in which $R^{9****}$ signifies a hydrogen atom or a metal atom of an alkali metal, if desired with protection of the carboxyl group is reacted with a hydrocarbyl derivative of the general formula VI****

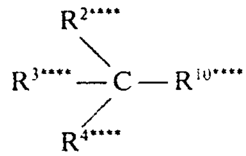

(VI****)

in which $R^{10****}$ signifies a leaving group and if desired is then converted into the salts or

d) a functional acylhydrocarbylaminoalkanoic acid derivative of the general formula VII****

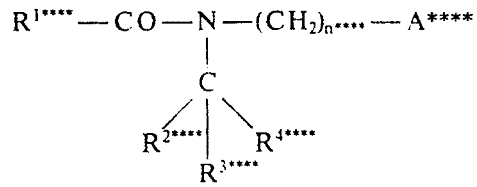

(VII****)

in which A**** signifies a functional derivative of a carboxyl group, is solvolysed and if desired is then converted into the salts, the substituents $R^{1****}$, $R^{2****}$, $R^{3****}$, $R^{4****}$ and n**** in the formulae I**** to VII**** having the meaning given in claim 8.

16. Compounds in accordance with one or more of the claims 1 to 9 for the treatment and prophylaxis of diseases which are attributable to desorders of the stomach or intestine or to reducted performances of the pancreas, bile and/or liver.

## Revendications

1. Acides acylhydrocarbylaminoalkancarboxyliques de la formule générale I

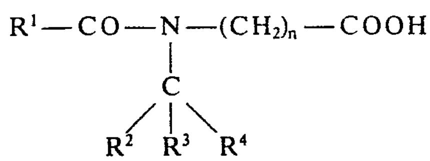

(I)

dans laquelle

$R^1$ représente un groupe alkyle comportant de 1 à 7 atomes de carbone, un groupe alkényle ou alkinyle comportant de 2 à 7 atomes de carbone, un groupe cycloalkyle comportant de 3 à 10 atomes de carbone ou un reste phényle

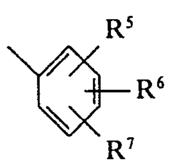

$R^2$ représente un atome d'hydrogène, un groupe alkyle comportant de 1 à 7 atomes de carbone, un reste alkényle ou alkinyle comportant de 2 à 7 atomes de carbone,

$R^3$ représente un atom d'hydrogène, un groupe alkyle comportant de 1 à 7 atomes de carbone, un groupe cycloalkyle comportant de 3 à 10 atomes de carbone ou un reste phényle

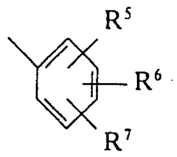

et

$R^4$ représente un groupe alkyle comportant de 1 à 7 atomes de carbone, un groupe cycloalkyle comportant de 23 à 10 atomes de carbone, un reste phényle

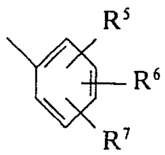

ou un reste phénylalkyle avec un groupe alkyle comportant de 1 à 4 atomes de carbone et un reste phényle

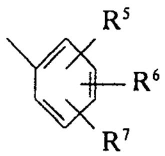

la somme des atomes de carbone des substituants alkyle $R^2$, $R^3$ et $R^4$ étant au moins 3, et $R^2$, $R^3$ et $R^4$ ne pouvant pas représenter, conjointement avec l'atome de carbone auquel ils sont liés, un groupe alkyle ou alkényle à chaîne linéaire ou ramifiée, lorsque $R^1$ est un résidu alkyle ou alkényle et que n égal à 3, ou

$R^3$ et $R^4$ forment ensemble un groupe alkylène comportant de 2 à 8 atomes de carbone ou encore

$R^2$, $R^3$ et $R^4$ en englobant l'atome de carbone vicinal forment un reste adamantyle,

n est 3, 4 ou 5 et

$R^5$, $R^6$ et $R^7$ sont identiques ou différents et représentent un atome d'hydrogène, un atome d'halogène, un groupe alkyle comportant de 1 à 4 atomes de carbone, un groupe alkoxy comportant de 1 à 4 atomes de carbone, un groupe alkylmercapto comportant de 1 à 4 atomes de carbone, un groupe alkanoyloxy comportant de 2 à 5 atomes de carbone, un groupe amino, un groupe nitro, un groupe trifluorométhyle, un groupe trifluorométhoxy, un groupe hydroxy ou un groupe trifluorométhylmercapto, $R^5$, $R^6$ et $R^7$ ne pouvant pas représenter ou même temps des atomes d'hydrogéne lorsque $R^1$ et $R^4$ sont des groupes phényle et $R^2$ et $R^3$ sont des atomes d'hydrogéne,

ainsi que les sels de ces composés avec des bases inorganiques ou organiques.

2. Acides acylhydrocarbylaminoalkancarboxyliques selon la revendication 1, charactérisés en ce qu'ils répondent à la formule générale I*

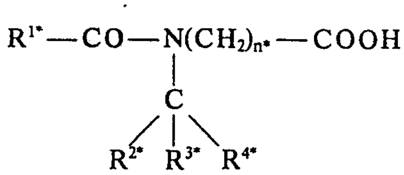

(I*)

dans laquelle

$R^{1*}$ représente un groupe alkyle comportant de 1 à 5 atomes de carbone, un groupe alkényle ou alkinyle comportant de 2 à 5 atomes de carbone, un reste cycloalkyle comportant de 5 à 7 atomes de carbone ou un reste phényle

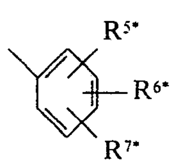

$R^{2*}$ représente un atome d'hydrogène, un groupe alkyle comportant de 1 à 5 atomes de carbone, un reste alkényle ou alkinyle comportant de 2 à 5 atomes de carbone,
$R^{3*}$ représente un atome d'hydrogène ou un groupe alkyle comportant de 1 à 5 atomes de carbone,
$R^{4*}$ représente un groupe alkyle comportant de 1 à 5 atomes de carbone, la somme des atomes de carbone des substituants alkyle $R^{2*}$, $R^{3*}$ et $R^{4*}$ étant au moins 3, et $R^{2*}$, $R^{3*}$ et $R^{4*}$ ne pouvant pas représenter, conjointement avec l'atome de carbone auquel ils sont liés, un groupe alkyle ou alkényle à chaîne linéaire ou ramifiée, lorsque $R^{1*}$ est un résidu alkyle ou alkényle et que $n^*$ est égal à 3,
$n^*$ représente 3, 4 ou 5,
$R^{5*}$, $R^{6*}$ et $R^{7*}$ sont identiques ou différents et représentent un atome d'hydrogène, un atome d'halogène, un groupe alkyle comportant de 1 à 4 atomes de carbone, un groupe alkoxy comportant de 1 à 4 atomes de carbone, un groupe alkanoyloxy comportant de 2 à 5 atomes de carbone, un groupe amino, un groupe nitro, un groupe hydroxy ou un groupe trifluorométhyle,

ainsi que les sels de ces composés avec des bases inorganiques ou organiques.

3. Composés selon la revendication 2, charactérisés en ce que $R^{1*}$ représente un groupe alkyle comportant de 1 à 5 atomes de carbone, un groupe alkényle ou alkinyle comportant de 2 à 5 atomes de carbone, ou un reste phényle substitué par $R^{5*}$, $R^{6*}$ et $R^{7*}$, $R^{2*}$ représente un atome d'hydrogène, un groupe alkyle comportant de 1 à 4 atomes de carbone ou un groupe éthinyle, $R^{3*}$ représente un atome d'hydrogène ou un groupe alkyle comportant de 1 à 3 atomes de carbone, $R^{4*}$ représente un groupe alkyle comportant de 1 à 5 atomes de carbone, la somme des atomes de carbone des substituants alkyle $R^{2*}$, $R^{3*}$ et $R^{4*}$ étant au moins 3, et $R^{2*}$, $R^{3*}$ et $R^{4*}$ ne pouvant pas représenter, conjointement avec l'atome de carbone auquel ils sont liés, un groupe alkyle ou alkényle à chaîne linéaire ou ramifiée, lorsque $R^{1*}$ est un résidu alkyle our alkényle et que $n^*$ est égal à 3, $R^{5*}$ représente un atome d'hydrogène et $n^*$ représente 3, 4 ou 5, $R^{6*}$ et $R^{7*}$ sont identiques ou différents et représentent un atome d'hydrogène, un atome d'halogène, un groupe méthyle, un groupe méthoxy, un groupe amino ou un groupe trifluorométhyle ainsi que les sels de ces composés avec des bases inorganiques ou organiques.

4. Acides acylhydrocarbylaminoalkancarboxyliques selon la revendication 1, charactérisés en ce qu'ils répondent à la formule générale I**

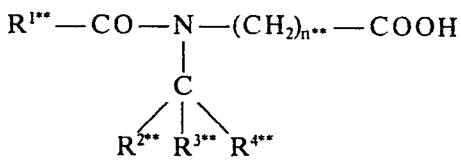

(I**)

dans laquelle

$R^{1**}$ a la signification de $R^{1**}$
$R^{2**}$ a la signification de $R^{2**}$
$R^{3**}$ représente un atome d'hydrogène, un groupe alkyle comportant de 1 à 5 atomes de carbone ou un groupe cycloalkyle comportant de 5 à 7 atomes de carbone,
$R^{4**}$ représente un groupe cycloalkyle comportant de 5 à 7 atomes de carbone ou
$R^{3**}$ et $R^{4**}$ forment ensemble le groupe alkylène $-(CH_2)_{q^{**}}$ ou encore
$R^{2**}$, $R^{3**}$ et $R^{4**}$ en englobant l'atome de carbone vicinal forment un reste adamantyle,
$n^{**}$ est 3, 4 ou 5,
$q^{**}$ est 4, 5, 6 ou 7,
$R^{5**}$, $R^{6**}$ et $R^{7**}$ sont identiques ou différents et représentent un atome d'hydrogène, un atome d'halogène, un groupe alkyle comportant de 1 à 4 atomes de carbone, un groupe alkoxy comportant de 1 à 4 atomes de carbone, un groupe alkanoyloxy comportant de 2 à 5 atomes de carbone, un groupe amino, un groupe nitro, un groupe hydroxy ou un groupe trifluorométhyle,

ainsi que les sels de ces composés avec des bases inorganiques ou organiques.

5. Composés selon la revendication 4, characterisés en ce que $R^{1**}$ représente un groupe alkyle comportant de 1 à 5 atomes de carbone, un groupe alkényle ou alkinyle comportant de 2 à 5 atomes de carbone, ou un reste phényle substitué par $R^{5**}$, $R^{6**}$ et $R^{7**}$, $R^{2**}$ représente un atome d'hydrogène, un groupe alkyle comportant de 1 à 4 atomes de carbone ou un groupe éthinyle, $R^{3**}$ représente un atome d'hydrogène, un groupe méthyle ou un groupe cyclohexyle, $R^{4**}$ représente un groupe cyclohexyle ou $R^{3**}$ et $R^{4**}$ forment ensemble un groupe alkylène $-(CH_2)_{q^{**}}$ ou $R^{2**}$, $R^{3**}$ et $R^{4**}$ en englobant l'atome de carbone vicinal forment un reste adamantyle-(1), $n^{**}$ est 3, 4 ou 5, $q^{**}$ est 4, 5, 6 ou 7, $R^{5**}$ représente un atome d'hydrogène, $R^{6**}$ et $R^{7**}$ sont identiques ou différents et représentent un atome d'hydrogène, un atome d'halogène, un groupe méthyle, un groupe méthoxy, un groupe amino, un groupe trifluorométhyle, ainsi que les sels de ces composés avec des bases inorganiques ou organiques.

6. Acides acylhydrocarbylaminoalkancarboxyliques selon la revendication 1, charactérisés en ce qu'ils répondent à la formule générale I***

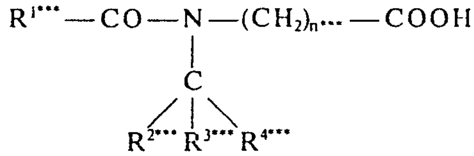

(I***)

dans laquelle

$R^{1***}$ a la signification de $R^{1*}$,
$R^{2***}$ a la signification de $R^{2*}$,
$R^{3***}$ un atome d'hydrogène, un groupe alkyle comportant de 1 à 5 atomes de carbone ou un reste phényle

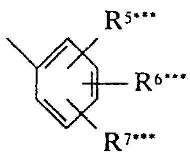

$R^{4***}$ représente un reste phényle

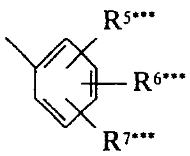

ou un reste

phénalkyle 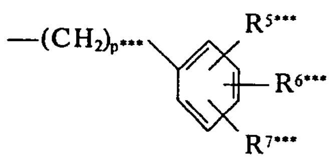

$n^{***}$ est 3, 4 ou 5,
$p^{***}$ est 1, 2, 3 ou 4,
$R^{5***}$, $R^{6***}$ et $R^{7***}$ sont identiques ou différents et représentent un atome d'hydrogène, un atome d'halogène, un groupe alkyle comportant de 1 à 4 atomes de carbone, un groupe alkoxy comportant de 1 à 4 atomes de carbone, un groupe alkanoyloxy comportant de 2 à 5 atomes de carbone, un groupe amino, un groupe nitro, un groupe hydroxy ou un groupe trifluorométhyle, $R^{5***}$, $R^{6***}$ et $R^{7***}$ ne pouvant pas représenter ou même temps des atomes d'hydrogène lorsque $R^{1***}$ et $R^{4***}$ sont des groupes phényle et $R^{2***}$ et $R^{3***}$ sont des atomes d'hydrogène,

ainsi que les sels de ces composés avec des bases inorganiques ou organiques.

7. Composés selon la revendication 6, chararactérisés en ce que $R^{1***}$ représente un groupe alkyle comportant de 1 à 5 atomes de carbone, un groupe alkényle ou alkinyle comportant de 2 à 5 atomes de carbone, ou un reste phényle substitué par $R^{5***}$, $R^{6***}$ et $R^{7***}$, $R^{2***}$ représente un atome d'hydrogène ou un groupe alkyle comportant de 1 à 4 atomes de carbone, $R^{3***}$ un atome d'hydrogène, un groupe alkyle comportant de 1 à 3 atomes de carbone ou un reste phényle substitué par $R^{7***}$, $R^{4***}$ un reste phényle substitué par $R^{7***}$ ou un reste benzyle substitué par $R^{7***}$, $R^{5***}$ représente un atome d'hydrogène, et $n^{***}$ est 3, 4 ou 5, $R^{6***}$ et $R^{7***}$ sont identiques ou différents et représentent un atome d'hydrogène, un atome d'halogène, un groupe méthyle, un groupe méthoxy, un groupe amino ou un groupe trifluorométhyle, $R^{5***}$, $R^{6***}$ et $R^{7***}$ ne pouvant pas représenter ou même temps des atomes d'hydrogène lorsque $R^{1***}$ et $R^{4***}$ sont des groupes phényle et $R^{2***}$ et $R^{3***}$ sont des atomes d'hydrogène, ainsi que les sels de ces composés avec des bases inorganiques ou organiques.

8. Acides acylhydrocarbylaminoalkancarboxyliques selon la revendication 1, charactérisés en ce qu'ils répondent à la formule générale $I^{****}$

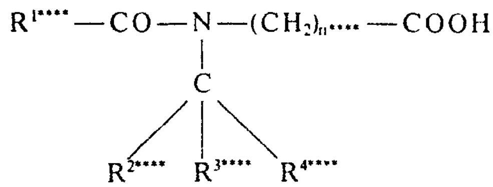

(I****)

dans laquelle

$R^{1****}$ a la signification de $R^{1*}$,
$R^{2****}$ a la signification de $R^{2*}$,
$R^{3****}$ représente un atome d'hydrogène ou un groupe alkyle comportant de 1 à 5 atomes de carbone,
$R^{4****}$ représente un reste phényle

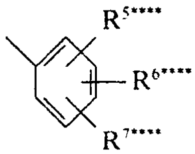

ou un reste phénalkyle

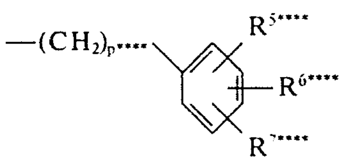

$n^{****}$ est 3, 4 ou 5,
$p^{****}$ est 1, 2, 3 ou 4,
$R^{5****}$, $R^{6****}$ et $R^{7****}$ sont identiques ou différents et représentent un atome d'hydrogène, un atome d'halogène, un groupe alkyle comportant de 1 à 4 atomes de carbone, un groupe alkoxy comportant de 1 à 4 atomes de carbone, un groupe alkanoyloxy comportant de 2 à 5 atomes de carbone, un groupe amino, un groupe nitro, un groupe hydroxy ou un groupe trifluorométhyle,

ainsi que les sels de ces composés avec des bases inorganiques ou organiques.

9. Composés selon la revendication 8, characterisés en ce que $R^{1****}$ représente un groupe alkyle comportant de 1 à 5 atomes de carbone, un groupe alkényle ou alkinyle comportant de 2 à 5 atomes de carbone, ou un reste phényle substitué par $R^{5****}$, $R^{6****}$ et $R^{7****}$, $R^{2****}$ représente un groupe éthinyle, $R^{3****}$ représente un atome d'hydrogène ou un groupe alkyle comportant de 1 à 3 atomes de carbone, $R^{4****}$ représente un reste phényle substitué par $R^{7****}$ ou un reste benzyle substitué par $R^{7****}$, $R^{5****}$ représente un atome d'hydrogène et $n^{****}$ est 3, 4 ou 5, $R^{6****}$ et $R^{7****}$ sont identiques ou différents et représentent un atome d'hydrogène, un atome d'halogène, un groupe méthyle, un groupe méthoxy, un groupe amino ou un groupe trifluorométhyle, ainsi que les sels de ces composés avec des bases inorganiques ou organiques.

10. Médicaments contenant un ou plusieurs composés suivant selon une ou plusieurs des revendications 1 à 9.

11. Procédé pour la préparation d'acides acylhydrocarbylaminoalkancarboxyliques de la formule générale I

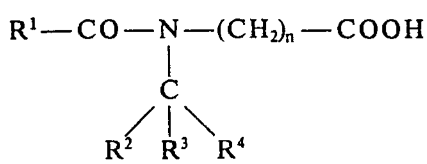

(I)

de même que leurs sels avec des bases inorganiques ou organiques, caractérisé en ce que

a) on acyle un acide hydrocarbylaminoalkancarboxyliques de la formule générale II

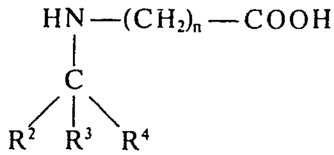

(II)

éventuellement en protégeant le groupe carboxylique, avec un dérivé acyle de la formule générale III

$$R^1—CO—R^8 \qquad (III)$$

dans laquelle $R^8$ constitue un groupe scindable ou représente un groupe $R^1-CO-O$, après quoi on transforme éventuellement en sels ou

b) on hydrogène un acide hydrocarbylaminoalkèncarboxylique de la formule générale IV

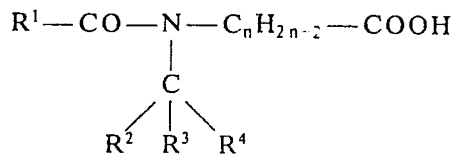

(IV)

éventuellement en protégeant le groupe carboxyliques après quoi on transforme éventuellement le composé en sels ou

c) on fait réagir un acide acylaminoalkancarboxylique de la formule générale V

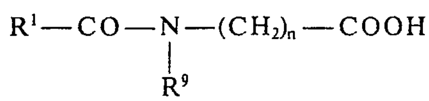

(V)

dans laquelle $R^9$ représente un atome d'hydrogène, une valence d'un métal alcalino terreux ou d'un métal alcalin, éventuellement en protégeant le groupe carboxylique, avec un dérivé hydrocarbyle de la formule générale VI

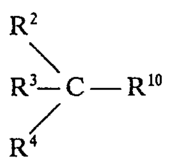

(VI)

dans laquelle $R^{10}$ représente un groupe scindable, après quoi on transforme éventuellement en sels ou

d) on soumet à une solvolyse un dérivé fonctionnel d'acide acylhydrocarbylaminoalkancarboxylique de la formule générale VII

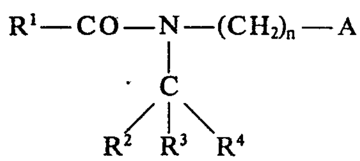

(VII)

dans laquelle A représente un dérivé fonctionnel d'un groupe carboxyle, après quoi on transforme éventuellement en sels, les substituants $R^1$, $R^2$, $R^3$, $R^4$ et n dans les formules I à VII ayant les significations indiquées dans la revendication 1.

12. Procédé selon la revendication 11 pour la préparation d'acides acylhydrocarbylaminoalkancarboxyliques de la formule générale I*

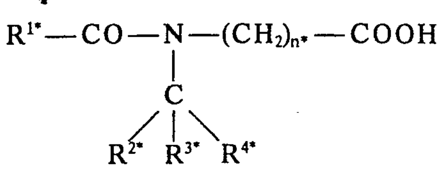

(I*)

de même que leurs sels avec des bases inorganiques ou organiques, caractérisé en ce que

a) on acyle un acide hydrocarbylaminoalkancarboxylique de la formule générale II*

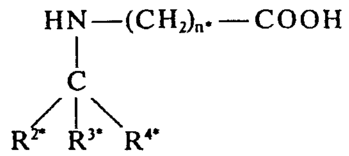

(II*)

éventuellement en protégeant le groupe carboxylique, avec un dérivé acyle de la formule générale III*

$$R^{1*}—CO—R^{8*} \quad (III^*)$$

dans laquelle $R^{8*}$ constitue un groupe scindable ou représente un groupe $R^{1*}$–CO–O–, après quoi on transforme éventuellement en sels ou

b) on hydrogène un acide hydrocarbylaminoalkèncarboxylique de la formule générale IV*

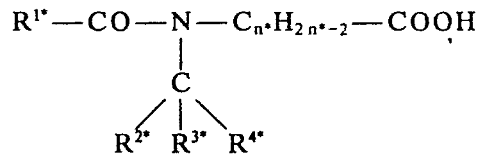

(IV*)

éventuellement en protégeant le groupe carboxylique, après quoi on transforme éventuellement le composé en sels ou

c) on fait réagir un acide acylaminoalkancarboxylique de la formule générale V*

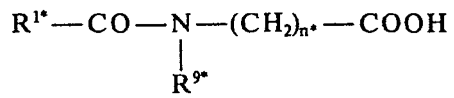

(V*)

dans laquelle $R^{9*}$ représente un atome d'hydrogène ou une valence d'un métal alcalin, éventuellement en protégeant le groupe carboxylique, avec un dérivé hydrocarbyle de la formule générale VI*

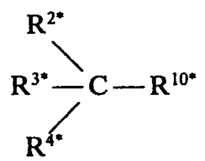

(VI*)

dans laquelle $R^{10*}$ représente un groupe scindable, après quoi on transforme éventuellement en sels ou

d) on soumet à une solvolyse un dérivé fonctionnel d'acide acylhydrocarbylaminoalkancarboxylique de la formule générale VII*

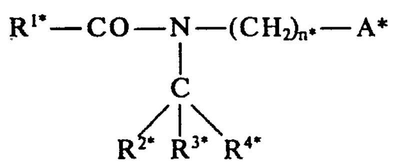

(VII*)

dans laquelle A* représente un dérivé fonctionnel d'un groupe carboxyle, après quoi on transforme éventuellement en sels, les substituants $R^{1*}$, $R^{2*}$, $R^{3*}$, $R^{4*}$ et n* dans les formules I* à VII* ayant les significations indiquées dans la revendication 2.

13. Procédé selon la revendication 11 pour la préparation d'acides acylhydrocarbylaminoalkancarboxyliques de la formule générale I**

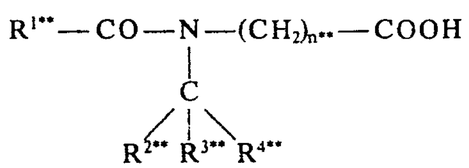

(I**)

de même que leurs sels avec des bases inorganiques ou organiques, caractérisé en ce que

a) on acyle un acide hydrocarbylaminoalkancarboxylique de la formule générale II**

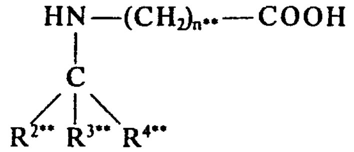

(II**)

éventuellement en protégeant le groupe carboxylique, avec un dérivé acyle de la formule générale III**

$$R^{1**}—CO—R^{8**} \qquad (III^{**})$$

dans laquelle $R^{8**}$ constitue un groupe scindable ou représente un groupe $R^{1**}$–CO–O, après quoi on transforme éventuellement en sels ou

b) on hydrogène un acide hydrocarbylaminoalkèncarboxylique de la formule générale IV**

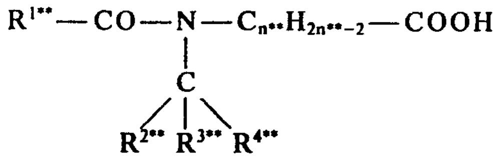

(IV**)

éventuellement en protégeant le groupe carboxylique, après quoi on transforme éventuellement le composé en sels ou

c) on fait réagir un acide acylaminoalkancarboxylique de la formule générale V**

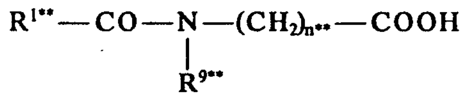

(V**)

dans laquelle $R^{9**}$ représente un atome d'hydrogène ou une valence d'un métal alcalin, éventuellement en protégeant le groupe carboxylique, avec un dérivé hydrocarbyle de la formule générale VI**

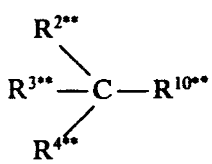

(VI**)

dans laquelle $R^{10**}$ représente un groupe scindable, après quoi on transforme éventuellement en sels ou

d) on soumet à une solvolyse un dérivé fonctionnel d'acide acylhydrocarbylaminoalkancarboxylique de la formule générale VII**

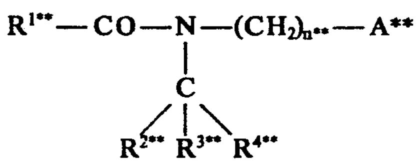

(VII**)

dans laquelle A** représente un dérivé fonctionnel d'un groupe carboxyle, après quoi on transforme éventuellement en sels, les substituants $R^{1**}$, $R^{2**}$, $R^{3**}$, $R^{4**}$ et n** dans les formules I** à VII** ayant les significations indiquées dans la revendication 4.

14. Procédé selon la revendication 11 pour la préparation d'acides acylhydrocarbylaminoalkancarboxyliques de la formule générale I***

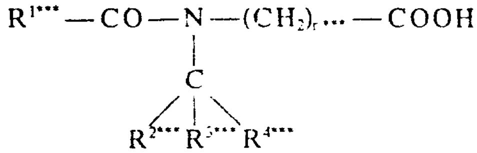

(I***)

de même que leurs sels avec des bases inorganiques ou organiques, caractérisé en ce que

a) on acyle un acide hydrocarbylaminoalkancarboxylique de la formule générale II***

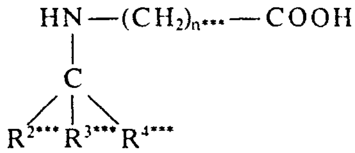

(II***)

éventuellement en protégeant le groupe carboxylique, avec un dérivé acyle de la formule générale III***

$R^{1***} — CO — R^{8***}$ (III***)

dans laquelle $R^{8***}$ constitue un groupe scindable ou représente un groupe $R^{1***} - CO - O$, après quoi on transforme éventuellement en sels ou

b) on hydrogène un acide hydrocarbylaminoalkèncarboxylique de la formule générale IV***

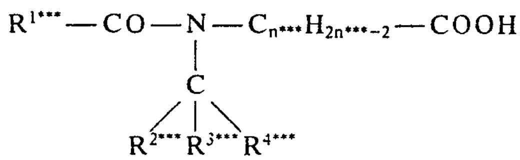

(IV**)

éventuellement en protégeant le groupe carboxylique, après quoi on transforme éventuellement le composé en sels ou

c) on fait réagir un acide acylaminoalkancarboxylique de la formule générale V***

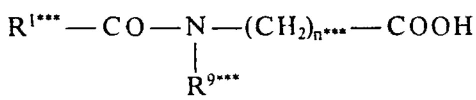

(V***)

dans laquelle $R^{9***}$ représente un atome d'hydrogène ou un valence d'un métal alcalin, éventuellement en protégeant le groupe carboxylique, avec un dérivé hydrocarbyle de la formule générale VI***

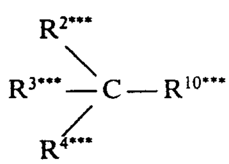

(VI***)

dans laquelle $R^{10***}$ représente un groupe scindable, après quoi on transforme éventuellement en sels ou

d) on soumet à une solvolyse un dérivé fonctionnel d'acide acylhydrocarbylaminoalkancarboxylique de la formule générale VII***

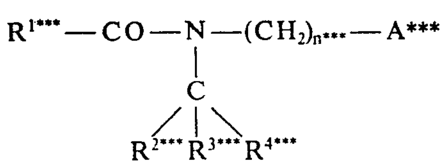

(VII***)

dans laquelle A*** représente un dérivé fonctionnel d'un groupe carboxyle, après quoi on transforme éventuellement en sels, les substituants $R^{1***}$, $R^{2***}$, $R^{3***}$, $R^{4***}$ et n*** dans les formule I*** à VII*** ayant les significations indiquées dans la revendication 6.

15. Procédé selon la revendication 11 pour la préparation d'acides acylhydrocarbylaminoalkancarboxyliques de la formule générale I****

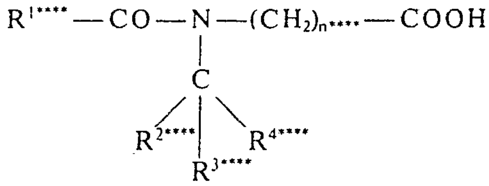

(I****)

de même que leurs sels avec des bases inorganiques ou organiques, caractérisé en ce que

a) on acyle un acide hydrocarbylaminoalkancarboxylique de la formule générale II***

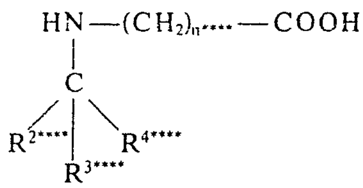

(II****)

éventuellement en protégeant le groupe carboxylique, avec un dérivé acyle de la formule générale III****

$$R^{1****}-CO-R^{8****} \qquad (III^{****})$$

dans laquelle $R^{8****}$ constitue un groupe scindable ou représente un groupe $R^{1****}-CO-O-$, après quoi on transforme éventuellement en sels ou

b) on hydrogène un acide hydrocarbylaminoalkèncarboxylique de la formule générale IV****

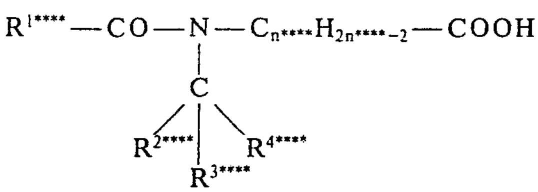

(IV****)

éventuellement en protégeant le groupe carboxylique, après quoi on transforme éventuellement le composé en sels ou

c) on fait réagir un acide acylaminoalkancarboxylique de la formule générale V****

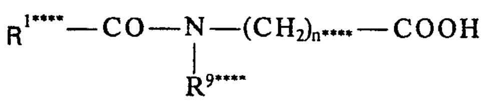

(V****)

dans laquelle $R^{9****}$ représente un atome d'hydrogène ou une valence d'un métal alcalin, éventuellement en protégeant le groupe carboxylique, avec un dérivé hydrocarbyle de la formule générale VI****

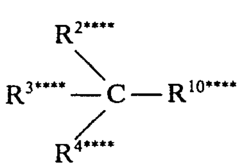

(VI****)

dans laquelle $R^{10****}$ représente un groupe scindable, après quoi on transforme éventuellement en sels ou

d) on soumet à une solvolyse un dérivé fonctionnel d'acide acylhydrocarbylaminoalkancarboxylique de la formule VI****

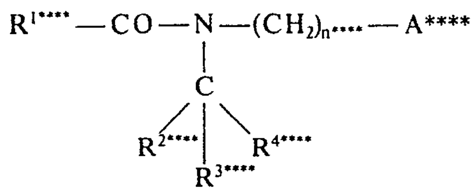

(VII****)

dans laquelle A**** représente un dérivé fonctionnel d'un groupe carboxyle, après quoi on transforme éventuellement en sels, les substituants $R^{1****}$, $R^{2****}$, $R^{3****}$, $R^{4****}$ et n**** dans les formules I**** à VII**** ayant les significations indiquées dans la revendication 8

16. Composés selon une ou plusieurs des revendications 1 à 9 pour le traitement et la prophylaxie de maladies dues à des affection de l'estomac ou de l'intestine ou encore à un rendement moins bon du pancréas, de la bile et/ou du foie.